# EUROPÄISCHE PATENTSCHRIFT

(11) **EP 3 057 947 B1**
(45) Veröffentlichungstag und Bekanntmachung des Hinweises auf die Patenterteilung: **17.10.2018**
(21) Anmeldenummer: 14721764.0
(22) Anmeldetag: 29.04.2014
(51) Int. Cl.: C07D 311/80, C07C 255/52, C07D 335/10, C07C 2/08, C07D 409/14, C07D 209/82, C07C 13/62, C07F 7/02

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 14.10.2013 EP 13004921
(43) Veröffentlichungstag der Anmeldung: 24.08.2016
(73) Patentinhaber: Merck Patent GmbH, 64293 Darmstadt (DE)
(72) Erfinder: HEIL, Holger, 60389 Frankfurt am Main (DE); RODRIGUEZ, Lara-lsabel, 64283 Darmstadt (DE); BURKHART, Beate, 64293 Darmstadt (DE); DARSY, Amandine, 60318 Frankfurt am Main (DE)
(86) Internationale Anmeldenummer: PCT/EP2014/001148
(87) Internationale Veröffentlichungsnummer: WO 2014/111269

(56) Entgegenhaltungen:
- COCHEREL, NICOLAS ET AL: "New 3.pi.-2spiro ladder-type phenylene materials: synthesis, physicochemical properties and applications in OLEDs", CHEMISTRY - A EUROPEAN JOURNAL, Bd. 14, Nr. 36, 2008, Seiten 11328-11342, XP002729016, ISSN: 0947-6539, DOI: 10.1002/CHEM.200801428
- PORIEL, CYRIL ET AL: "Incorporation of Spiroxanthene Units in Blue-Emitting Oligophenylene Frameworks: A New Molecular Design for OLED Applications", CHEMISTRY - A EUROPEAN JOURNAL, Bd. 17, Nr. 45, 2011, Seiten 12631-12645, XP002729017, ISSN: 0947-6539, DOI: 10.1002/CHEM.201100790
- JIANG, ZUOQUAN ET AL: "Bent ladder-type hexaphenylene with carbazole core and spiro linkage as stable and efficient blue emitter", ORGANIC LETTERS, Bd. 11, Nr. 18, 2009, Seiten 4132-4135, XP002729018, ISSN: 1523-7060, DOI: 10.1021/OL901635V
- DATABASE CA [Online] CHEMICAL ABSTRACTS SERVICE, COLUMBUS, OHIO, US; KIM, BOK YEONG ET AL: "Substituted carbazole-based organic light compound as an electroluminescent material or/and electron/hole transport material for organic light device", XP002684350, gefunden im STN Database accession no. 2012:893981
- DATABASE CA [Online] CHEMICAL ABSTRACTS SERVICE, COLUMBUS, OHIO, US; WANG, CHANGSHENG ET AL: "Synthesis, characterization, and OFET and OLED properties of .pi.-extended ladder-type heteroacenes based on indolodibenzothiophene", XP002729020, gefunden im STN Database accession no. 2012:350021

## Beschreibung

Die vorliegende Anmeldung betrifft eine Verbindung der untenstehenden Formel (I) sowie ihre Verwendung in elektronischen Vorrichtungen, inbesondere in organischen elektronischen Vorrichtungen (OLEDs). Weiterhin betrifft die Erfindung bestimmte Ausführungsformen von elektronischen Vorrichtungen enthaltend die Verbindung der Formel (I), sowie Verfahren zur Herstellung der Verbindung der Formel (I).

Unter dem Begriff elektronische Vorrichtung werden gemäß der vorliegenden Erfindung allgemein elektronische Vorrichtungen verstanden, welche organische Materialien enthalten. Bevorzugt werden darunter OLEDs verstanden.

Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten der elektronischen Vorrichtungen sind weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der elektronischen Vorrichtungen sowie die realisierten Farbwerte. Insbesondere bei blau emittierenden OLEDs besteht Verbesserungspotential bezüglich der Lebensdauer der Vorrichtungen und den erreichten Farbwerten des emittierten Lichts.

Ein wichtiger Ansatzpunkt, um die genannten Verbesserungen zu erreichen, ist die Auswahl der emittierenden Verbindung, die in der elektronischen Vorrichtung eingesetzt wird. Im Stand der Technik sind als blaues Licht emittierende Verbindungen eine Vielzahl von Verbindungen beschrieben, insbesondere Arylamine mit Indenofluoren-Grundgerüst. Beispiele dafür sind Benzoindenofluorenamine, z. B. gemäß WO 2008/006449 und WO 2007/140847.

Im Stand der Technik sind als Verbindungen für die emittierende Schicht, welche nicht Licht emittieren (Matrixverbindungen), unter anderem Anthracenverbindungen, wie beispielsweise in WO 2008/145239 und WO 2009/100925 beschrieben, bekannt. Weiterhin sind für diese Verwendung Benzindenofluoren-Verbindungen beschrieben, beispielsweise in den oben genannten Anmeldungen WO 2008/006449 und WO 2007/140847.
Als Verbindungen zur Verwendung in lochtransportierenden oder lochinjizierenden Schichten sind im Stand der Technik unter anderem ebenfalls Verbindungen mit Indenofluoren-Grundgerüst beschrieben, beispielsweise in WO 2006/108497, WO 2006/122630, und WO 2009/141026. Weitere Verbindungen für OLEDs sind bekannt von; N. Cocherel et al., Chemistry - A European Journal, 14(36), 11328-11342.

Es besteht jedoch weiterhin Bedarf an neuen Verbindungen zur Verwendung in elektronischen Vorrichtungen. Insbesondere besteht Bedarf an Verbindungen, mit denen hervorragende Leistungsdaten für die elektronischen Vorrichtungen erreicht werden können. Hervorzuheben sind dabei besonders niedrige Betriebsspannung, lange Lebensdauer, hohe Leistungseffizienz und geeignete Farbkoordinaten des emittierten Lichts.

In Untersuchungen zu neuen Verbindungen zur Verwendung in elektronischen Vorrichtungen wurde nun unerwartet gefunden, dass Verbindungen einer Formel (I) mit einem Bis-Indenofluoren-Grundgerüst hervorragend zur Verwendung in elektronischen Vorrichtungen geeignet sind.

Bevorzugt weisen diese Verbindungen eine oder mehrere Eigenschaften gewählt aus niedriger Betriebsspannung, langer Lebensdauer, hoher Leistungseffizienz und geeigneten Farbkoordinaten des emittierten Lichts auf. Insbesondere ermöglichen sie bei Verwendung in der emittierenden Schicht eine hervorragende Lebensdauer in Kombination mit tiefblauen Farbkoordinaten des emittierten Lichts.

Gegenstand der Erfindung ist somit eine Verbindung gemäß einer Formel (I) 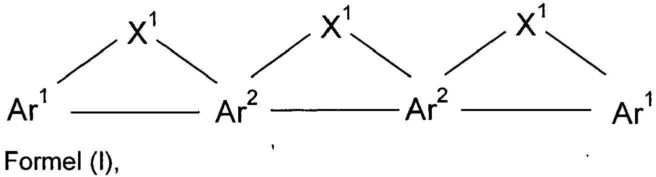 wobei gilt:
- Ar¹: ist bei jedem Auftreten gleich oder verschieden eine Aryl- oder Heteroarylgruppe mit 6 bis 18 aromatischen Ringatomen, die mit einem oder mehreren Resten R¹ substituiert sein kann;
- Ar²: ist bei jedem Auftreten gleich oder verschieden eine Aryl- oder Heteroarylgruppe mit 6 aromatischen Ringatomen, die mit einem oder mehreren Resten R² substituiert sein kann;
- X¹: ist bei jedem Auftreten gleich oder verschieden BR³, C(R³)₂, -C(R³)₂-C(R³)₂-, -C(R³)₂-O-, -C(R³)₂-S-,-R³C=CR³-, -R³C=N-, Si(R³)₂, -Si(R³)₂-Si(R³)₂-, C=O, O, S, S=O, SO₂, NR³, PR³ oder P(=O)R³;
- R¹, R², R³: ist bei jedem Auftreten gleich oder verschieden H, D, F, Cl, Br, I, C(=O)R⁴, CN, Si(R⁴)₃, N(R⁴)₂, P(=O)(R⁴)₂, OR⁴, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R³ miteinander verknüpft sein können und einen Ring bilden können;
- R⁴: ist bei jedem Auftreten gleich oder verschieden H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁵ substituiert sein kann, wobei zwei oder mehr Reste R⁴ miteinander verknüpft sein können und einen Ring bilden können;
- R⁵: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁵ miteinander verknüpft sein und einen Ring bilden;
wobei mindestens eine der beiden Gruppen Ar¹ 10 oder mehr aromatische Ringatome aufweisen muss; und
wobei wenn eine der beiden Gruppen Ar¹ eine Phenylgruppe ist, die andere der beiden Gruppen Ar¹ nicht mehr als 14 aromatische Ringatome aufweisen darf.

Für die Formel (I) gilt, dass die Bindungen zu benachbarten Gruppen Ar¹ bzw. Ar² und zu Gruppen X¹ jeweils an beliebigen Positionen der Gruppen Ar² bzw. Ar¹ vorliegen können. Insbesondere wird durch die Darstellung der Formel (I) nicht impliziert, dass die Gruppen X¹ in cis-Stellung zueinander vorliegen müssen. Die Gruppen X¹ können in cis- oder in trans-Stellung zueinander vorliegen.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt ist, dass an den Gruppen Ar² die Bindungen zur benachbarten Gruppe Ar¹ bzw. Ar² jeweils in para-Position zueinander vorliegen.

Es ist bevorzugt, dass Ar¹ bei jedem Auftreten gleich oder verschieden gewählt ist aus Arylgruppen oder Heteroarylgruppen mit 6 bis 14 aromatischen Ringatomen, besonders bevorzugt 6 bis 10 aromatischen Ringatomen, wobei Ar¹ jeweils mit einem oder mehreren Resten R¹ substituiert sein kann.

Es ist bevorzugt, dass Ar¹ bei jedem Auftreten gleich oder verschieden gewählt ist aus Arylgruppen mit 6 bis 14 aromatischen Ringatomen, besonders bevorzugt 6 bis 10 aromatischen Ringatomen, wobei Ar¹ jeweils mit einem oder mehreren Resten R¹ substituiert sein kann.

Es ist bevorzugt, dass die Gruppen Ar² Phenylgruppen sind, die mit einem oder mehreren Resten R² substituiert sein können.

Gemäß einer besonders bevorzugten Ausführungsform sind die Gruppen Ar¹ Naphthylgruppen, die mit einem oder mehreren Resten R¹ substituiert sein können, und die Gruppen Ar² sind Phenylgruppen, die mit einem oder mehreren Resten R² substituiert sein können.

Gemäß einer alternativen besonders bevorzugten Ausführungsform ist eine der beiden Gruppen Ar¹ eine Phenylgruppe, die mit einem oder mehreren Resten R¹ substituiert sein kann, und die andere der beiden Gruppen Ar¹ ist eine Naphthylgruppe, die mit einem oder mehreren Resten R¹ substituiert sein kann, und die Gruppen Ar² sind Phenylgruppen, die mit einem oder mehreren Resten R² substituiert sein können.

Es ist bevorzugt, dass X¹ bei jedem Auftreten gleich oder verschieden gewählt ist aus C(R³)₂, -C(R³)₂-C(R³)₂-, -C(R³)₂-O-, -R³C=CR³-, Si(R³)₂, C=O, O, S, S=O, SO₂ und NR³, besonders bevorzugt C(R³)₂, -C(R³)₂-C(R³)₂-, -C(R³)₂-O-, Si(R³)₂, O, S, und NR³, ganz besonders bevorzugt C(R³)₂.

Bevorzugt sind R¹ und R² bei jedem Auftreten gleich oder verschieden H, D, F, CN, Si(R⁴)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, -NR⁴-, -O- oder -S- ersetzt sein können, oder ein aromatisches oder heteroaromatisches Ringsystem mit 5 bis 20 aromatischen Ringatomen, das jeweils mit einem oder mehreren Resten R⁴ substituiert sein kann.

Besonders bevorzugt ist R² gleich H oder D, besonders bevorzugt gleich H.

Bevorzugt ist R³ bei jedem Auftreten gleich oder verschieden F, CN, Si(R⁴)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, -NR⁴-, -O- oder -S- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

Gemäß einer bevorzugten Ausführungsform bilden zwei Reste R³, die Bestandteil einer Gruppe X¹ sind, die C(R³)₂ oder Si(R³)₂ darstellt, miteinander einen Ring, so dass eine Spiro-Verbindung entsteht. Bevorzugt wird dabei ein Fünf- oder ein Sechsring gebildet. Weiterhin ist es in diesem Fall bevorzugt, dass die Reste R³ Alkylgruppen darstellen, so dass ein spirocyclischer Alkylring gebildet wird, besonders bevorzugt ein Spiro-Cyclohexanring oder ein Spiro-Cyclopentanring.

Bevorzugt ist R⁴ bei jedem Auftreten gleich oder verschieden F, CN, Si(R⁵)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁵ substituiert sein können und wobei in den oben genannten Gruppen eine oder mehrere CH₂-Gruppen durch -C=C-, -R⁵C=CR⁵-, Si(R⁵)₂, C=O, -NR⁵-, -O- oder -S- ersetzt sein können, oder ein aromatisches oder heteroaromatisches Ringsystem mit 5 bis 20 aromatischen Ringatomen, das jeweils mit einem oder mehreren Resten R⁵ substituiert sein kann, wobei zwei oder mehr Reste R⁴ miteinander verknüpft sein können und einen Ring bilden können.

Gemäß einer bevorzugten Ausführungsform der Erfindung sind alle Gruppen R¹ und R² in Formel (I) gleich H oder D, besonders bevorzugt gleich H.

Gemäß einer weiteren bevorzugten Ausführungsform der Erfindung sind eine oder mehrere Gruppen R¹ gleich CN, besonders bevorzugt genau zwei Gruppen R¹ gleich CN.

Gemäß einer weiteren bevorzugten Ausführungsform der Erfindung sind eine oder mehrere Gruppen R¹ gleich einem aromatischen oder heteroaromatischen Ringsystem mit 6 bis 20 aromatischen Ringatomen, das mit einem oder mehreren Resten R⁴ substituiert sein kann, besonders bevorzugt genau zwei Gruppen R¹ gleich einem aromatischen oder heteroaromatischen Ringsystem mit 6 bis 20 aromatischen Ringatomen, das mit einem oder mehreren Resten R⁴ substituiert sein kann.

Gemäß einer besonders bevorzugten Ausführungsform ist R¹ eine Benzindenofluorengruppe, besonders bevorzugt eine Monobenzindenofluorengruppe, welche jeweils mit Resten R⁴ substituiert sein kann.

Gemäß einer weiteren bevorzugten Ausführungsform der Erfindung sind keine der Gruppen R¹ und R² Gruppen der Formel N(R⁴)₂. In diesem Fall sind bevorzugt die Gruppen X¹ nicht gleich NR³, besonders bevorzugt sind die Gruppen X¹ in diesem Fall gleich C(R³)₂.

Gemäß einer weiteren bevorzugten Ausführungsform der Erfindung sind eine oder mehrere Gruppen R¹ gleich N(R⁴)₂, besonders bevorzugt genau zwei Gruppen R¹ gleich N(R⁴)₂.

Eine bevorzugte Ausführungsform der Verbindung entspricht der Formel (I-1) 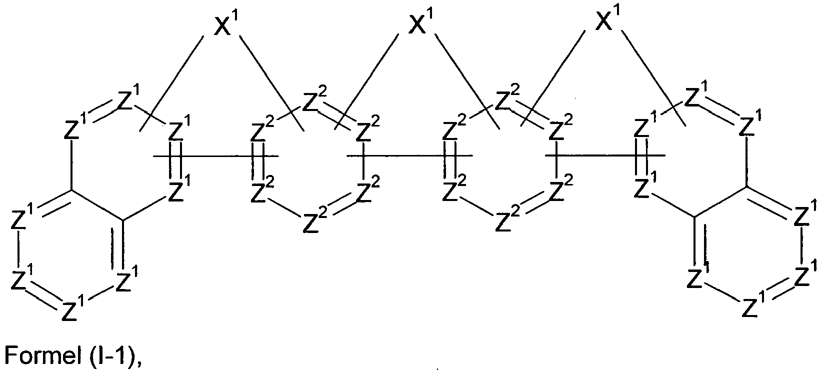 wobei gilt:
- Z¹: ist bei jedem Auftreten gleich oder verschieden CR¹ oder N, wobei Z¹ gleich C ist, wenn eine Gruppe gebunden ist;
- Z²: ist bei jedem Auftreten gleich oder verschieden CR² oder N, wobei Z² gleich C ist, wenn eine Gruppe gebunden ist; und
die Gruppen X¹ sind definiert wie oben.

Eine alternative bevorzugte Ausführungsform der Verbindung entspricht der Formel (I-2) 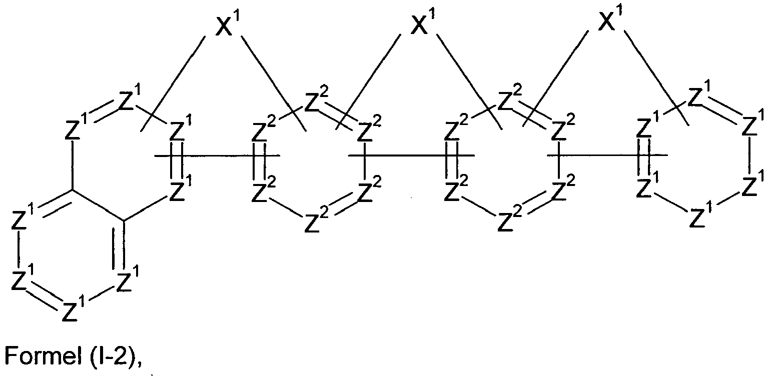 wobei gilt:
- Z¹: ist bei jedem Auftreten gleich oder verschieden CR¹ oder N, wobei Z¹ gleich C ist, wenn eine Gruppe gebunden ist;
- Z²: ist bei jedem Auftreten gleich oder verschieden CR² oder N, wobei Z² gleich C ist, wenn eine Gruppe gebunden ist; und
die Gruppen X¹ sind definiert wie oben.

Für die Formel (I-1) und (I-2) gilt, dass die Bindungen zu Gruppen X¹ und die Bindungen zwischen den aromatischen Ringen jeweils an beliebigen Positionen der aromatischen Ringe vorliegen können, ebenso wie die Bindungen zwischen den einzelnen aromatischen Ringen. Insbesondere wird durch die Darstellung der Formeln (I-1) und (I-2) nicht impliziert, dass die Gruppen X¹ in cis-Stellung zueinander vorliegen müssen. Die Gruppen X¹ können in cis- oder in trans-Stellung zueinander vorliegen.

Es ist bevorzugt, dass maximal zwei Gruppen Z¹ pro aromatischem Ring gleich N sind, besonders bevorzugt maximal eine Gruppe Z¹ pro aromatischem Ring gleich N ist, und ganz besonders bevorzugt keine Gruppe Z¹ in einem aromatischen Ring gleich N ist.

Allgemein bevorzugt ist, dass Z¹ gleich CR¹ ist.

Es ist bevorzugt, dass maximal zwei Gruppen Z² pro aromatischem Ring gleich N sind, besonders bevorzugt maximal eine Gruppe Z² pro aromatischem Ring gleich N ist, und ganz besonders bevorzugt keine Gruppe Z² in einem aromatischen Ring gleich N ist.

Allgemein bevorzugt ist, dass Z² gleich CR² ist.

Bevorzugte Ausführungsformen der Formel (I-1) entsprechen den folgenden Formeln (I-1-1) bis (I-1-11) und (I-2-1) bis (I-2-8)

| |
|---|
| 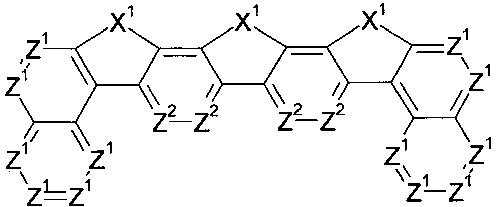 |
| Formel (I-1-1) |
| 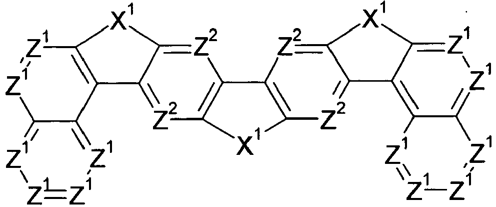 |
| Formel (I-1-2) |
| 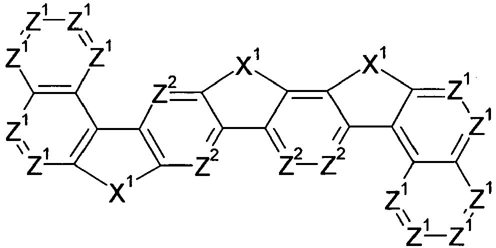 |
| Formel (I-1-3) |
| 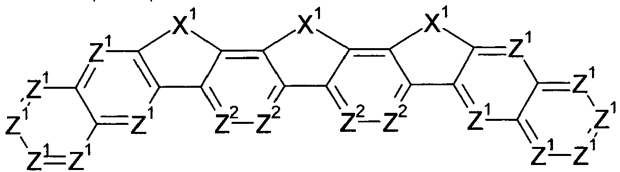 |
| Formel (I-1-4) |
| 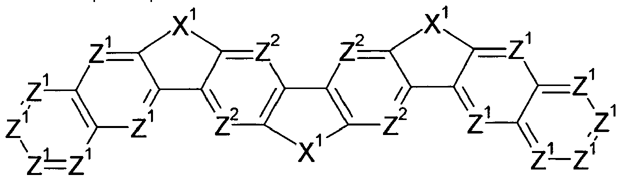 |
| Formel (I-1-5) |
| 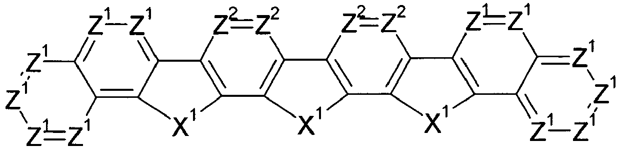 |
| Formel (I-1-6) |
| 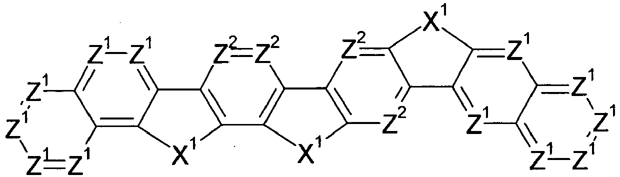 |
| Formel (I-1-7) |
| 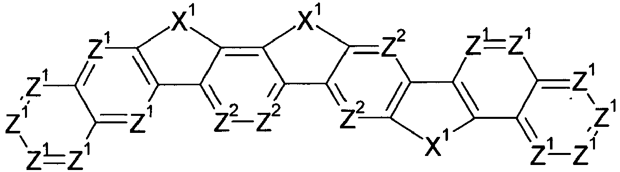 |
| Formel (I-1-8) |
| 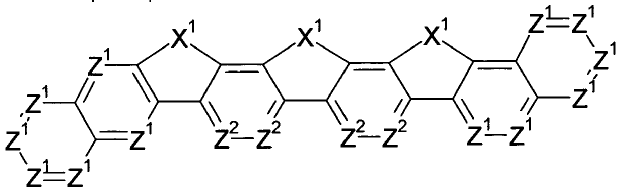 |
| Formel (I-1-9) |
| 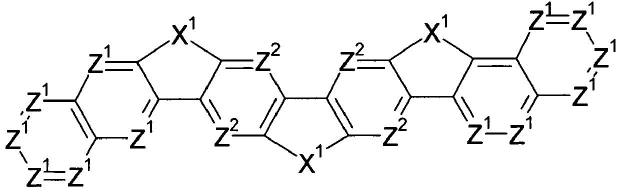 |
| Formel (I-1-10) |
| 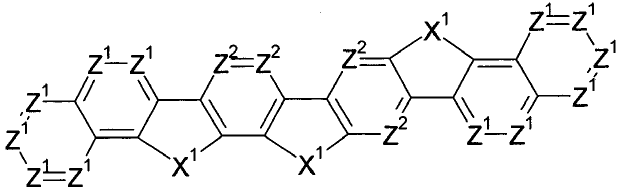 |
| Formel (I-1-11) |
| 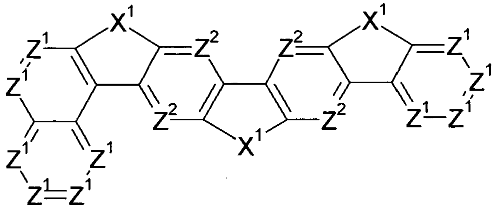 |
| Formel (I-2-1) |
| 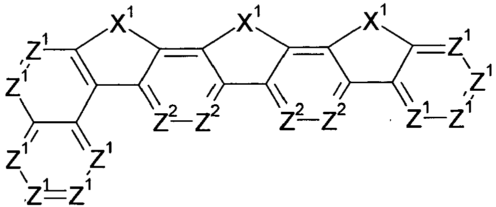 |
| Formel (I-2-2) |
| 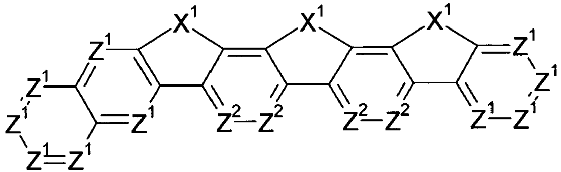 |
| Formel (I-2-3) |
| 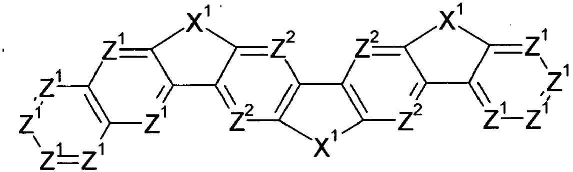 |
| Formel (I-2-4) |
| 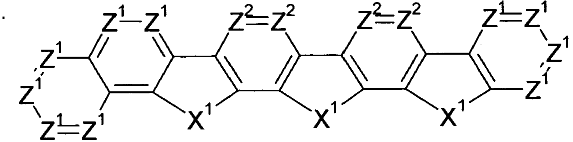 |
| Formel (I-2-5) |
| 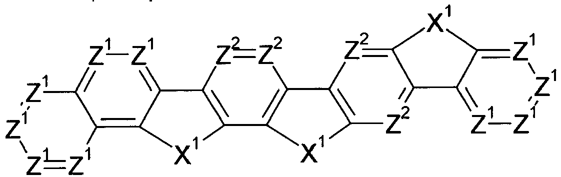 |
| Formel (I-2-6) |
| 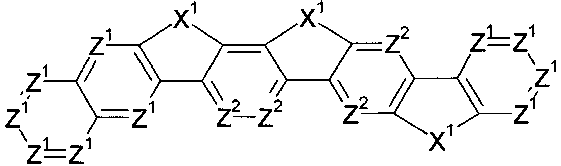 |
| Formel (I-2-7) |
| 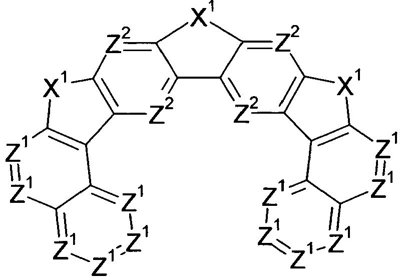 |
| Formel (I-2-8) |

wobei gilt:
- Z¹: ist bei jedem Auftreten gleich oder verschieden CR¹ oder N;
- Z²: ist bei jedem Auftreten gleich oder verschieden CR² oder N; und
die Gruppen X¹ sind definiert wie oben.

Insbesondere für die Gruppen Z¹, Z² und X¹ sind die oben angegebenen bevorzugten Ausführungsformen auch für die obenstehenden Formeln bevorzugt.

Nochmals insbesondere bevorzugt ist in den Formeln (1-1-1) bis (1-1-11) und (I-2-1) bis (I-2-8) Z¹ gleich CR¹, Z² ist gleich CR², und X¹ ist gleich C(R³)₂.

Unter den Formeln (I-1-1) bis (I-1-11) und (I-2-1) bis (I-2-8) sind die Formeln (I-1-2), (I-2-1) und (I-2-8) besonders bevorzugt, am stärksten bevorzugt ist die Formel (I-1-2).

Bevorzugt entsprechen Verbindungen der Formel (I) einer der Formeln (I-1-2-1) und (I-2-1-1)

| |
|---|
| 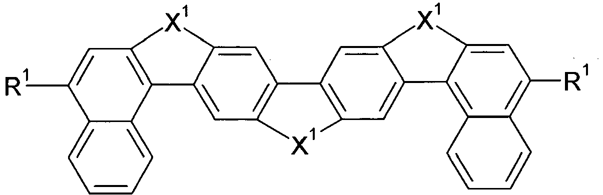 |
| Formel (I-1-2-1) |
| |
| 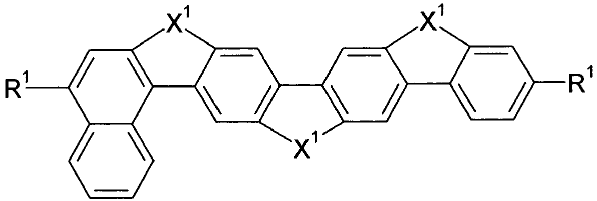 |
| Formel (I-2-1-1) |

wobei X¹ und R¹ wie oben definiert ist.

Bevorzugt ist X¹ in den Formeln (1-1-2-1) und (1-2-1-1) bei jedem Auftreten gleich oder verschieden gewählt aus C(R³)₂, -C(R³)₂-C(R³)₂-, -C(R³)₂-O-, Si(R³)₂, O, S, und NR³, besonders bevorzugt ist X¹ gleich C(R³)₂.

Bevorzugt ist R¹ in den Formeln (I-1-2-1) und (I-2-1-1) bei jedem Auftreten gleich oder verschieden H, D, F, CN, Si(R⁴)₃, oder ein aromatisches oder heteroaromatisches Ringsystem mit 5 bis 20 aromatischen Ringatomen, das jeweils mit einem oder mehreren Resten R⁴ substituiert sein kann.

Gemäß einer besonders bevorzugten Ausführungsform ist R¹ in den Formeln (I-1-2-1) und (I-2-1-1) eine Benzindenofluorengruppe, besonders bevorzugt eine Monobenzindenofluorengruppe, welche jeweils mit Resten R⁴ substituiert sein kann.

Ganz besonders bevorzugte Ausführungsformen der Verbindungen der Formel (I) entsprechen den folgenden Formeln, wobei bevorzugt gilt: Z¹ ist CR¹ und Z² ist CR²:

| | Grundstruktur gemäß | X¹ (links) | X¹ (Mitte) | X¹ (rechts) |
|---|---|---|---|---|
| 1 | Formel (I-1-1) | C(R³)₂ | C(R³)₂ | C(R³)₂ |
| 2 | " | " | -C(R³)₂-C(R³)₂- | " |
| 3 | " | " | -C(R³)₂-O- | " |
| 4 | " | " | Si(R³)₂ | " |
| 5 | " | " | O | " |
| 6 | " | " | S | " |
| 7 | " | " | NR³ | " |
| 8 | Formel (I-1-2) | C(R³)₂ | C(R³)₂ | C(R³)₂ |
| 9 | " | " | -C(R³)₂-C(R³)₂- | " |
| 10 | " | " | -C(R³)₂-O- | " |
| 11 | " | " | Si(R³)₂ | " |
| 12 | " | " | O | " |
| 13 | " | " | S | " |
| 14 | " | " | NR³ | " |
| 15 | Formel (I-1-3) | C(R³)₂ | C(R³)₂ | C(R³)₂ |
| 16 | " | " | -C(R³)₂-C(R³)₂- | " |
| 17 | " | " | -C(R³)₂-O- | " |
| 18 | " | " | Si(R³)₂ | " |
| 19 | " | " | O | " |
| 20 | " | " | S | " |
| 21 | " | " | NR³ | " |
| 22 | Formel (I-1-4) | C(R³)₂ | C(R³)₂ | C(R³)₂ |
| 23 | " | " | -C(R³)₂-C(R³)₂- | " |
| 24 | " | " | -C(R³)₂-O- | " |
| 25 | " | " | Si(R³)₂ | " |
| 26 | " | " | O | " |
| 27 | " | " | S | " |
| 28 | " | " | NR³ | " |
| 29 | Formel (I-1-5) | C(R³)₂ | C(R³)₂ | C(R³)₂ |
| 30 | " | " | -C(R³)₂-C(R³)₂- | " |
| 31 | " | " | -C(R³)₂-O- | " |
| 32 | " | " | Si(R³)₂ | " |
| 33 | " | " | O | " |
| 34 | " | " | S | " |
| 35 | " | " | NR³ | " |
| 36 | Formel (I-1-6) | C(R³)₂ | C(R³)₂ | C(R³)₂ |
| 37 | " | " | -C(R³)₂-C(R³)₂- | " |
| 38 | " | " | -C(R³)₂-O- | " |
| 39 | " | " | Si(R³)₂ | " |
| 40 | " | " | O | " |
| 41 | " | " | S | " |
| 42 | " | " | NR³ | " |
| 43 | Formel (I-1-7) | C(R³)₂ | C(R³)₂ | C(R³)₂ |
| 44 | " | " | -C(R³)₂-C(R³)₂- | " |
| 45 | " | " | -C(R³)₂-O- | " |
| 46 | " | " | Si(R³)₂ | " |
| 47 | " | " | O | " |
| 48 | " | " | S | " |
| 49 | " | " | NR³ | " |
| 50 | Formel (I-1-8) | C(R³)₂ | C(R³)₂ | C(R³)₂ |
| 51 | " | " | -C(R³)₂-C(R³)₂- | " |
| 52 | " | " | -C(R³)₂-O- | " |
| 53 | " | " | Si(R³)₂ | " |
| 54 | " | " | O | " |
| 55 | " | " | S | " |
| 56 | " | " | NR³ | " |
| 57 | Formel (I-1-9) | C(R³)₂ | C(R³)₂ | C(R³)₂ |
| 58 | " | " | -C(R³)₂-C(R³)₂- | " |
| 59 | " | " | -C(R³)₂-O- | " |
| 60 | " | " | Si(R³)₂ | " |
| 61 | " | " | O | " |
| 62 | " | " | S | " |
| 63 | " | " | NR³ | " |
| 64 | Formel (I-1-10) | C(R³)₂ | C(R³)₂ | C(R³)₂ |
| 65 | " | " | -C(R³)₂-C(R³)₂- | " |
| 66 | " | " | -C(R³)₂-O- | " |
| 67 | " | " | Si(R³)₂ | " |
| 68 | " | " | O | " |
| 69 | " | " | S | " |
| 70 | " | " | NR³ | " |
| 71 | Formel (I-1-11) | C(R³)₂ | C(R³)₂ | C(R³)₂ |
| 72 | " | " | -C(R³)₂-C(R³)₂- | " |
| 73 | " | " | -C(R³)₂-O- | " |
| 74 | " | " | Si(R³)₂ | " |
| 75 | " | " | O | " |
| 76 | " | " | S | " |
| 77 | " | " | NR³ | " |
| 78 | Formel (I-2-1) | C(R³)₂ | C(R³)₂ | C(R³)₂ |
| 79 | " | " | -C(R³)₂-C(R³)₂- | " |
| 80 | " | " | -C(R³)₂-O- | " |
| 81 | " | " | Si(R³)₂ | " |
| 82 | " | " | O | " |
| 83 | " | " | S | " |
| 84 | " | " | NR³ | " |
| 85 | Formel (I-2-2) | C(R³)₂ | C(R³)₂ | C(R³)₂ |
| 86 | " | " | -C(R³)₂-C(R³)₂- | " |
| 87 | " | " | -C(R³)₂-O- | " |
| 88 | " | " | Si(R³)₂ | " |
| 89 | " | " | O | " |
| 90 | " | " | S | " |
| 91 | " | " | NR³ | " |
| 92 | Formel (I-2-3) | C(R³)₂ | C(R³)₂ | C(R³)₂ |
| 93 | " | " | -C(R³)₂-C(R³)₂- | " |
| 94 | " | " | -C(R³)₂-O- | " |
| 95 | " | " | Si(R³)₂ | " |
| 96 | " | " | O | " |
| 97 | " | " | S | " |
| 98 | " | " | NR³ | " |
| 99 | Formel (I-2-4) | C(R³)₂ | C(R³)₂ | C(R³)₂ |
| 100 | " | " | -C(R³)₂-C(R³)₂- | " |
| 101 | " | " | -C(R³)₂-O- | " |
| 102 | " | " | Si(R³)₂ | " |
| 103 | " | " | O | " |
| 104 | " | " | S | " |
| 105 | " | " | NR³ | " |
| 106 | Formel (I-2-5) | C(R³)₂ | C(R³)₂ | C(R³)₂ |
| 107 | " | " | -C(R³)₂-C(R³)₂- | " |
| 108 | " | " | -C(R³)₂-O- | " |
| 109 | " | " | Si(R³)₂ | " |
| 110 | " | " | O | " |
| 111 | " | " | S | " |
| 112 | " | " | NR³ | " |
| 113 | Formel (I-2-6) | C(R³)₂ | C(R³)₂ | C(R³)₂ |
| 114 | " | " | -C(R³)₂-C(R³⁾₂- | " |
| 115 | " | " | -C(R³)₂-O- | " |
| 116 | " | " | Si(R³)₂ | " |
| 117 | " | " | O | " |
| 118 | " | " | S | " |
| 119 | " | " | NR³ | " |
| 120 | Formel (I-2-7) | C(R³)₂ | C(R³)₂ | C(R³)₂ |
| 121 | " | " | -C(R³)₂-C(R³)₂- | " |
| 122 | " | " | -C(R³)₂-O- | " |
| 123 | " | " | Si(R³)₂ | " |
| 124 | " | " | O | " |
| 125 | " | " | S | " |
| 126 | " | " | NR³ | " |
| 127 | Formel (I-2-8) | C(R³)₂ | C(R³)₂ | C(R³)₂ |
| 128 | " | " | -C(R³)₂-C(R³)₂- | " |
| 129 | " | " | -C(R³)₂-O- | " |
| 130 | " | " | Si(R³)₂ | " |
| 131 | " | " | O | " |
| 132 | " | " | S | " |
| 133 | " | " | NR³ | " |

Folgende Verbindungen sind Beispiele für Verbindungen der Formel (I):

| | |
|---|---|
| 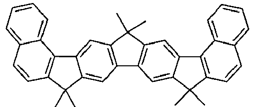 | 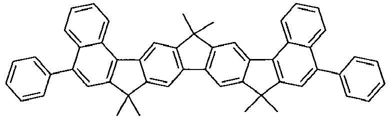 |
| 1 | 2 |
| 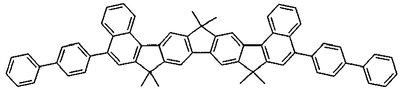 | 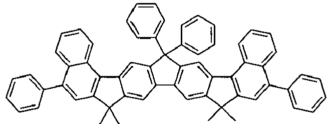 |
| 3 | 4 |
| 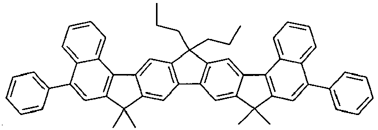 | 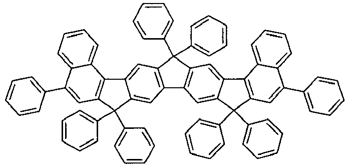 |
| 5 | 6 |
| 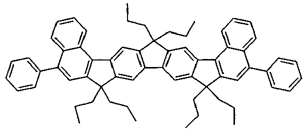 | 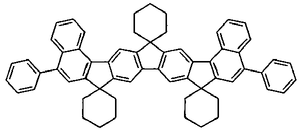 |
| 7 | 8 |
| 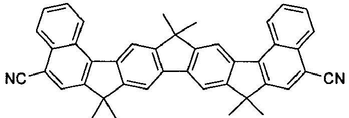 | 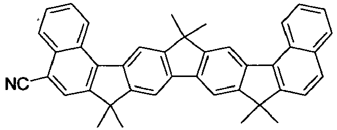 |
| 9 | 10 |
| 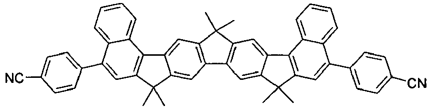 | 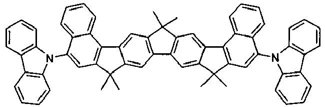 |
| 11 | 12 |
| 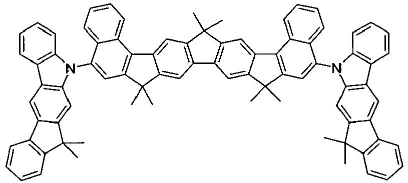 | 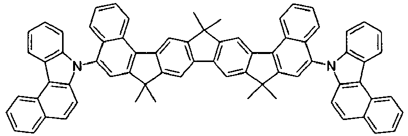 |
| 13 | 14 |
| 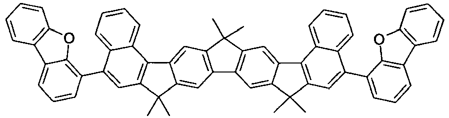 | 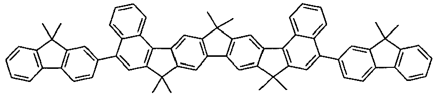 |
| 15 | 16 |
| 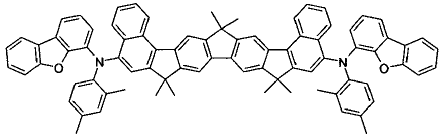 | 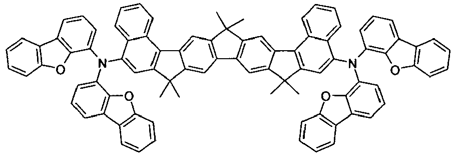 |
| 17 | 18 |
| 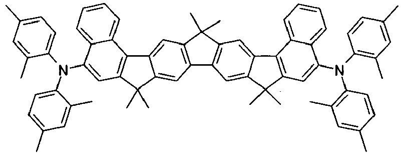 | 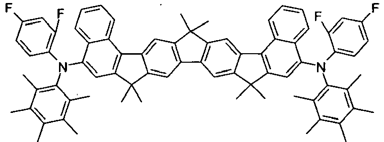 |
| 19 | 20 |
| 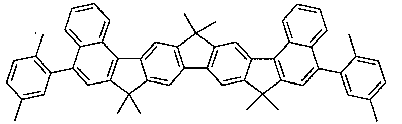 | 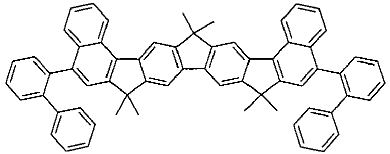 |
| 21 | 22 |
| 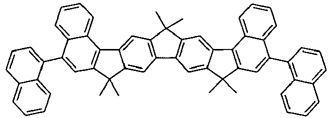 | 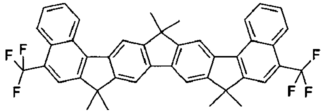 |
| 23 | 24 |
| 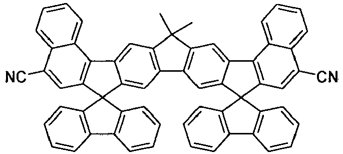 | 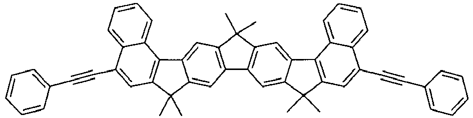 |
| 25 | 26 |
| 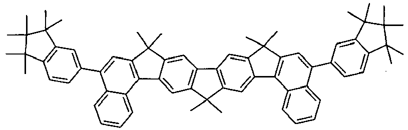 | 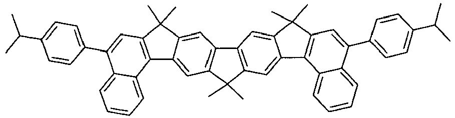 |
| 27 | 28 |
| 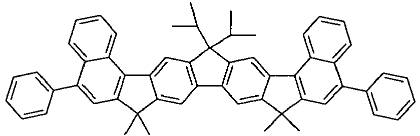 | 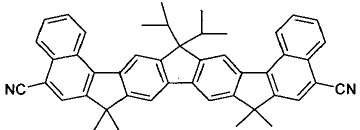 |
| 29 | 30 |
| 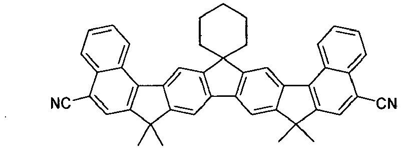 | 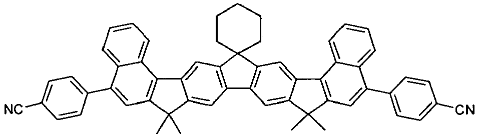 |
| 31 | 32 |
| 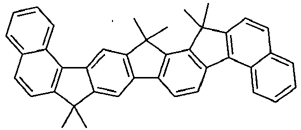 | 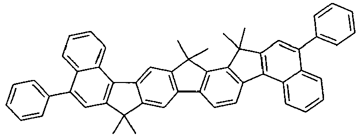 |
| 33 | 34 |
| 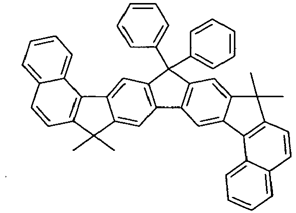 | 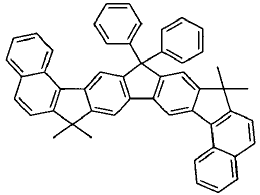 |
| 35 | 36 |
| 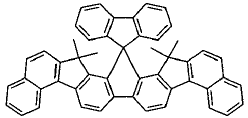 | 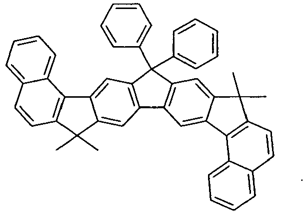 |
| 37 | 38 |
| 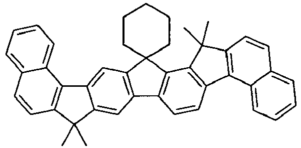 | 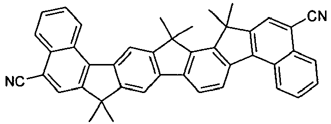 |
| 39 | 40 |
| 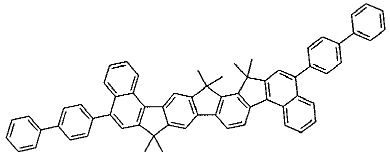 | 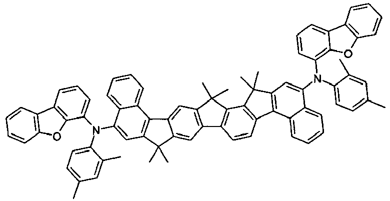 |
| 41 | 42 |
| 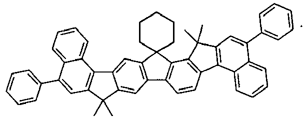 | 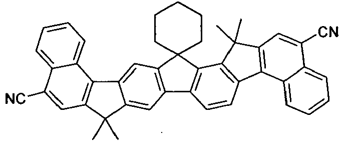 |
| 43 | 44 |
| 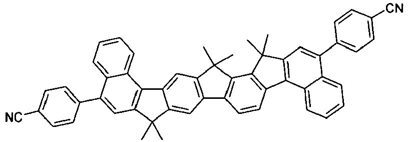 | 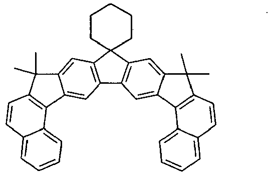 |
| 45 | 46 |
| 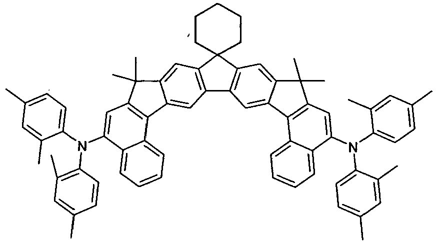 | 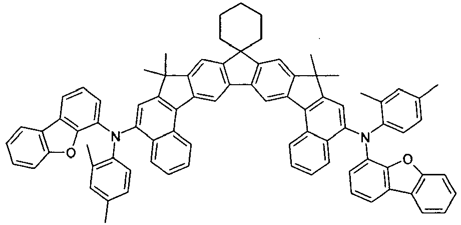 |
| 47 | 48 |
| 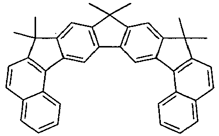 | 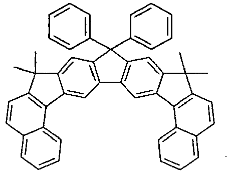 |
| 49 | 50 |
| 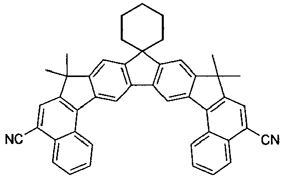 | 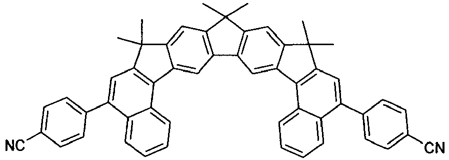 |
| 51 | 52 |
| 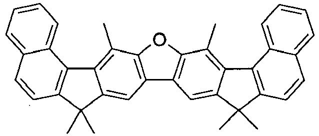 | 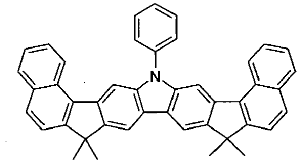 |
| 53 | 54 |
| 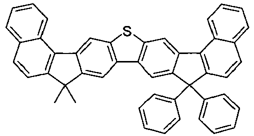 | 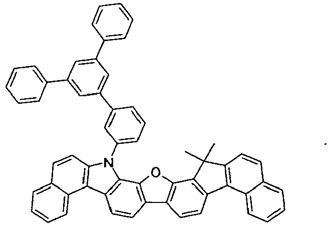 |
| 55 | 56 |
| 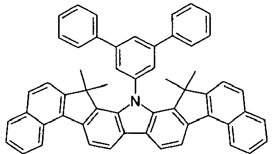 | 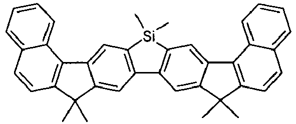 |
| 57 | 58 |
| 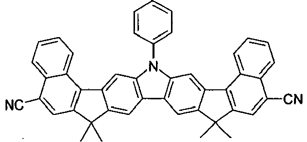 | 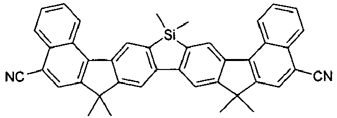 |
| 59 | 60 |
| 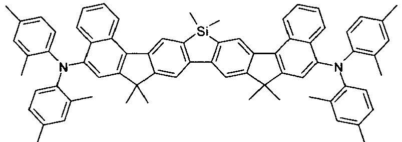 | 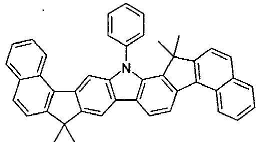 |
| 61 | 62 |
| 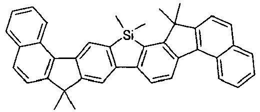 | 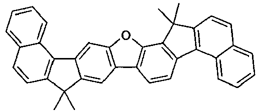 |
| 63 | 64 |
| 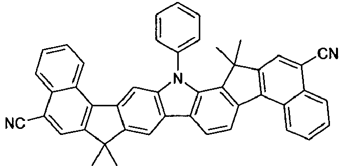 | 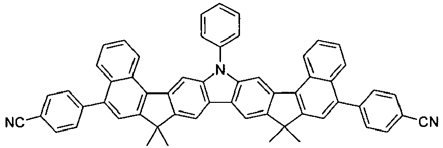 |
| 65 | 66 |
| 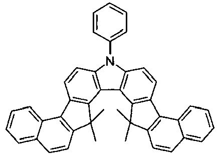 | 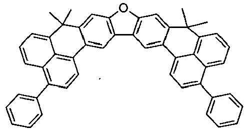 |
| 67 | 68 |
| 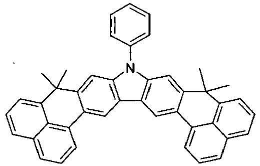 | 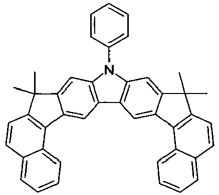 |
| 69 | 70 |
| 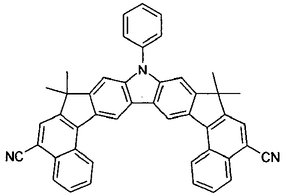 | 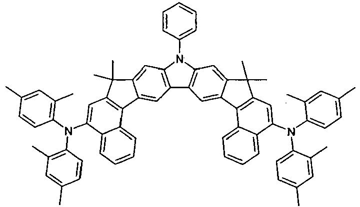 |
| 71 | 72 |
| 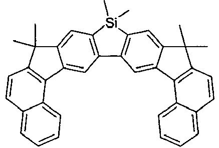 | 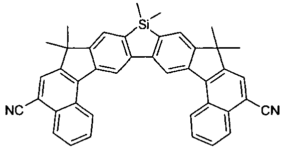 |
| 73 | 74 |
| 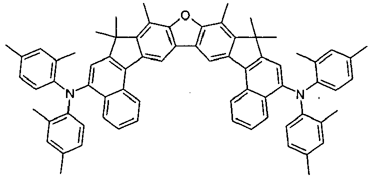 | 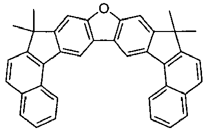 |
| 75 | 76 |
| 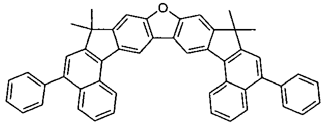 | 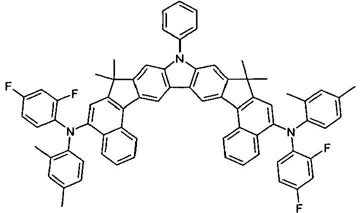 |
| 77 | 78 |
| 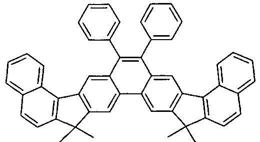 | 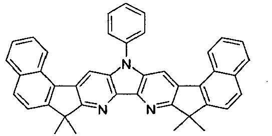 |
| 79 | 80 |
| 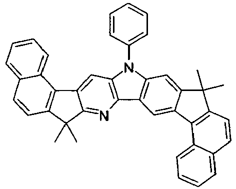 | 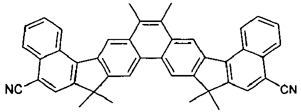 |
| 81 | 82 |
| 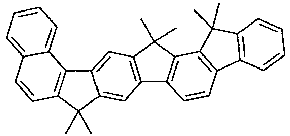 | 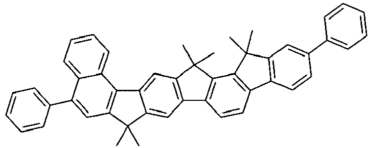 |
| 83 | 84 |
| 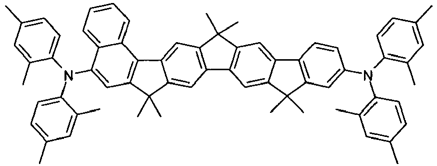 | 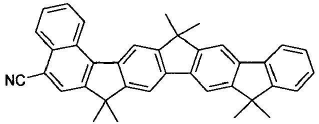 |
| 85 | 86 |
| 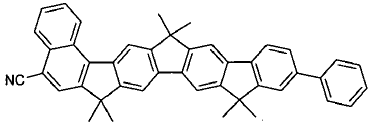 | 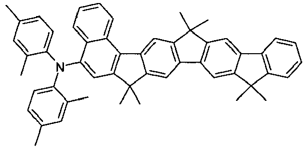 |
| 87 | 88 |
| 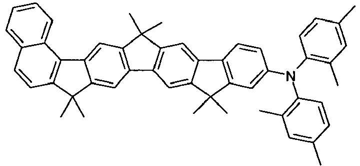 | 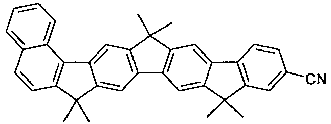 |
| 89 | 90 |
| 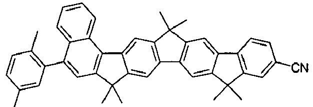 | 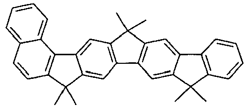 |
| 91 | 92 |
| 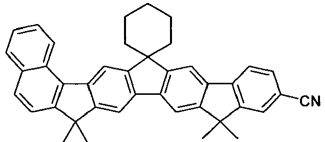 | 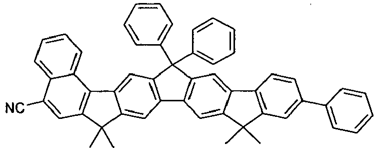 |
| 93 | 94 |
| 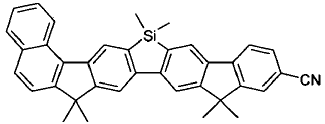 | 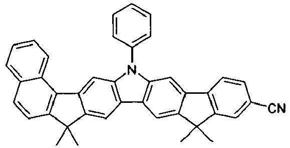 |
| 95 | 96 |
| 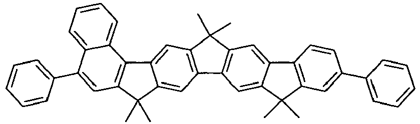 | 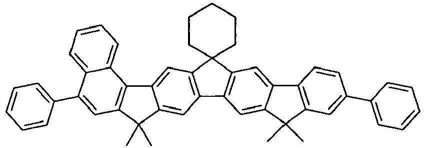 |
| 97 | 98 |
| 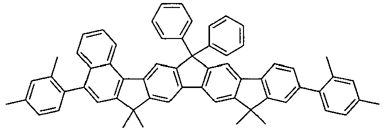 | 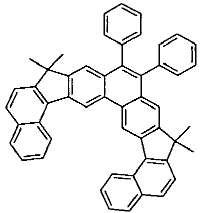 |
| 99 | 100 |
| 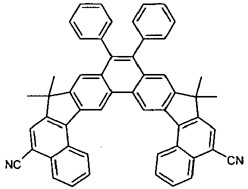 | 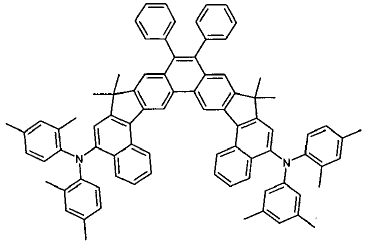 |
| 101 | 102 |
| 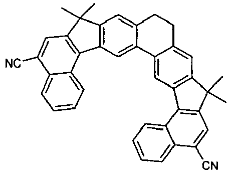 | 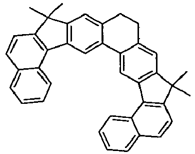 |
| 103 | 104 |
| 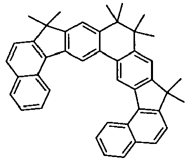 | 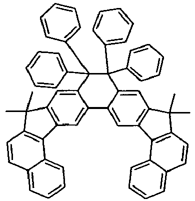 |
| 105 | 106 |
| 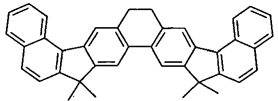 | 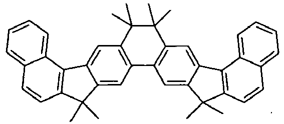 |
| 107 | 108 |
| 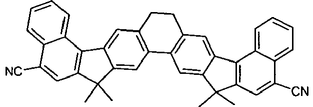 | 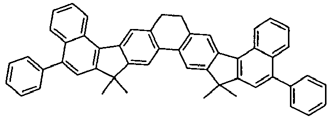 |
| 109 | 110 |
| 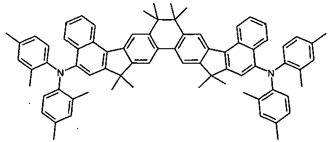 | 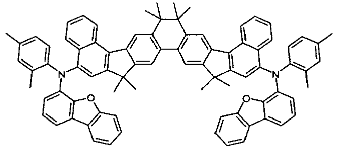 |
| 111 | 112 |
| 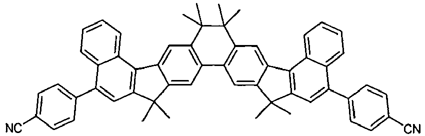 | 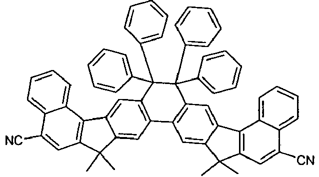 |
| 113 | 114 |
| 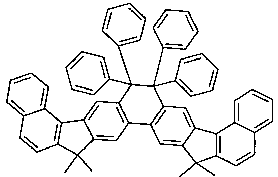 | 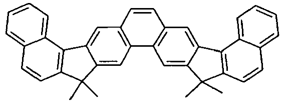 |
| 115 | 116 |
| 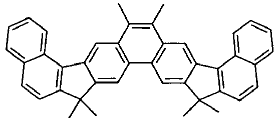 | 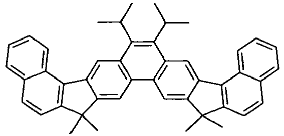 |
| 117 | 118 |
| 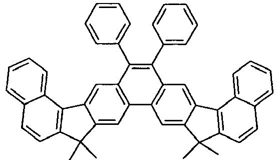 | 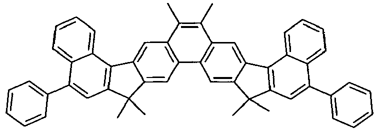 |
| 119 | 120 |
| 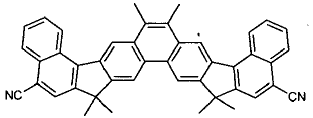 | 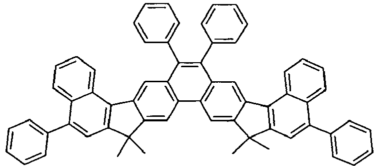 |
| 121 | 122 |
| 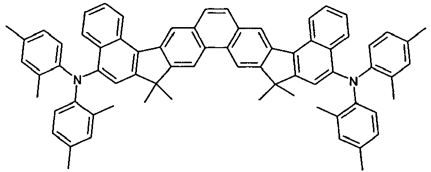 | 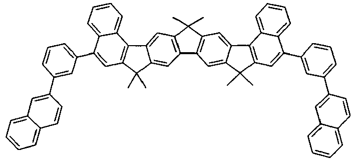 |
| 123 | 124 |
| 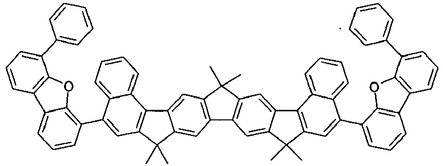 | 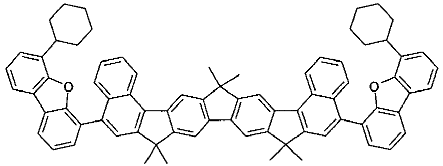 |
| 125 | 126 |
| 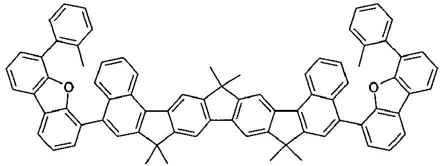 | 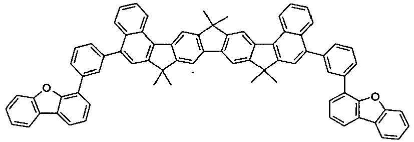 |
| 127 | 128 |
| 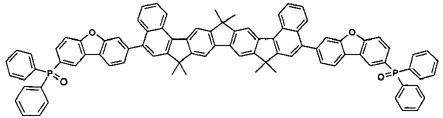 | 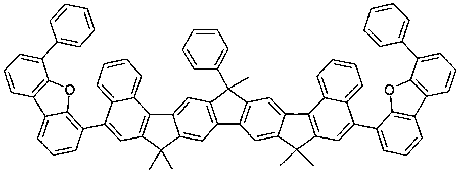 |
| 129 | 130 |
| 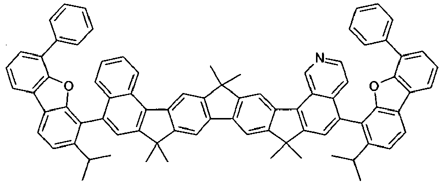 | 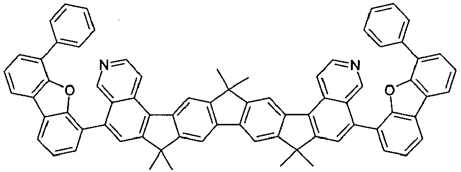 |
| 131 | 132 |
| 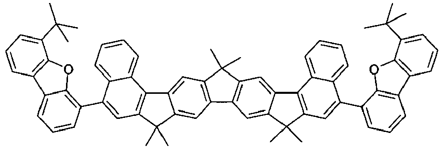 | 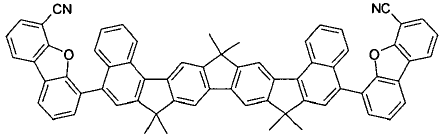 |
| 133 | 134 |
| 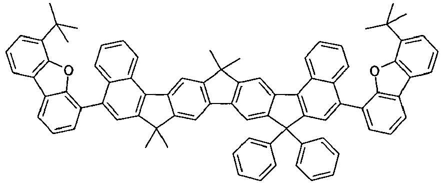 | 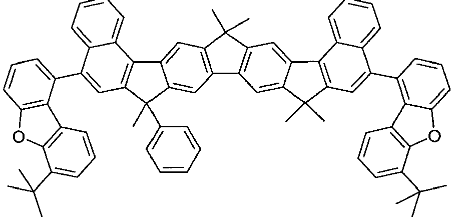 |
| 135 | 136 |
| 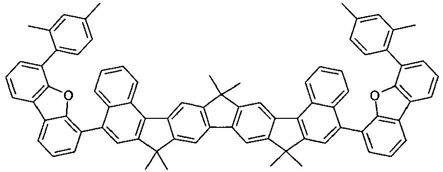 | 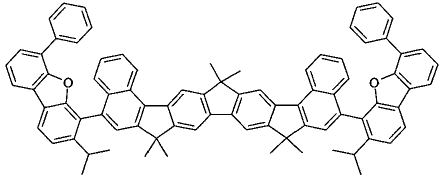 |
| 137 | 138 |
| 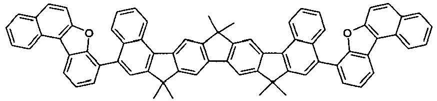 | 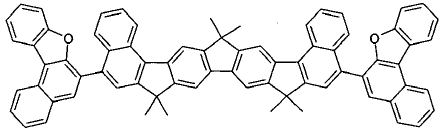 |
| 139 | 140 |
| 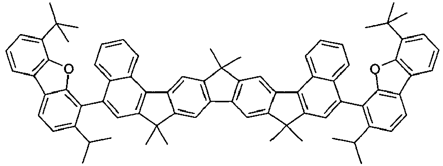 | 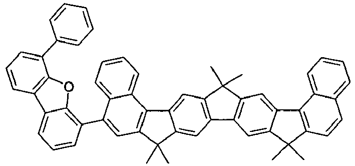 |
| 141 | 142 |
| 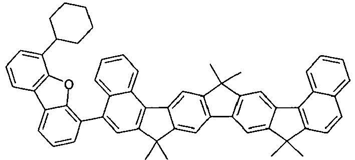 | 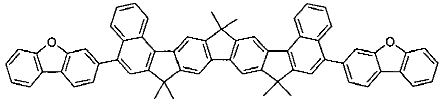 |
| 143 | 144 |
| 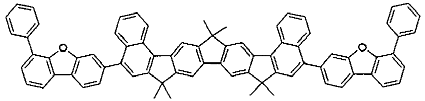 | 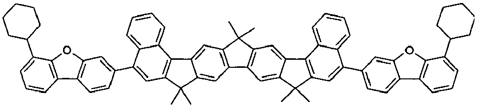 |
| 145 | 146 |
| 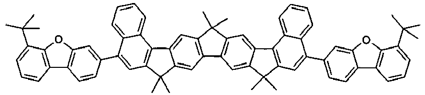 | 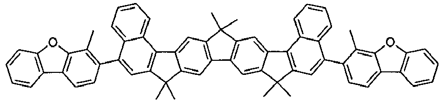 |
| 147 | 148 |
| 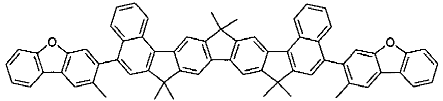 | 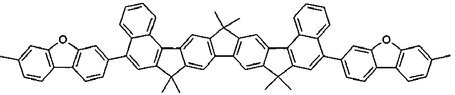 |
| 149 | 150 |
| 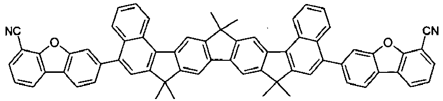 | 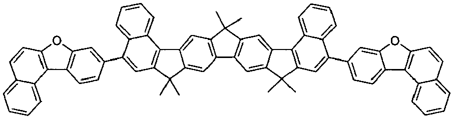 |
| 151 | 152 |
| 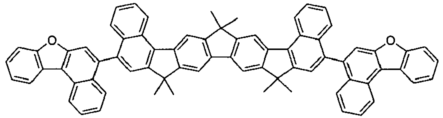 | 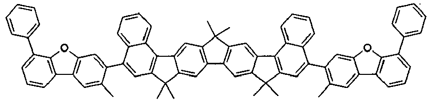 |
| 153 | 154 |
| 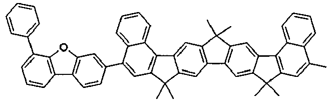 | 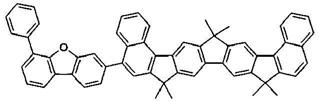 |
| 155 | 156 |
| 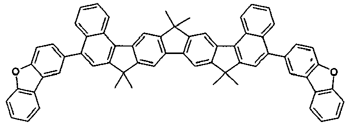 | 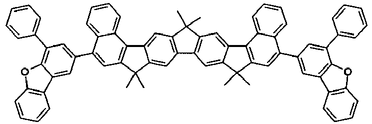 |
| 157 | 158 |
| 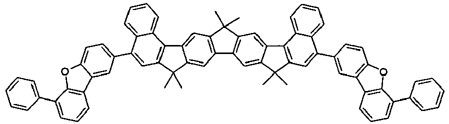 | 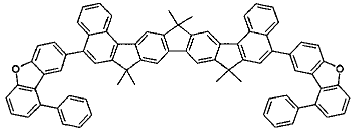 |
| 159 | 160 |
| 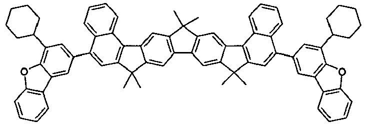 | 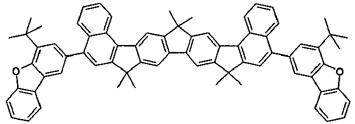 |
| 161 | 162 |
| 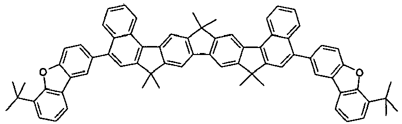 | 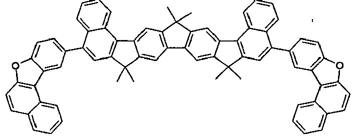 |
| 163 | 164 |
| 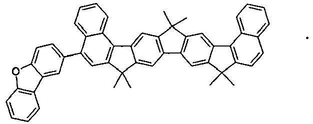 | 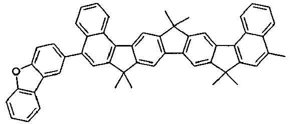 |
| 165 | 166 |
| 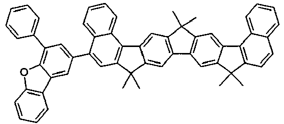 | 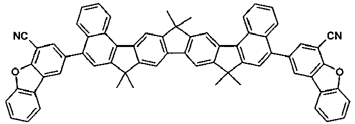 |
| 167 | 168 |
| 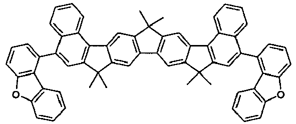 | 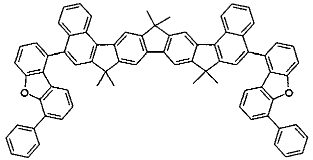 |
| 169 | 170 |
| 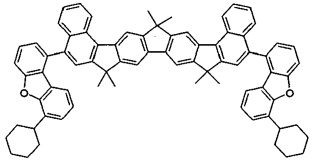 | 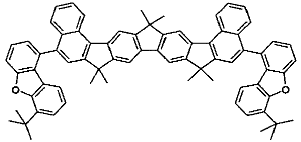 |
| 171 | 172 |
| 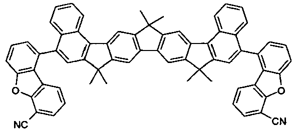 | 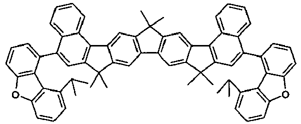 |
| 173 | 174 |
| 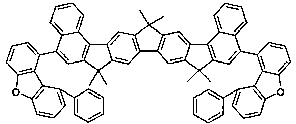 | 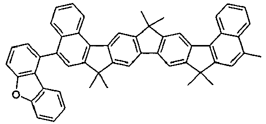 |
| 175 | 176 |
| 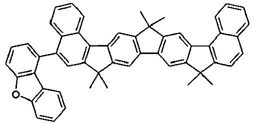 | 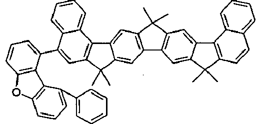 |
| 177 | 178 |
| 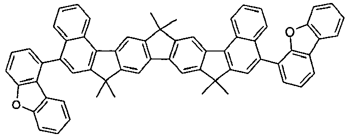 | 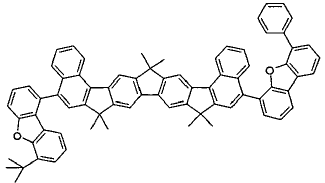 |
| 179 | 180 |
| 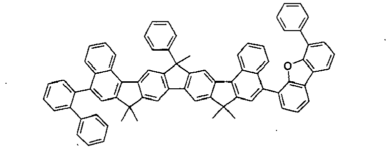 | 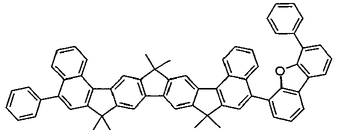 |
| 181 | 182 |
| 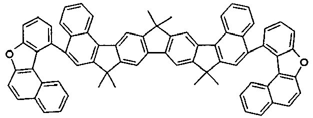 | 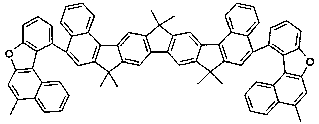 |
| 183 | 184 |
| 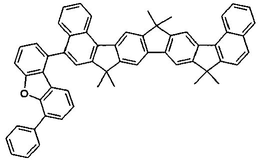 | 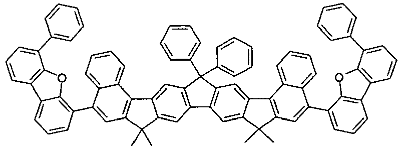 |
| 185 | 186 |
| 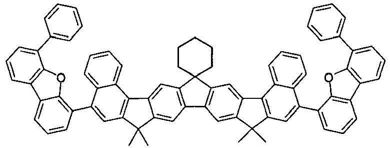 | 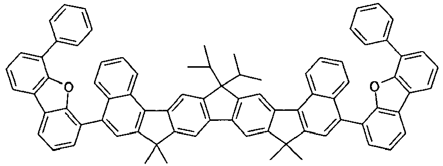 |
| 187 | 188 |
| 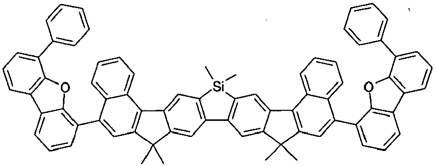 | 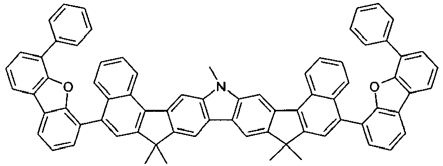 |
| 189 | 190 |
| 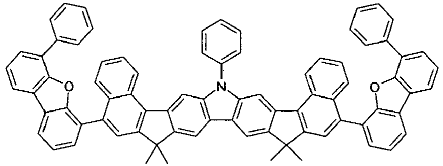 | 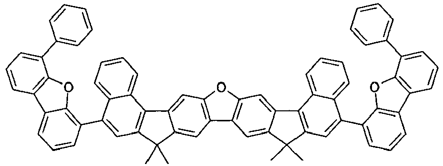 |
| 191 | 192 |
| 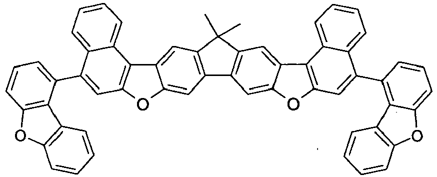 | 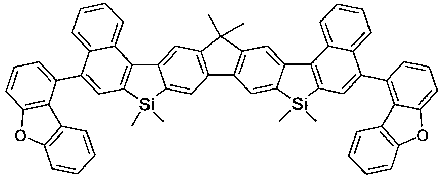 |
| 193 | 194 |
| 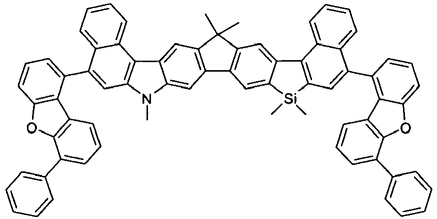 | 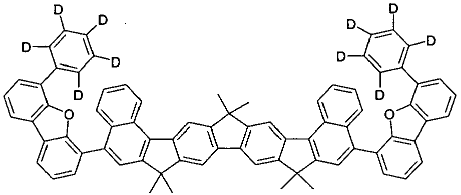 |
| 195 | 196 |
| 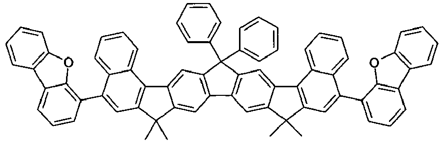 | 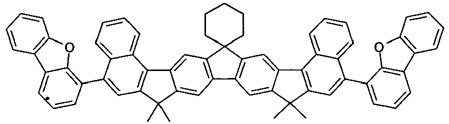 |
| 197 | 198 |
| 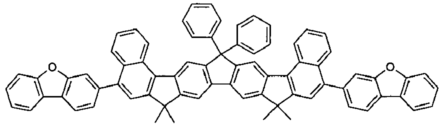 | 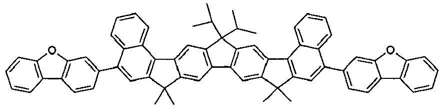 |
| 199 | 200 |
| 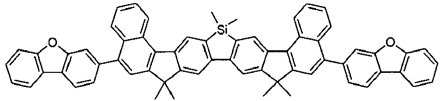 | 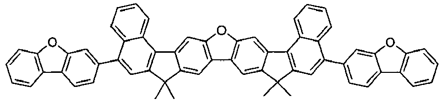 |
| 201 | 202 |
| 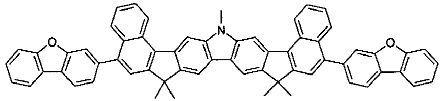 | 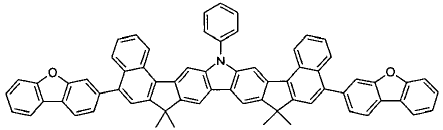 |
| 203 | 204 |
| 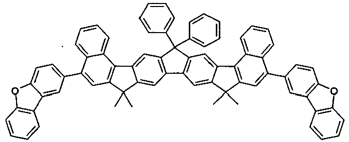 | 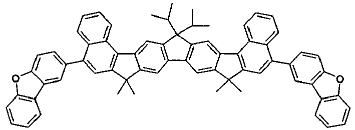 |
| 205 | 206 |
| 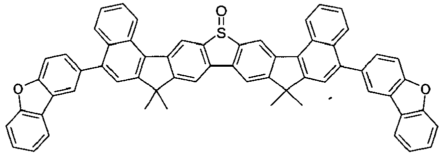 | 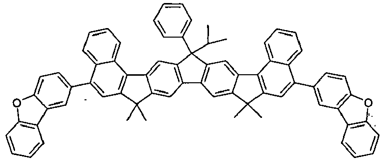 |
| 207 | 208 |
| 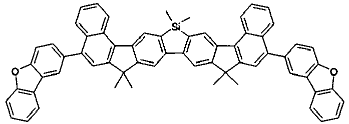 | 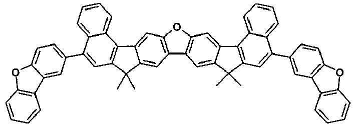 |
| 209 | 210 |
| 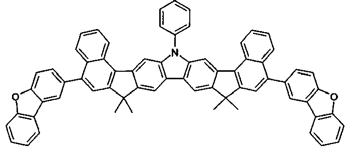 | 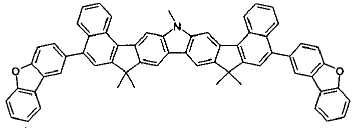 |
| 211 | 212 |
| 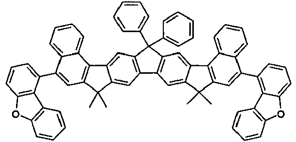 | 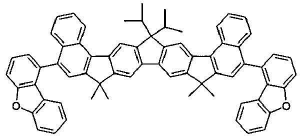 |
| 213 | 214 |
| 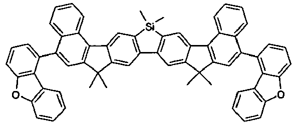 | 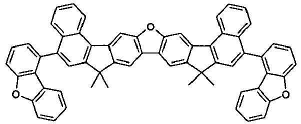 |
| 215 | 216 |
| 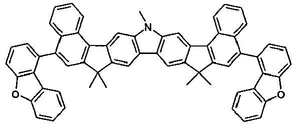 | 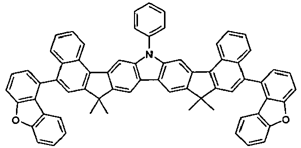 |
| 217 | 218 |
| 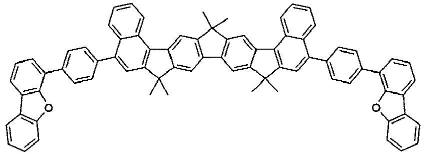 | 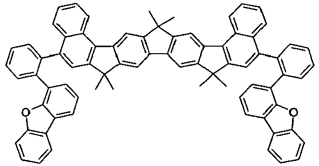 |
| 219 | 220 |
| 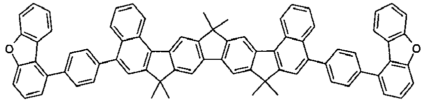 | 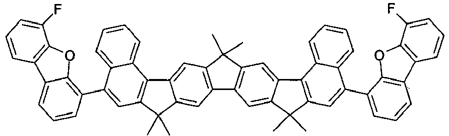 |
| 221 | 222 |
| 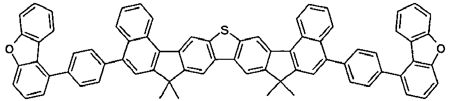 | 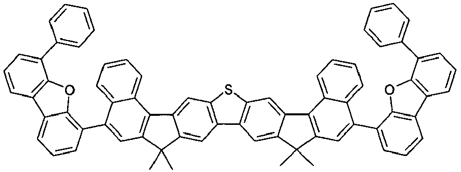 |
| 223 | 224 |
| 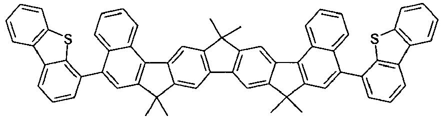 | 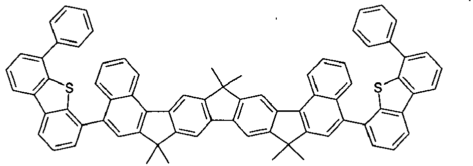 |
| 225 | 226 |
| 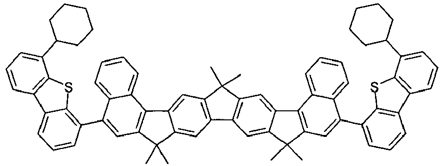 | 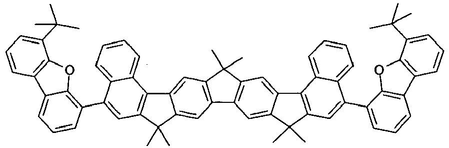 |
| 227 | 228 |
| 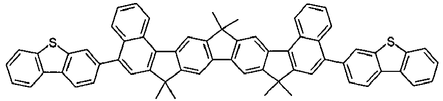 | 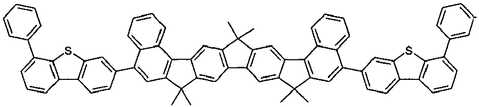 |
| 229 | 230 |
| 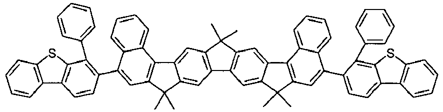 | 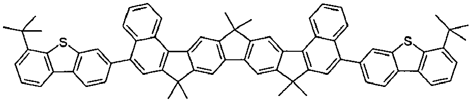 |
| 231 | 232 |
| 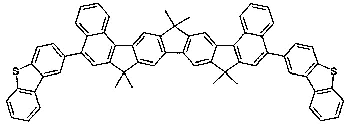 | 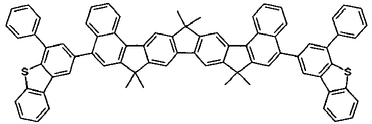 |
| 233 | 234 |
| 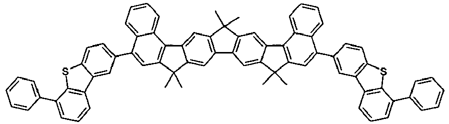 | 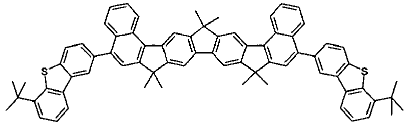 |
| 235 | 236 |
| 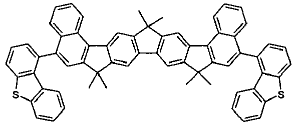 | 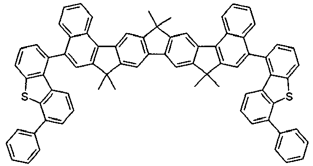 |
| 237 | 238 |
| 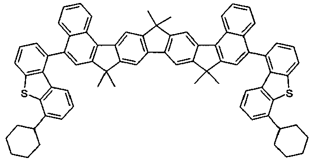 | 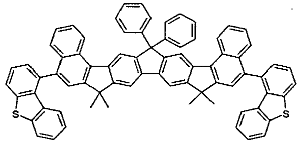 |
| 239 | 240 |
| 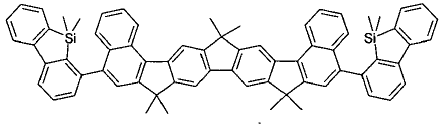 | 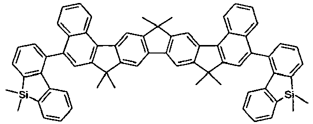 |
| 241 | 242 |
| 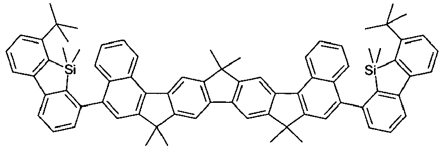 | 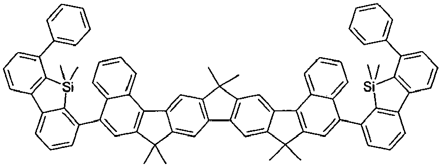 |
| 243 | 244 |
| 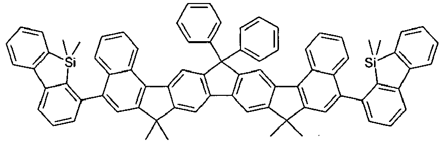 | 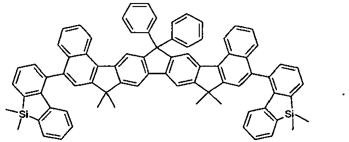 |
| 245 | 246 |
| 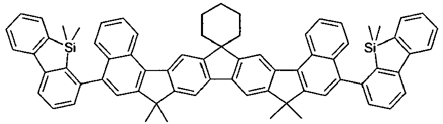 | 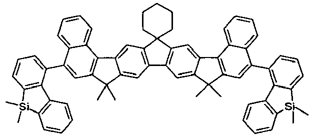 |
| 247 | 248 |
| 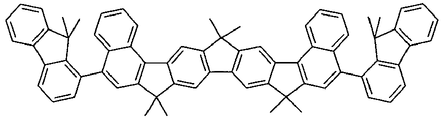 | 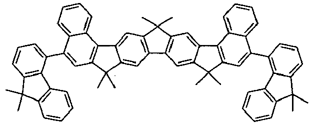 |
| 249 | 250 |
| 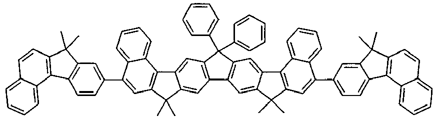 | 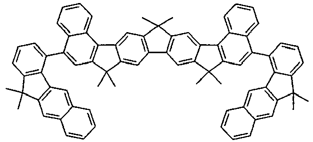 |
| 251 | 252 |
| 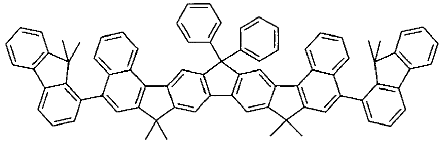 | 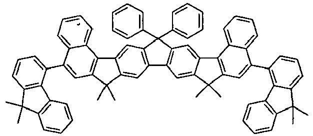 |
| 253 | 254 |
| 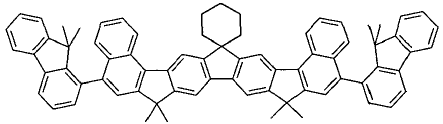 | 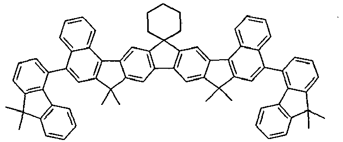 |
| 255 | 256 |
| 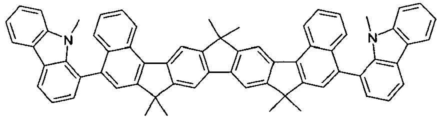 | 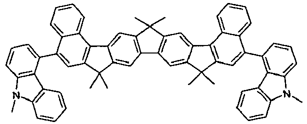 |
| 257 | 258 |
| 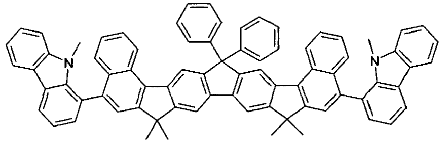 | 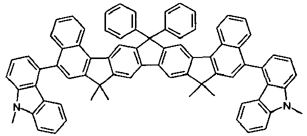 |
| 259 | 260 |
| 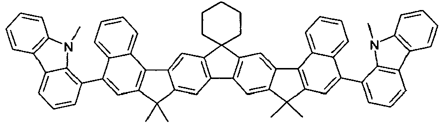 | 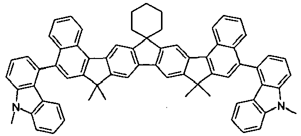 |
| 261 | 262 |
| 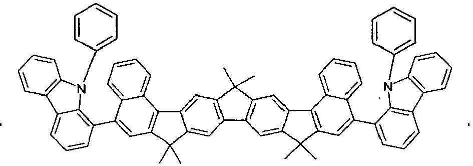 | 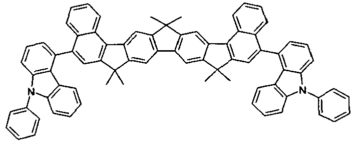 |
| 263 | 264 |
| 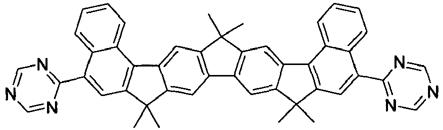 | 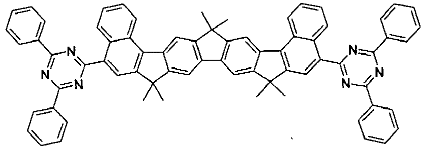 |
| 265 | 266 |
| 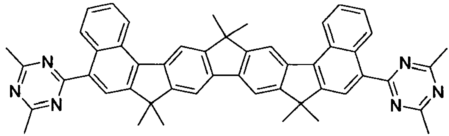 | 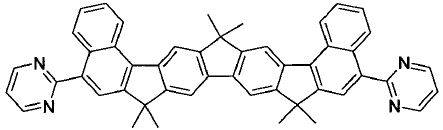 |
| 267 | 268 |
| 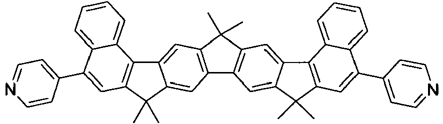 | 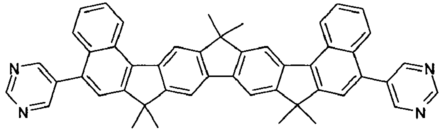 |
| 269 | 270 |
| 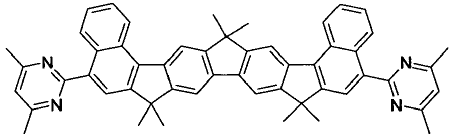 | 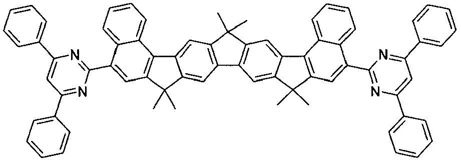 |
| 271 | 272 |
| 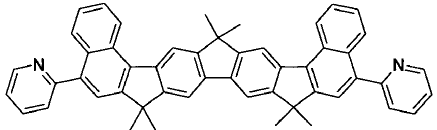 | 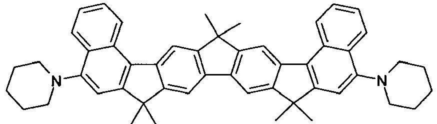 |
| 273 | 274 |
| 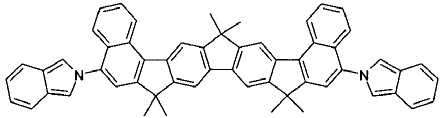 | 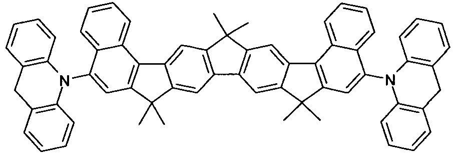 |
| 275 | 276 |
| 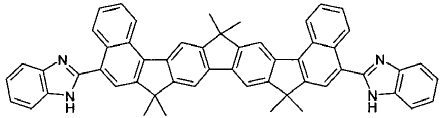 | 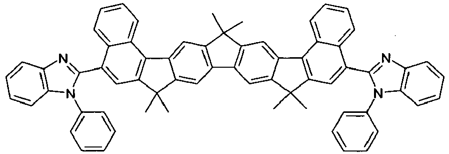 |
| 277 | 278 |
| 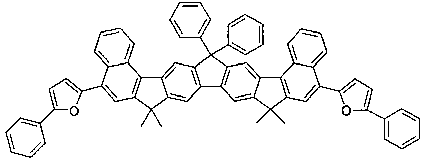 | 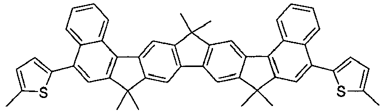 |
| 279 | 280 |
| 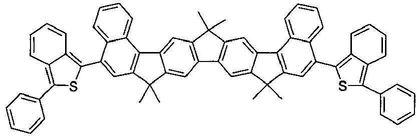 | 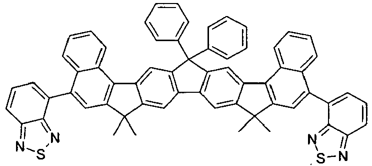 |
| 281 | 282 |
| 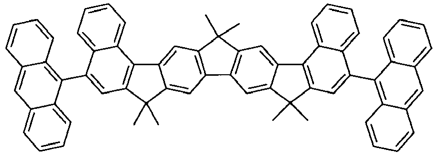 | 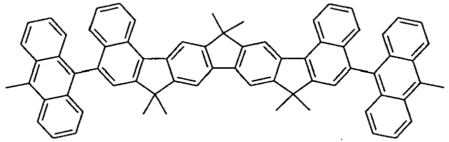 |
| 283 | 284 |
| 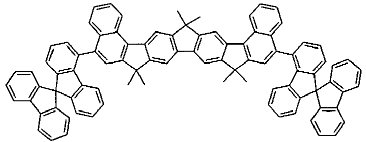 | 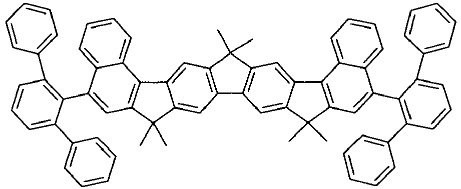 |
| 285 | 286 |
| 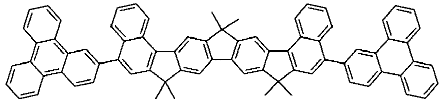 | 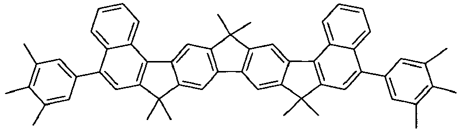 |
| 287 | 288 |
| 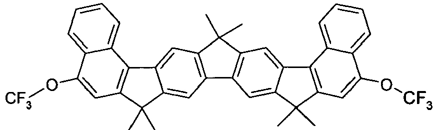 | 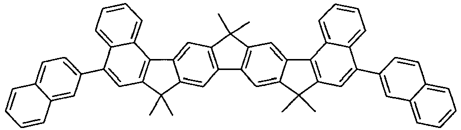 |
| 289 | 290 |
| 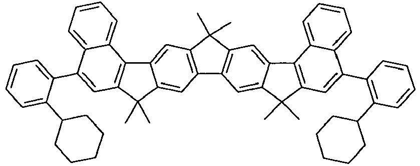 | 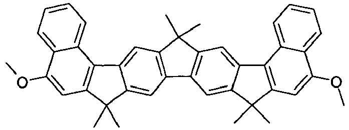 |
| 291 | 292 |
| 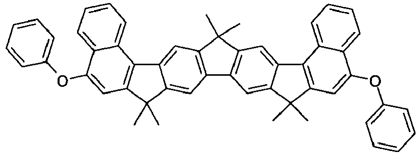 | 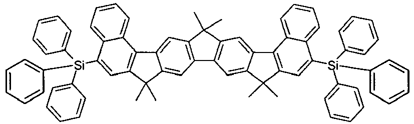 |
| 293 | 294 |
| 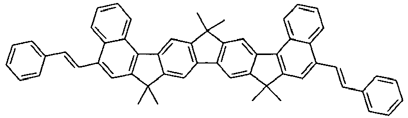 | 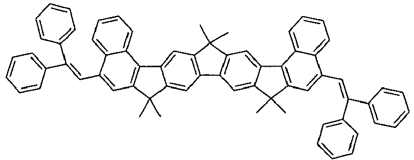 |
| 295 | 296 |
| 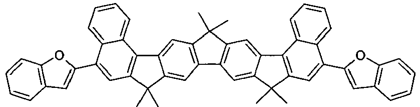 | 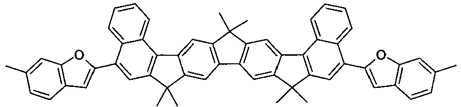 |
| 297 | 298 |
| 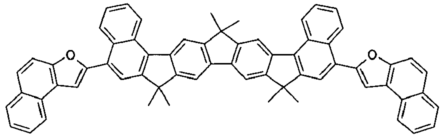 | 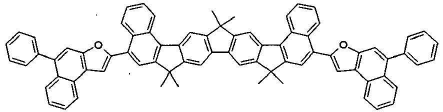 |
| 299 | 300 |
| 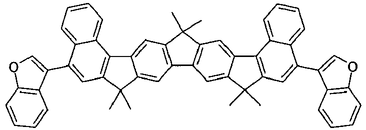 | 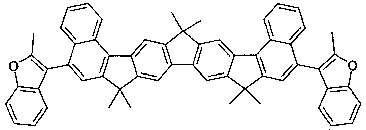 |
| 301 | 302 |
| 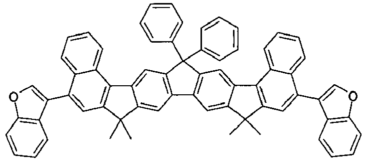 | 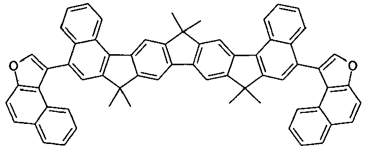 |
| 303 | 304 |
| 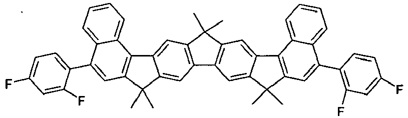 | 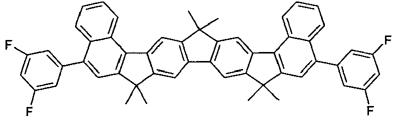 |
| 305 | 306 |
| 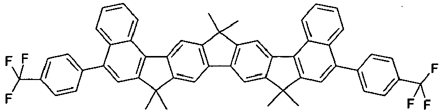 | 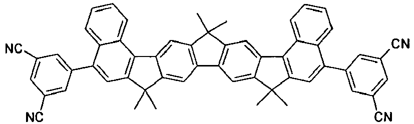 |
| 307 | 308 |
| 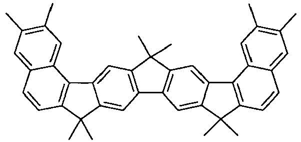 | 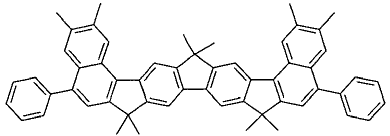 |
| 309 | 310 |
| 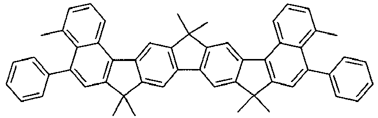 | 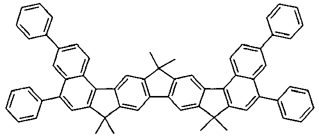 |
| 311 | 312 |
| 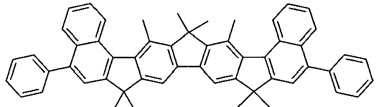 | 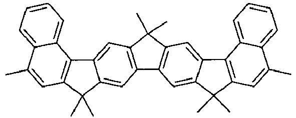 |
| 313 | 314 |
| 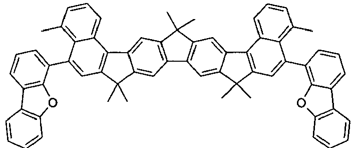 | 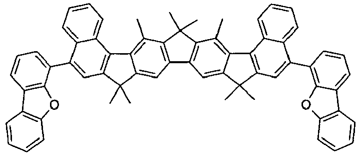 |
| 315 | 316 |
| 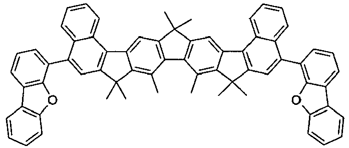 | 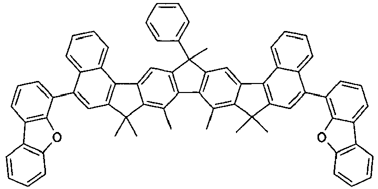 |
| 317 | 318 |
| 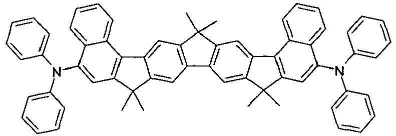 | 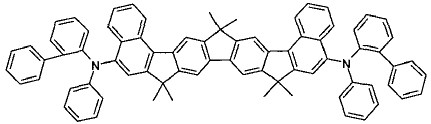 |
| 319 | 320 |
| 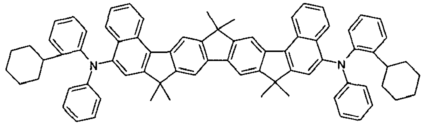 | 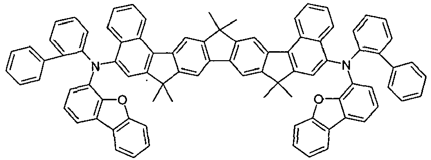 |
| 321 | 322 |
| 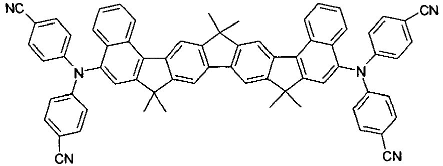 | 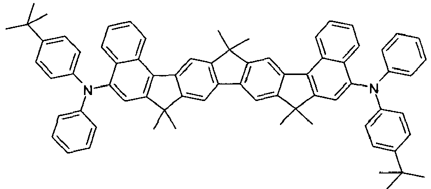 |
| 323 | 324 |
| 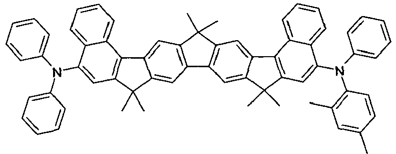 | 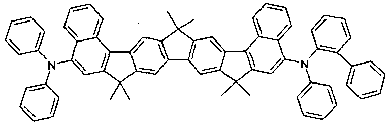 |
| 325 | 326 |
| 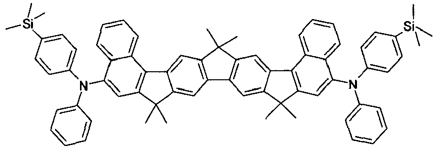 | 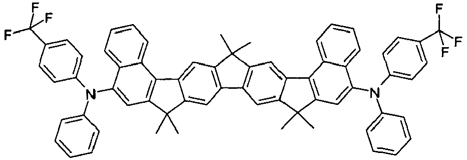 |
| 327 | 328 |
| 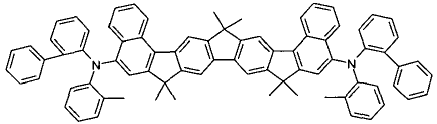 | 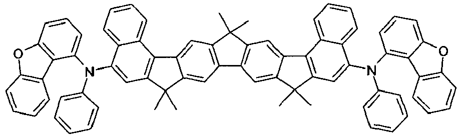 |
| 329 | 330 |
| 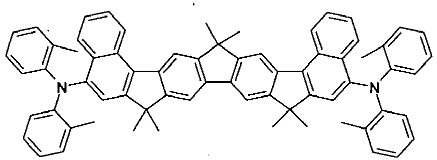 | 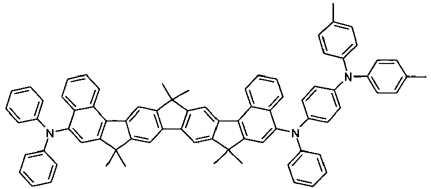 |
| 331 | 332 |
| 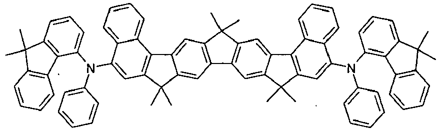 | 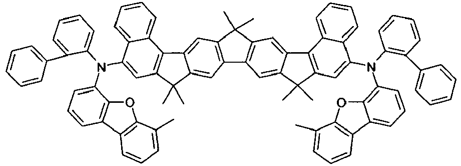 |
| 333 | 334 |
| 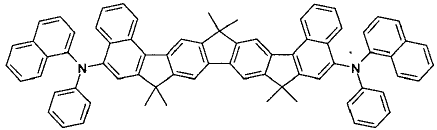 | 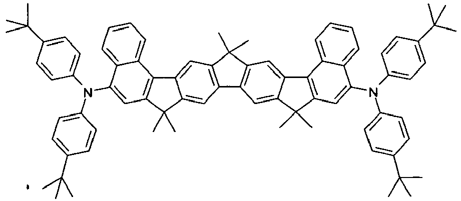 |
| 335 | 336 |
| 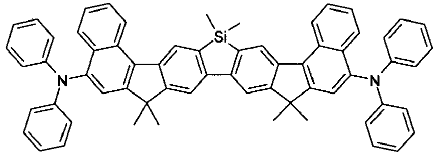 | 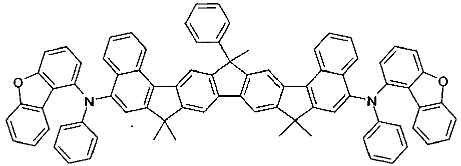 |
| 337 | 338 |
| 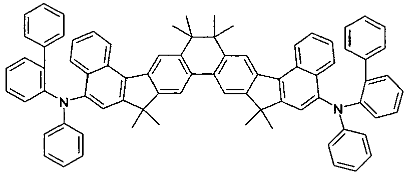 | 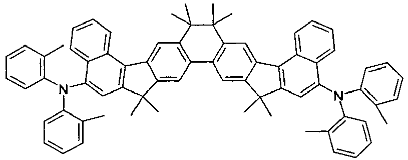 |
| 339 | 340 |
| 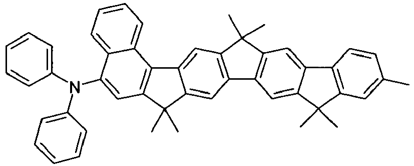 | 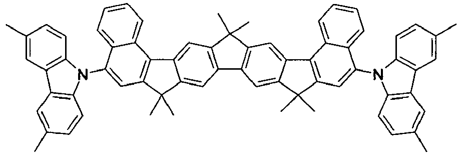 |
| 341 | 342 |
| 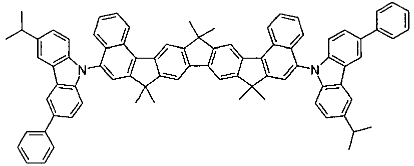 | 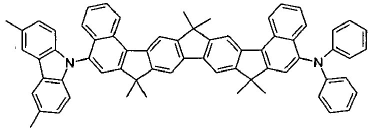 |
| 343 | 344 |
| 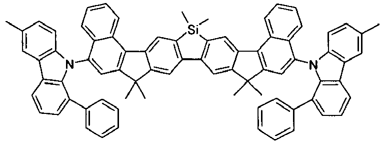 | 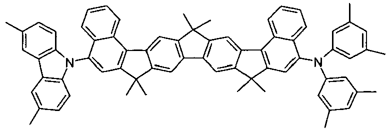 |
| 345 | 346 |
| 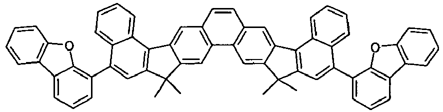 | 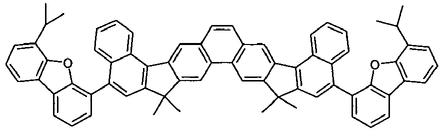 |
| 347 | 348 |
| 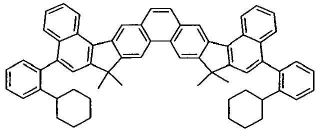 | 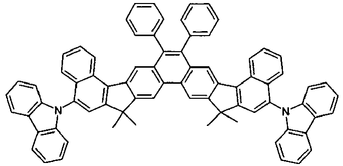 |
| 349 | 350 |
| 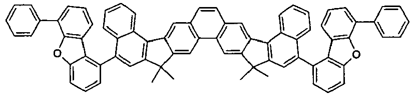 | 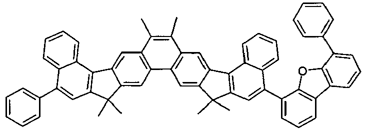 |
| 351 | 352 |
| 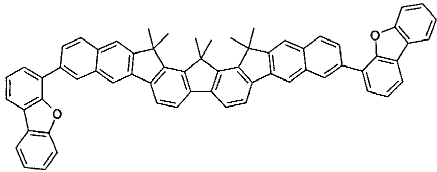 | 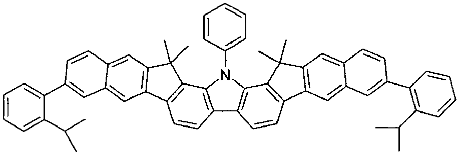 |
| 353 | 354 |
| 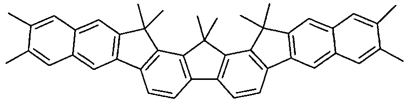 | 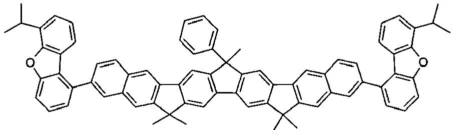 |
| 355 | 356 |
| 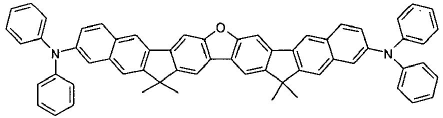 | 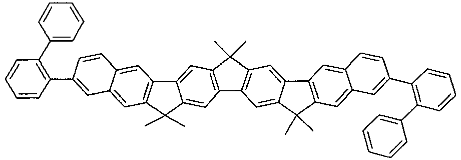 |
| 357 | 358 |
| 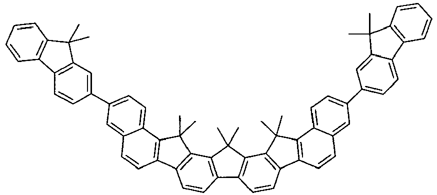 | 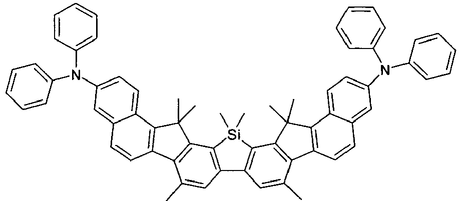 |
| 359 | 360 |
| 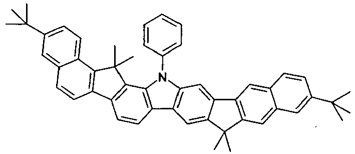 | 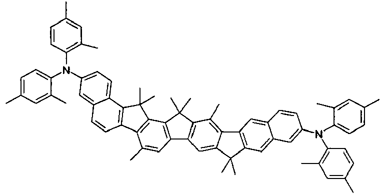 |
| 361 | 362 |
| 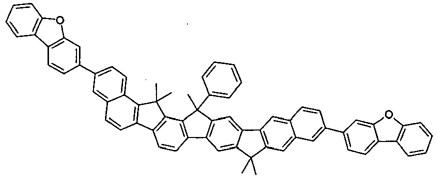 | 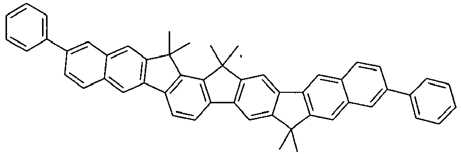 |
| 363 | 364 |
| 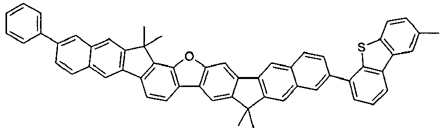 | 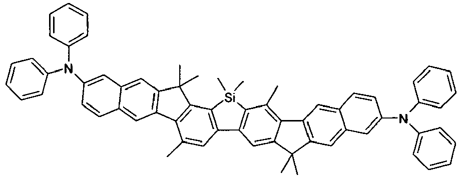 |
| 365 | 366 |
| 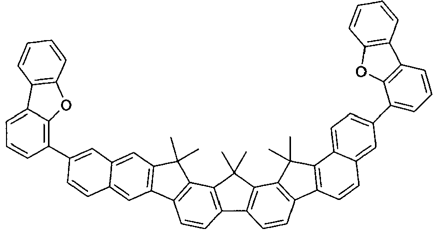 | 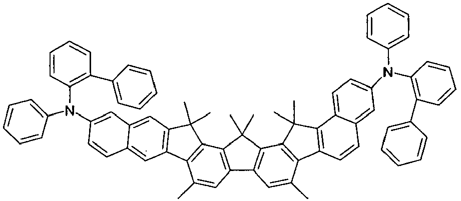 |
| 367 | 368 |
| 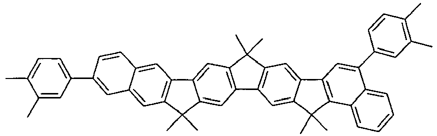 | 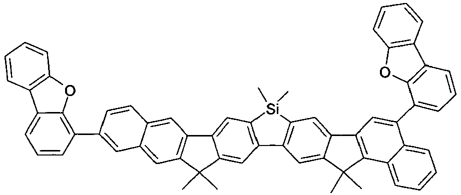 |
| 369 | 370 |
| 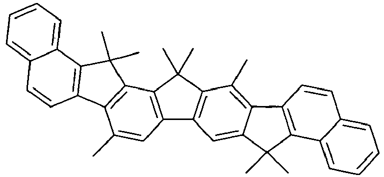 | 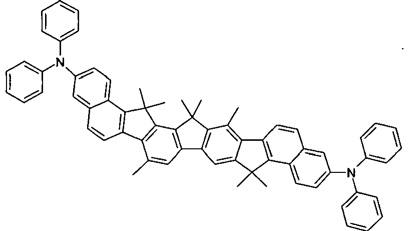 |
| 371 | 372 |
| 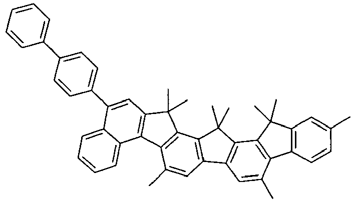 | 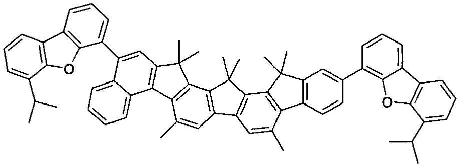 |
| 373 | 374 |
| 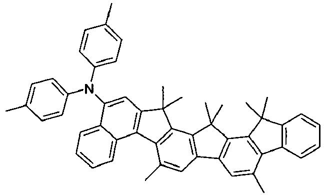 | 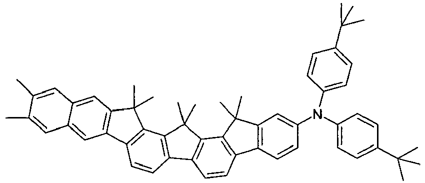 |
| 375 | 376 |
| 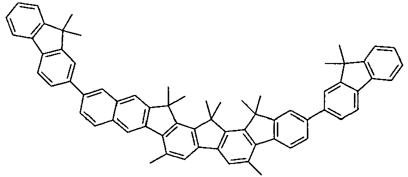 | 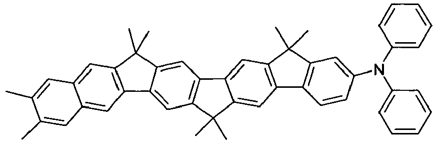 |
| 377 | 378 |
| 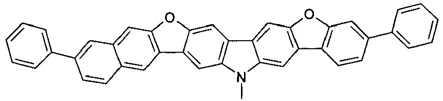 | 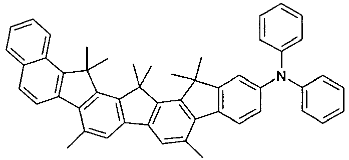 |
| 379 | 380 |
| 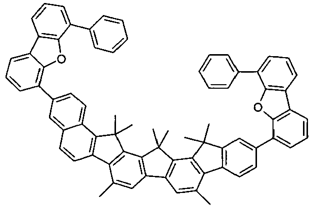 | 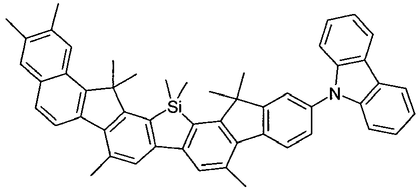 |
| 381 | 382 |
| 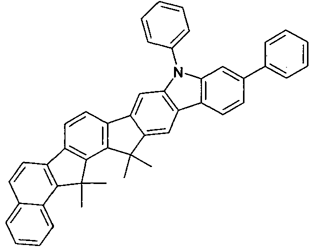 | 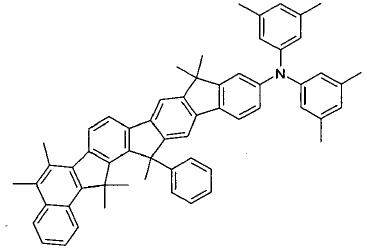 |
| 383 | 384 |
| 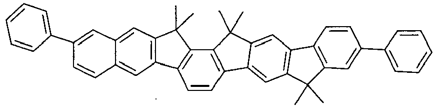 | 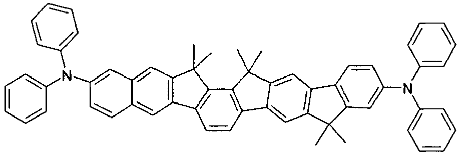 |
| 385 | 386 |
| 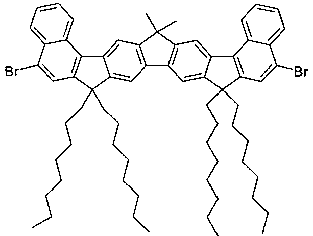 | 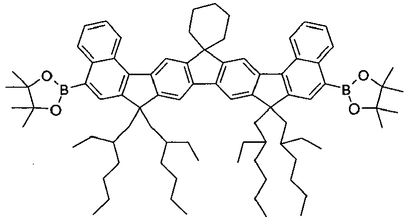 |
| 387 | 388 |
| 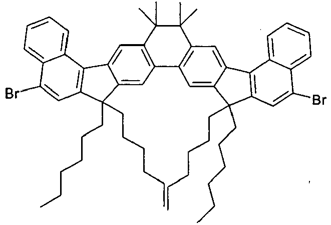 | 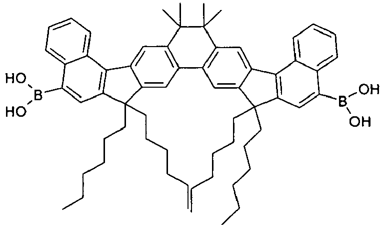 |
| 389 | 390 |
| 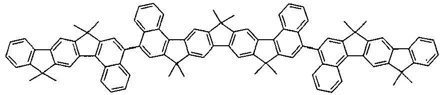 | 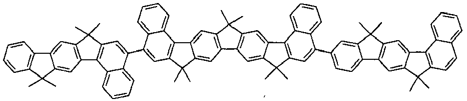 |
| 391 | 392 |

Die Verbindungen der Formel (I) können gemäß bekannten Verfahren bzw. Reaktionsschritten der organischen Chemie hergestellt werden.

Ein bevorzugtes Verfahren zur Herstellung von Verbindungen der Formel (I) ist im Folgenden gezeigt (Schema 1): 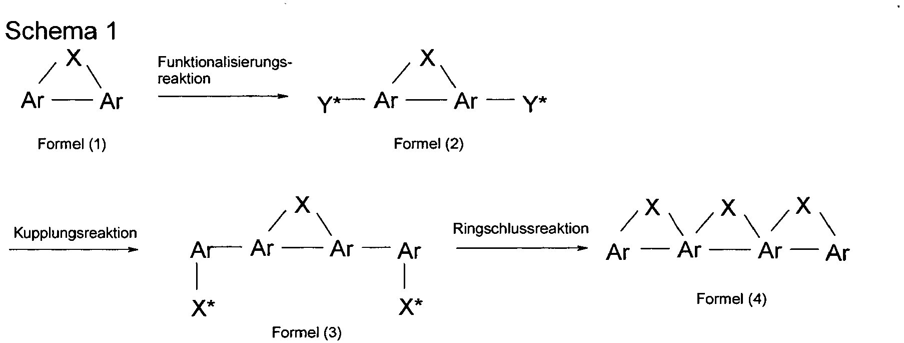
Ar: aromatische oder heteroaromatische Gruppe
X: verbrückende Gruppe
X*: Vorläufergruppe der verbrückenden Gruppe
Y*: reaktive Gruppe, beispielsweise Cl, Br, I

Hierzu werden in eine Ausgangsverbindung (Formel (1)), die in vielen Fällen kommerziell erhältlich ist, reaktive Gruppen eingeführt, beispielsweise durch Bromierung, oder durch Bromierung und anschließende Boronierung. Anschließend wird eine doppelte Kupplungsreaktion, beispielsweise eine Suzuki-Kupplungsreaktion, durchgeführt, mit der zwei weitere aromatische Gruppen eingeführt werden. Diese weiteren aromatischen Gruppen enthalten eine funktionelle Gruppe X*, die einen Ringschluss unter Ausbildung einer verbrückenden Gruppe X durchführen kann. Nach Ringschlussreaktion wird eine Verbindung der Formel (I) (Formel (4) in Schema 1) erhalten, welche optional weiter funktionalisiert werden kann.

Alternativ kann, wie in Schema 2 gezeigt, von einer Verbindung ausgegangen werden, welche bereits zwei verbrückende Gruppen X enthält (Formel (5) in Schema 2). Verfahren zur Herstellung solcher Verbindungen sind dem Fachmann bekannt, beispielsweise aus WO 2008/006449. Die weiteren Schritte entsprechen den bei Schema 1 angegebenen. 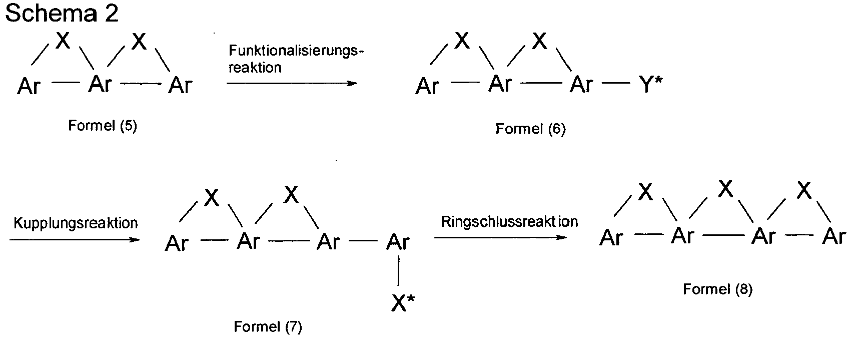
Ar: aromatische oder heteroaromatische Gruppe
X: verbrückende Gruppe
X*: Vorläufergruppe der verbrückenden Gruppe
Y*: reaktive Gruppe, beispielsweise Cl, Br, I

Details zu den oben schematisch angegebenen Verfahren können den Ausführungsbeispielen entnommen werden.

Der Fachmann kann von den oben schematisch angegebenen Verfahren abweichen oder diese modifizieren, um zu Verbindungen der Formel (I) zu gelangen, sofern dies erforderlich ist. Dies erfolgt im Rahmen der üblichen Fähigkeiten des Fachmanns.

Gegenstand der vorliegenden Anmeldung ist somit ein Verfahren zur Herstellung einer Verbindung der Formel (I), dadurch gekennzeichnet, dass es mindestens eine metalkatalysierte Kupplungsreaktion und mindestens eine Ringschlussreaktion umfasst.

Die metallkatalysierte Kupplungsreaktion ist dabei bevorzugt eine übergangsmetallkatalysierte Kupplungsreaktion, besonders bevorzugt eine Suzuki-Reaktion.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹, R² oder R³ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß
EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß
WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die Verbindungen gemäß Formel (I) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Die Verbindung der Formel (I) kann in jeder Funktion in der organischen Elektrolumineszenzvorrichtung eingesetzt werden, beispielsweise als lochtransportierendes Material, als Matrixmaterial, als emittierendes Material, oder als elektronentransportierendes Material.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist eine elektronische Vorrichtung, enthaltend mindestens eine Verbindung der Formel (I). Bevorzugt ist die elektronische Vorrichtung gewählt aus den oben angegebenen Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode. Dabei müssen nicht alle der genannten Schichten vorhanden sein, und es können zusätzlich weitere Schichten vorhanden sein, beispielsweise eine Elektronenblockierschicht anodenseitig an die emittierende Schicht angrenzend, oder eine Lochblockierschicht kathodenseitig an die emittierende Schicht angrenzend.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne, gelbe, orangefarbene oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Alternativ und/oder zusätzlich können in einer derartigen organischen Elektrolumineszenzvorrichtung die erfindungsgemäßen Verbindungen auch in der Lochtransportschicht oder in einer anderen Schicht vorhanden sein. Die verschiedenen emittierenden Schichten können direkt aneinander grenzen, oder sie können durch nicht emittierende Schichten voneinander getrennt sein. Gemäß einer bevorzugten Ausführungsform der Erfindung ist eine weiß emittierende OLED eine sogenannte Tandem-OLED, das heißt es liegen zwei oder mehr vollständige OLED-Schichtabfolgen in der OLED vor, wobei die OLED-Schichtabfolgen jeweils Lochtransportschicht, emittierende Schicht und Elektronentransportschicht umfassen, die jeweils durch eine Ladungserzeugungsschicht (charge generation layer) voneinander getrennt sind.

Es ist bevorzugt, wenn die Verbindung gemäß Formel (I) in einer emittierenden Schicht eingesetzt wird. Insbesondere eignet sich die Verbindung gemäß Formel (I) zur Verwendung als emittierende Verbindung oder als Matrixmaterial in einer emittierenden Schicht.

Die erfindungsgemäße Verbindung eignet sich besonders zur Verwendung als blau emittierende Emitterverbindung oder als Matrixverbindung für eine blau emittierende Emitterverbindung.

Wenn die erfindungsgemäße Verbindung als Matrixmaterial in einer emittierenden Schicht einer OLED eingesetzt wird, ist es bevorzugt, dass keiner der Substituenten R¹, R² und R³ gewählt ist aus mit dem Grundgerüst der Formel (I) konjugierten Gruppen, insbesondere keiner der Substituenten R¹, R² und R³ gewählt ist aus Cyanogruppen, Arylaminogruppen oder Aryl- oder Heteroarylgruppen. Besonders bevorzugt sind bei Verwendung der erfindungsgemäßen Verbindung als Matrixmaterial R¹ und R² gewählt aus H, D, F und Alkylgruppen mit 1 bis 10 C-Atomen, besonders bevorzugt aus H und D, ganz besonders bevorzugt sind R¹ und R² gleich H.

Wenn die erfindungsgemäße Verbindung als Emitterverbindung in einer emittierenden Schicht einer OLED eingesetzt wird, ist es bevorzugt, dass ein oder mehrere Substituenten R¹, R² und R³ gewählt sind aus mit dem Grundgerüst der Formel (I) konjugierten Gruppen, beispielsweise Cyanogruppen, Arylaminogruppen oder Aryl- oder Heteroarylgruppen.

Wenn die erfindungsgemäße Verbindung als emittierende Verbindung in einer emittierenden Schicht eingesetzt wird, wird sie bevorzugt in Kombination mit einem oder mehreren Matrixmaterialien eingesetzt. Unter einem Matrixmaterial wird dabei ein Material verstanden, welches in der emittierenden Schicht vorliegt, bevorzugt als Hauptkomponente, und welches im Betrieb der Vorrichtung nicht Licht emittiert.

Der Anteil der emittierenden Verbindung in der Mischung der emittierenden Schicht beträgt zwischen 0.1 und 50.0 %, bevorzugt zwischen 0.5 und 20.0 %, besonders bevorzugt zwischen 1.0 und 10.0 %. Entsprechend beträgt der Anteil des Matrixmaterials bzw. der Matrixmaterialien zwischen 50.0 und 99.9 %, bevorzugt zwischen 80.0 und 99.5 %, besonders bevorzugt zwischen 90.0 und 99.0 %.

Dabei wird unter den Angaben der Anteile in % im Rahmen der vorliegenden Anmeldung Vol.-% verstanden, wenn die Verbindungen aus der Gasphase aufgebracht werden, und es wird darunter Gew.-% verstanden, wenn die Verbindungen aus Lösung aufgebracht werden.

Wird die erfindungsgemäße Verbindung als Matrixmaterial eingesetzt, kann sie mit allen bekannten emittierenden Verbindungen kombiniert eingesetzt werden. Bevorzugt wird sie in Kombination mit den unten angegebenen bevorzugten emittierenden Verbindungen eingesetzt, besonders den unten angegebenen bevorzugten fluoreszierenden Verbindungen.

Wenn die Verbindung der Formel (I) als Matrixmaterial in Kombination mit einem phosphoreszierenden Emitter in einer emittierenden Schicht eingesetzt wird, ist der phosphoreszierende Emitter bevorzugt ausgewählt aus den unten aufgeführten Ausführungsformen von phosphoreszierenden Emittern. Weiterhin sind in diesem Fall in der emittierenden Schicht bevorzugt ein oder mehrere weitere Matrixmaterialien vorhanden.

Solche sogenannten Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Bevorzugt stellt die Verbindung der Formel (I) das Material mit lochtransportierenden Eigenschaften dar.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

Die erfindungsgemäßen Verbindungen können auch in anderen Schichten als der emittierenden Schicht eingesetzt werden, beispielsweise als Lochtransportmaterialien in einer Lochinjektions- oder Lochtransportschicht oder Elektronenblockierschicht.

Wird die Verbindung gemäß Formel (I) als Lochtransportmaterial eingesetzt, beispielsweise in einer Lochtransportschicht, einer Lochinjektionsschicht oder einer Elektronenblockierschicht, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Weiterhin ist es in diesem Fall bevorzugt, dass die elektronische Vorrichtung mehrere lochtransportierende Schichten zwischen Anode und emittierender Schicht aufweist. Es kann der Fall auftreten, dass alle diese Schichten eine Verbindung der Formel (I) enthalten, oder dass nur einzelne dieser Schichten eine Verbindung der Formel (I) enthalten.

Wird die Verbindung der Formel (I) als Lochtransportmaterial eingesetzt, so ist es bevorzugt, dass sie einen großen Abstand zwischen dem HOMO- und dem LUMO-Energieniveau aufweist. Weiterhin ist es bevorzugt, dass sie keine Aminogruppen als Substituenten aufweist. Weiterhin ist es bevorzugt, dass sie überhaupt keine Substituenten an den aromatischen Ringen aufweist, d.h. dass R¹ und R² gleich H oder D, besonders bevorzugt gleich H sind.

Die Verbindung der Formel (I) kann weiterhin als elektronentransportierende Verbindung in einer Elektronentransportschicht, einer Lochblockierschicht oder einer Elektroneninjektionsschicht eingesetzt werden. Hier für ist es bevorzugt, dass die Verbindung der Formel (I) einen oder mehrere Substituenten gewählt aus elektronenarmen Heteroarylgruppen wie beispielsweise Triazin, Pyrimidin oder Benzimidazol enthält.

Im Folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende emittierende Verbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden emittierenden Verbindungen können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter sind neben den erfindungsgemäßen Verbindungen ausgewählt aus der Klasse der Arylamine. Unter einem Arylamin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und WO 2013/185871 offenbarten Pyren-Arylamine. Ebenfalls bevorzugt sind die in WO 2014/037077 offenbarten Benzoindenofluoren-Amine, die in der noch nicht offengelegten EP 13000012.8 offenbarten Benzofluoren-Amine und die in der noch nicht offengelegten EP13004921.6 offenbarten erweiterten Indenofluorene.

Bevorzugte fluoreszierende emittierende Verbindungen sind in der folgenden Tabelle abgebildet:

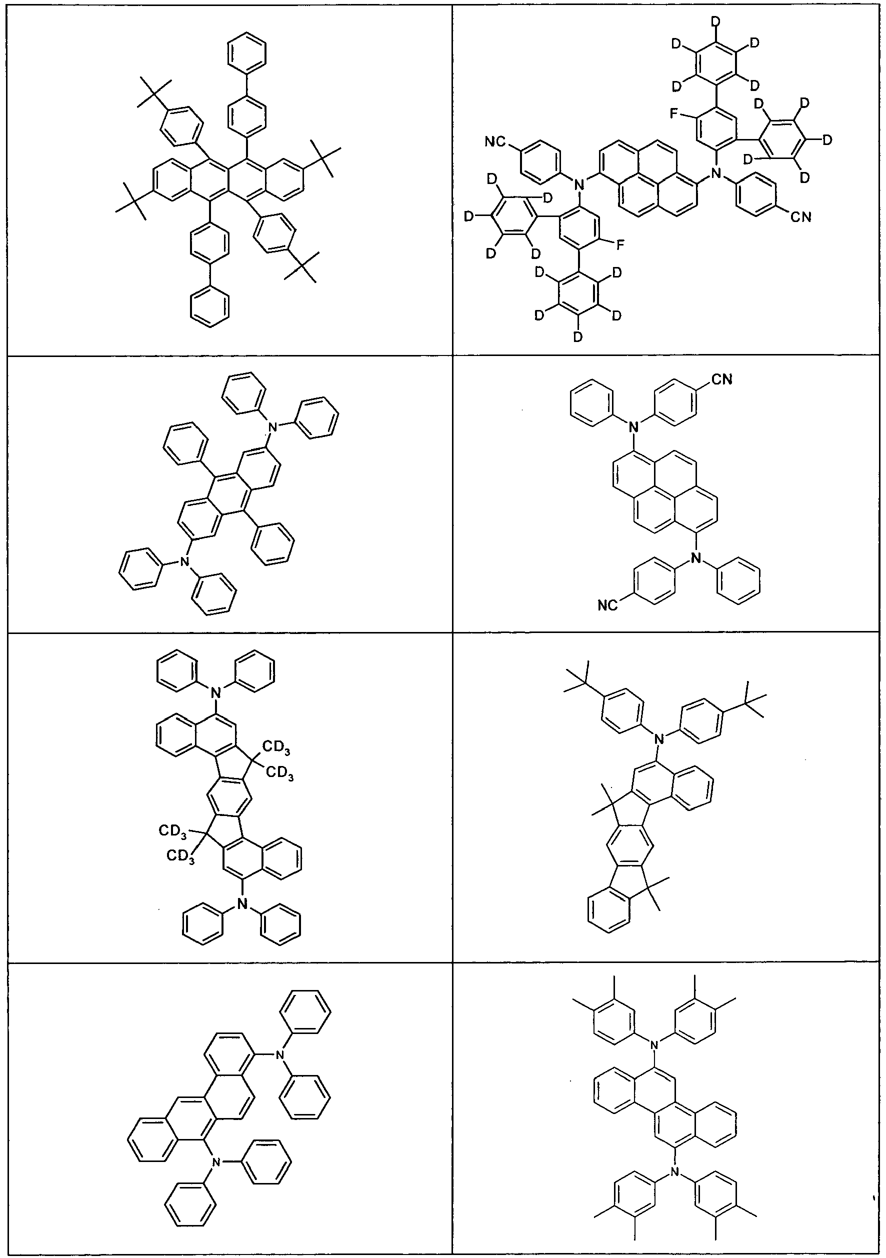

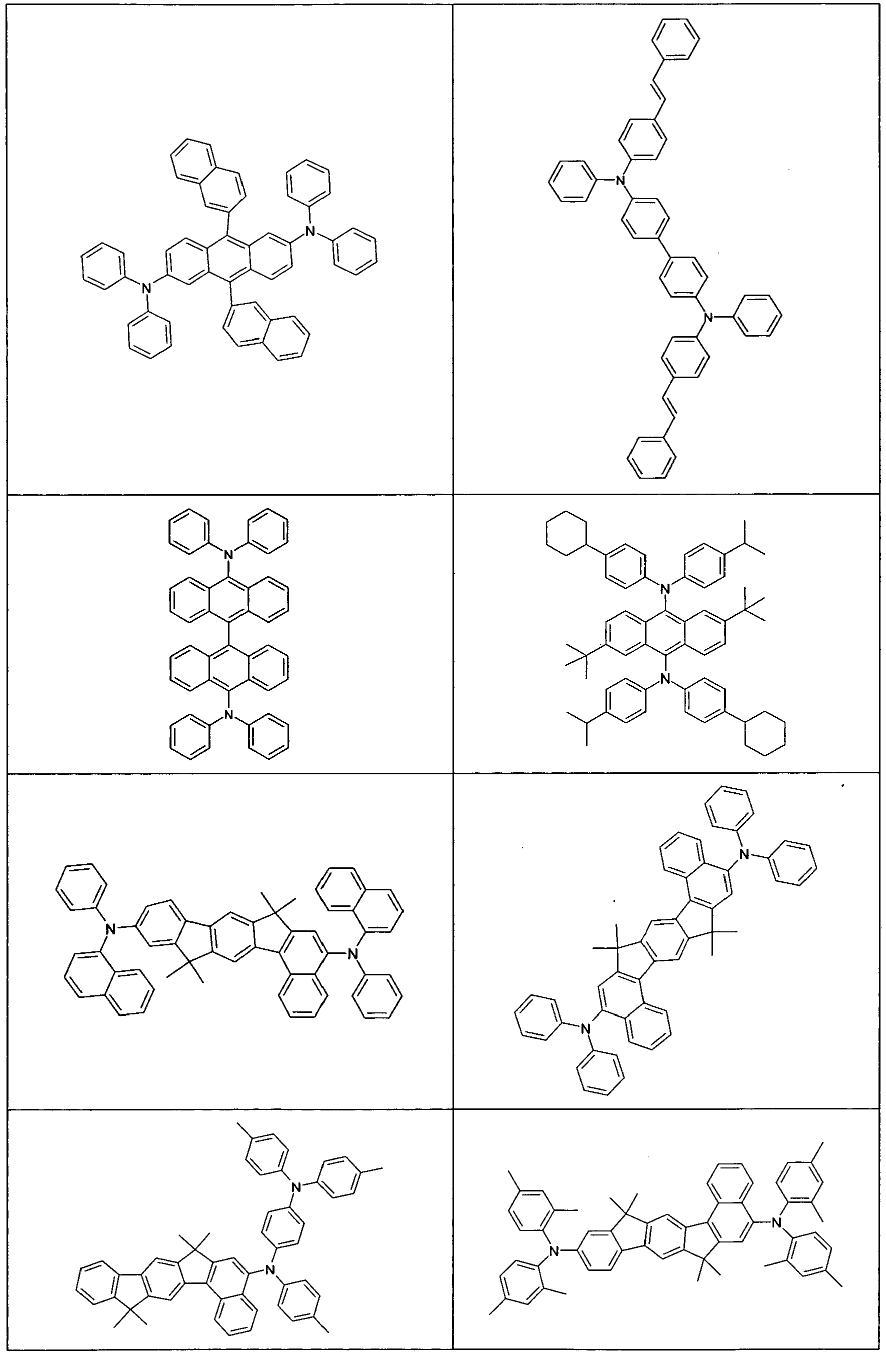

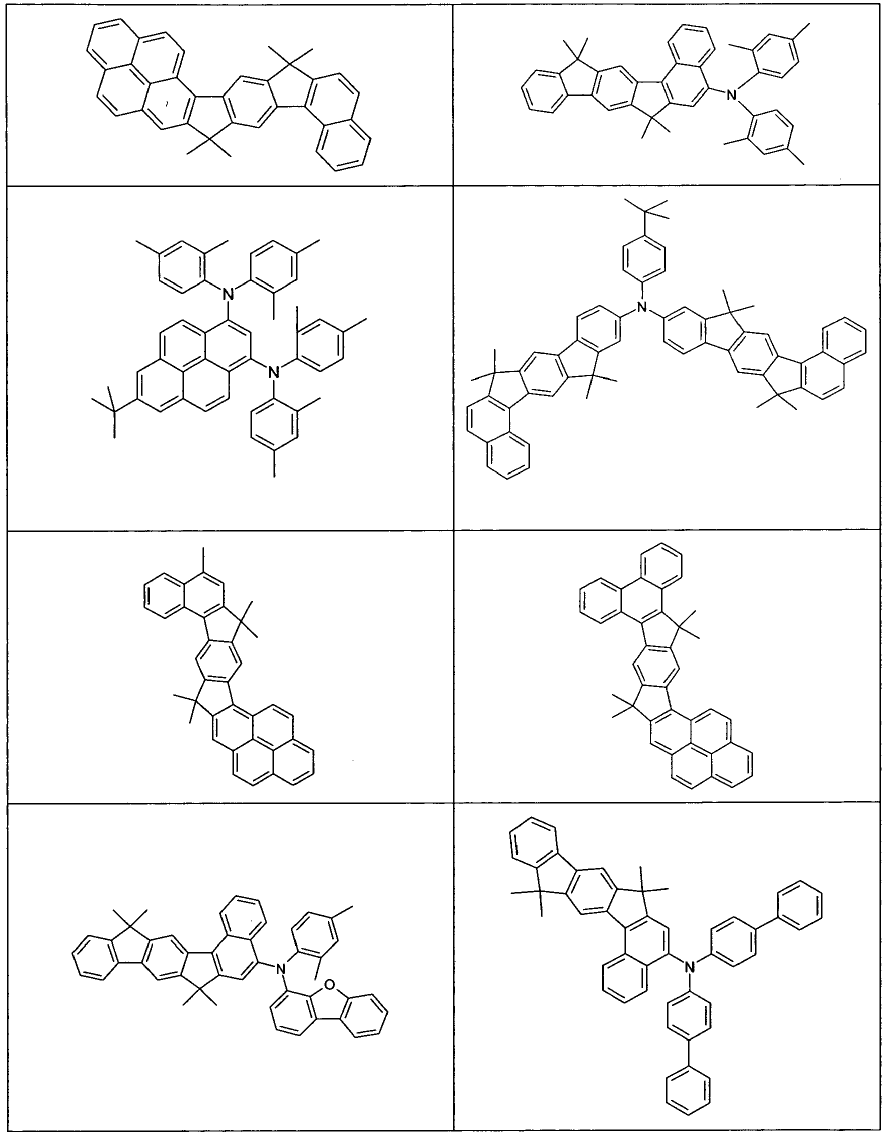

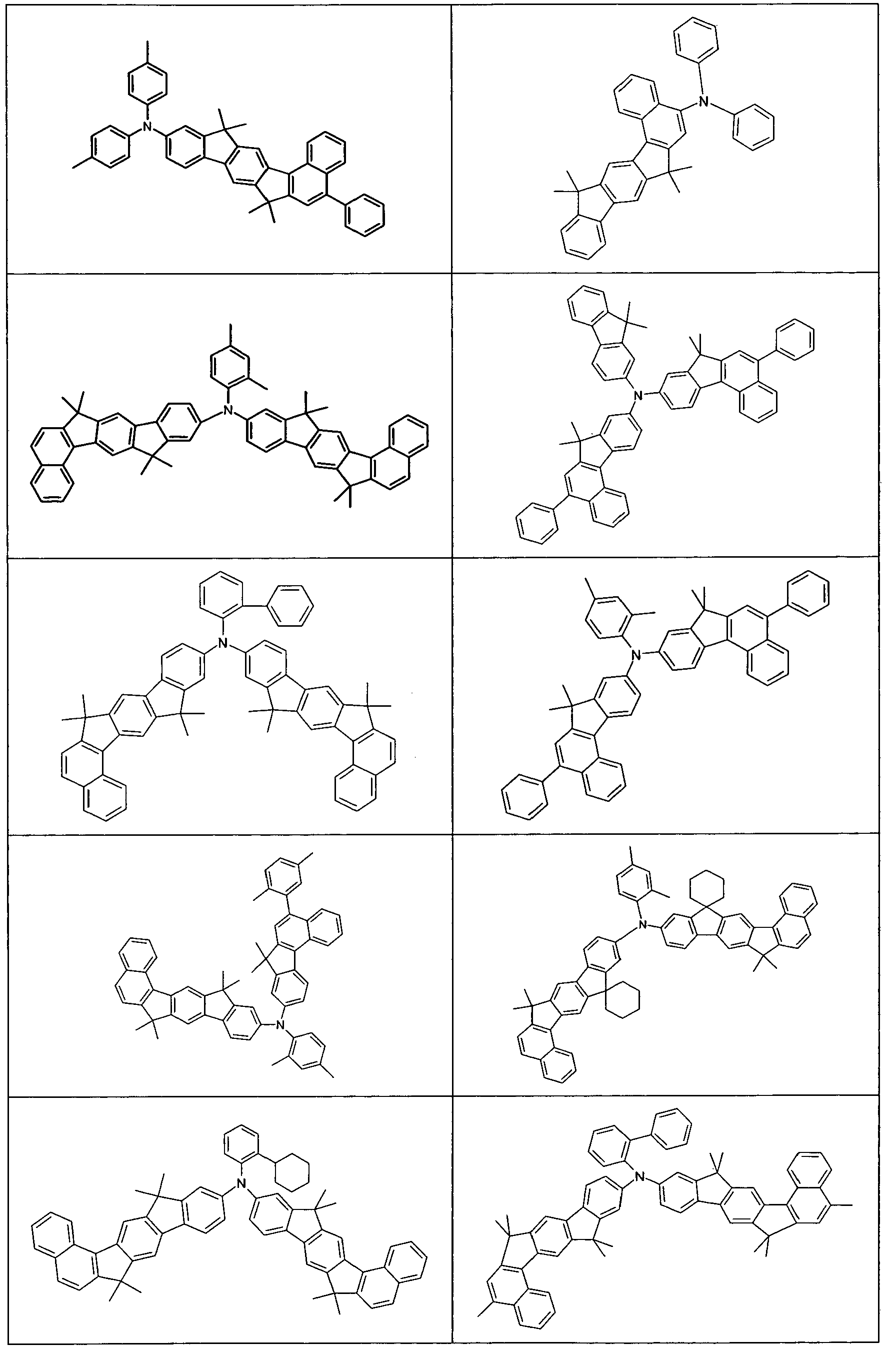

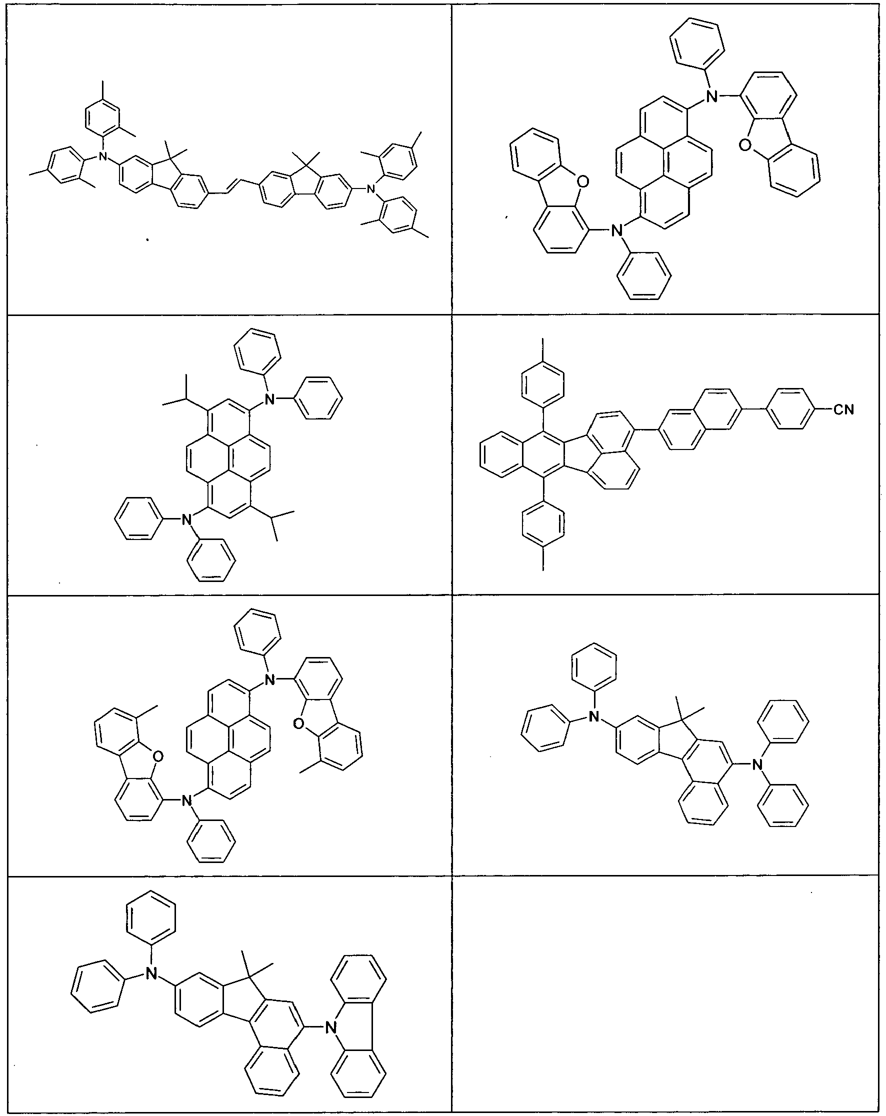

Bevorzugte Matrixmaterialien zur Verwendung in Kombination mit fluoreszierenden emittierenden Verbindungen sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Besonders bevorzugte Matrixmaterialien zur Verwendung in Kombination mit der Verbindung der Formel (I) in der emittierenden Schicht sind in der folgenden Tabelle abgebildet.

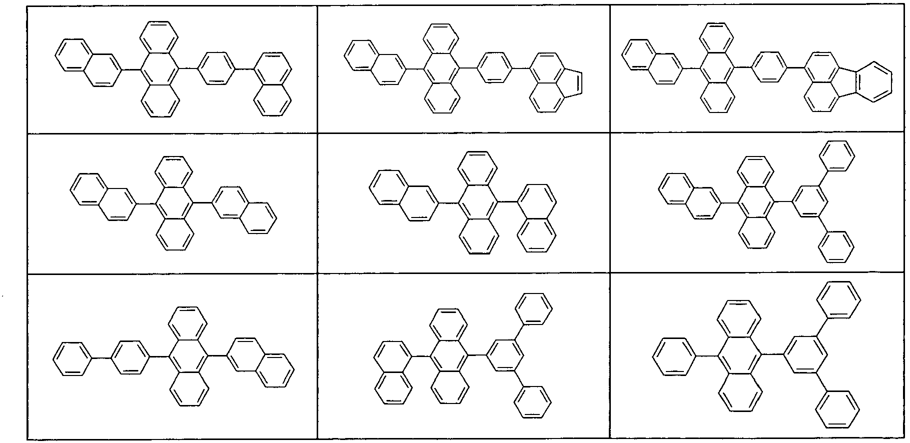

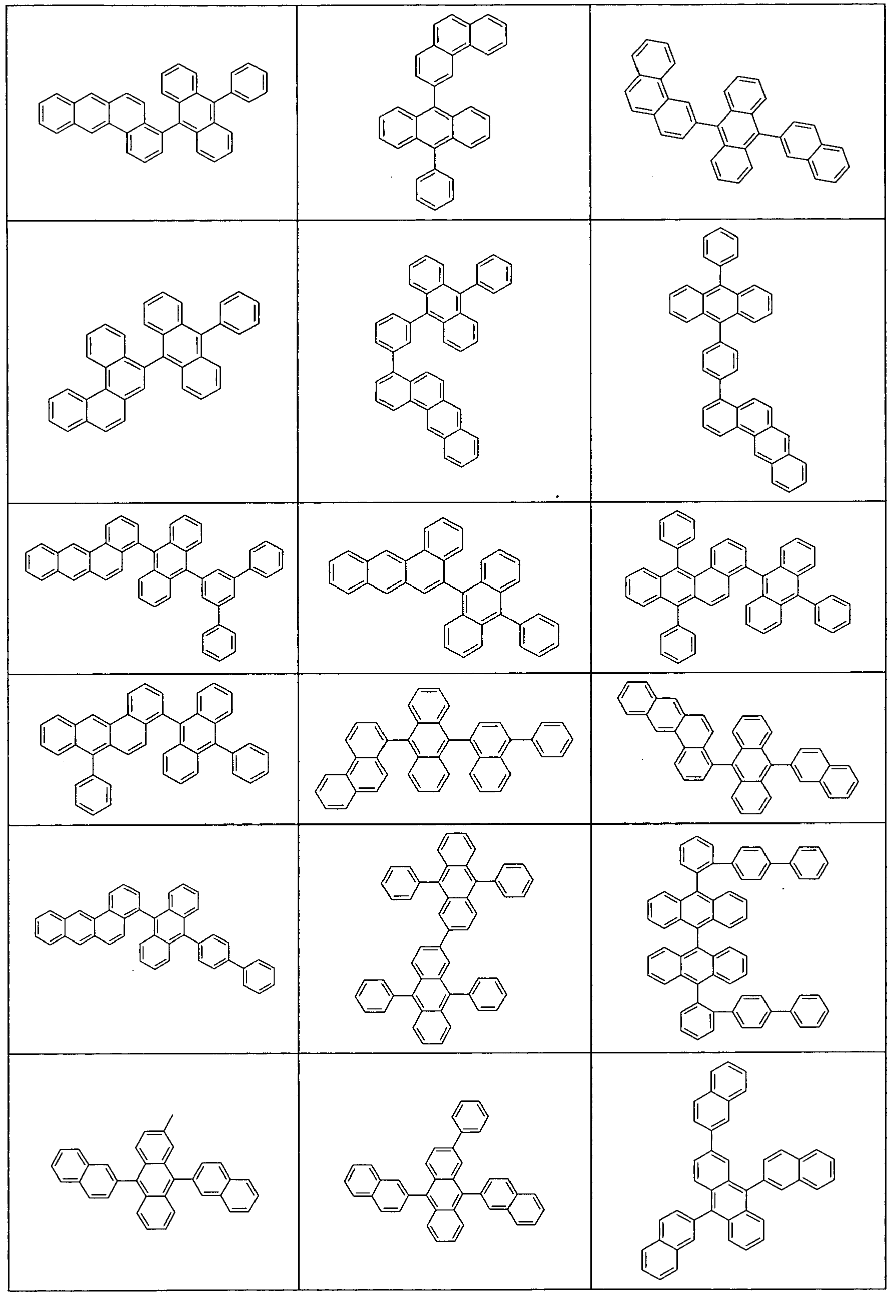

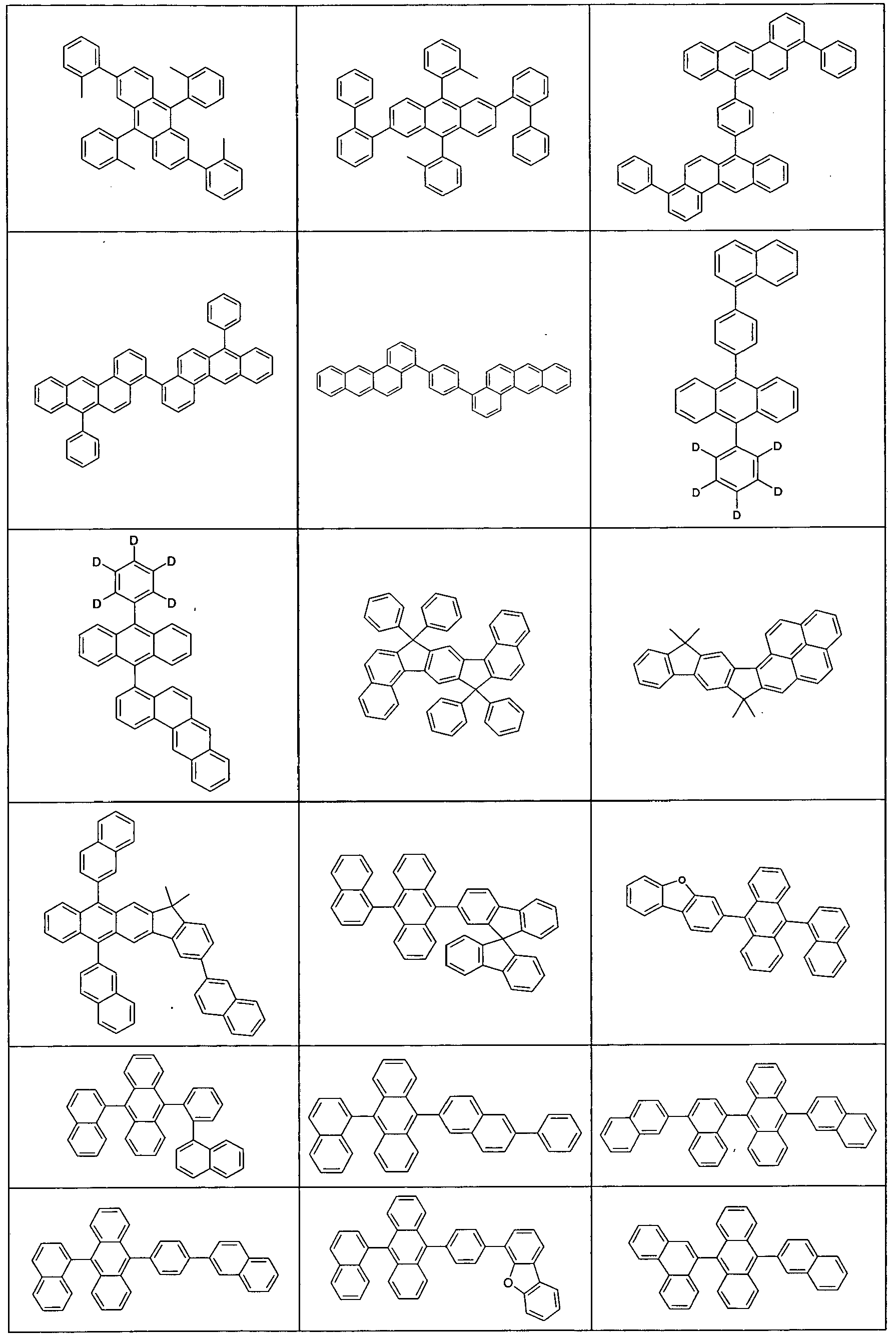

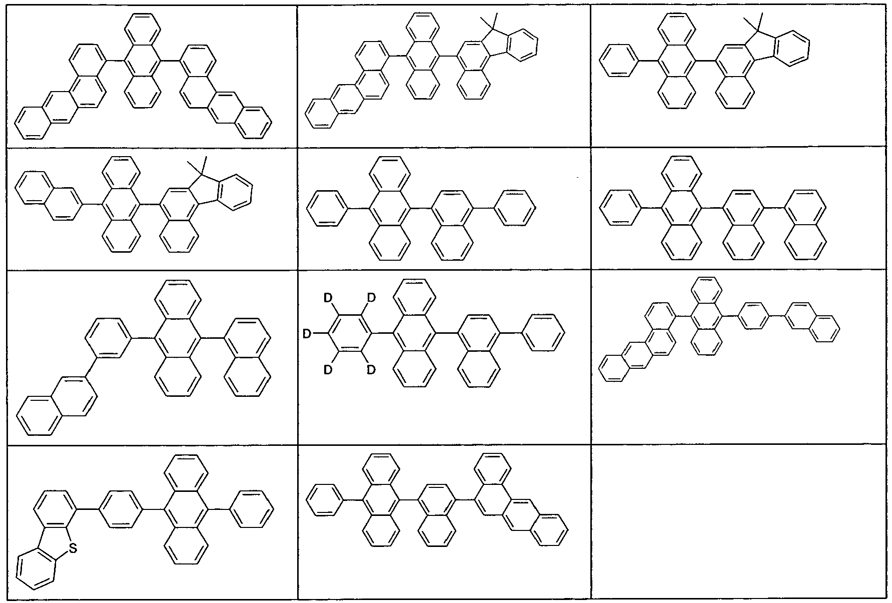

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport-, Lochinjektions- oder Elektronenblockierschicht in der erfindungsgemäßen Elektrolumineszenzvorrichtung verwendet werden können, sind neben der Verbindungen der Formel (I) Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthese-Beispiele

### A-1) Variante I

Es wird nach folgendem allgemeinen Schema vorgegangen:

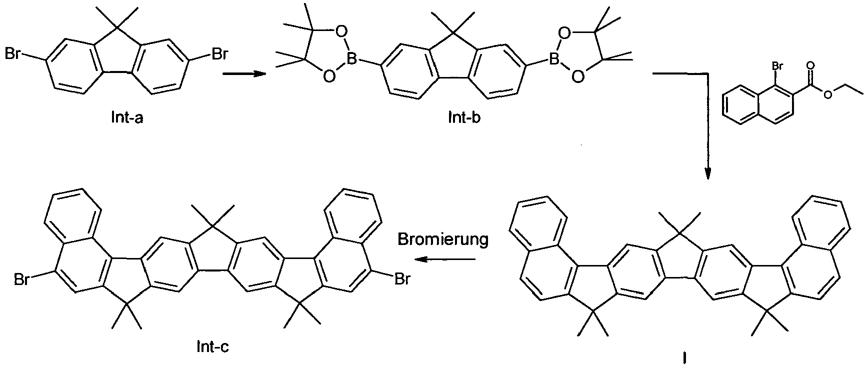

| **Verbindung** | 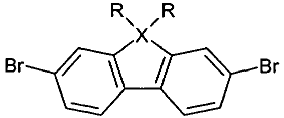 | **Synthese/ Ausbeute** |
|---|---|---|
| | 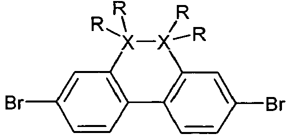 | |
| **Int-a1** | 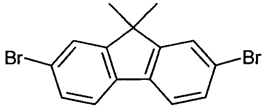 | Kommerziell erhältlich |
| **Int-a2** | 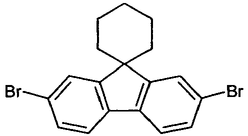 | Kommerziell erhältlich |
| **Int-a3** | 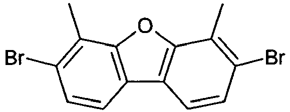 | Bioorg. & Med. Chem. Lett., 2012, 22, 5108-5113 |
| **Int-a4** | 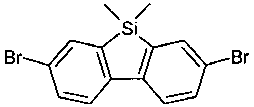 | Tetrahedron 2001, 57, 10047-10053 |
| **Int-a5** | 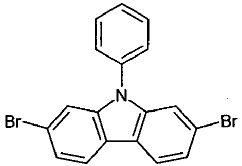 | Synthesis 2003, 16, 2470-2472 |
| **Int-a6** | 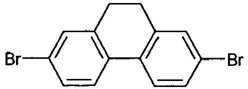 | J. Mat. Chem. 2009, 19, 4148-4156 |
| **Int-a7** | 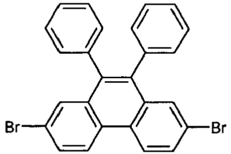 | Org. Lett. 2008, 10, 773-776 |
| **Int-a8** | 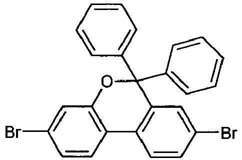 | WO 2012/60283 A1 |

### Verbindung Int-b

2,7-Dibromo-9,9-dimethyl-9H-fluoren (130 g, 369 mmol), Bis-(pinacolato)-diboran (225 g, 886 mmol) und Kaliumacetat (217 g, 2.22 mol) werden in 1.4 L Dioxan suspendiert. Die Lösung wird entgast und mit Argon gesättigt. Danach wird PdCl₂(dppf)-CH₂Cl₂ (15 g, 18 mmol) zugegeben. Die Reaktionsmischung wird für 4h unter Schutzgasatmosphäre zum Sieden erhitzt. Das Gemisch wird filtriert und mit Dioxan gewaschen. Nach Filtration des Rohproduktes wird der verbleibende Rückstand im Soxhlet mit THF extrahiert, dann filtriert. Die Ausbeute beträgt 137 g (83 % d. Th.) als grauer Feststoff. Reinheit >95 % (NMR in CDCl₃).

Analog dazu werden folgende Verbindungen hergestellt:

| **Verbindung** | 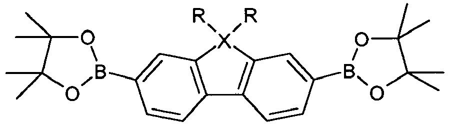 | **Ausbeute** |
|---|---|---|
| | 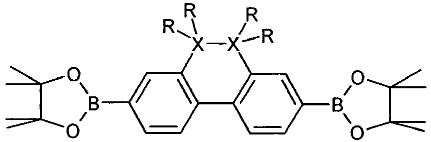 | |
| **Int-b1** | 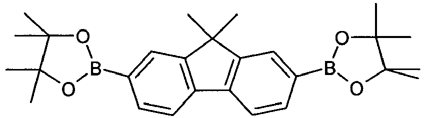 | 83% |
| **Int-b2** | 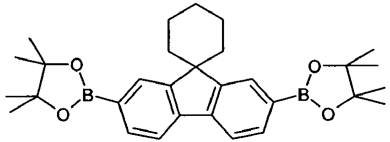 | 80% |
| **Int-b3** | 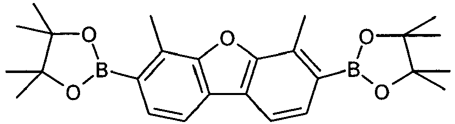 | 50% |
| **Int-b4** | 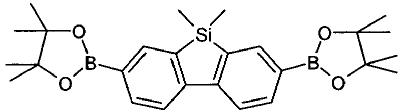 | 75% |
| **Int-b5** | 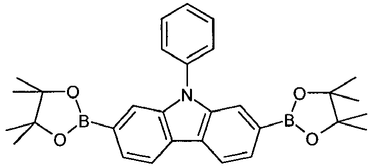 | 85% |
| **Int-b6** | 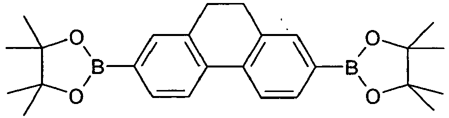 | 90% |
| **Int-b7** | 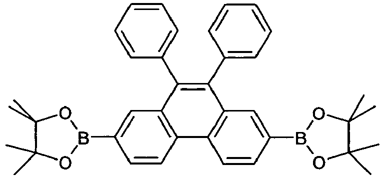 | 80% |
| **Int-b8** | 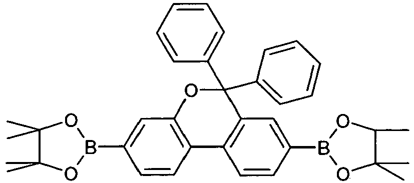 | 70% |

### Verbindung I

2,7-Bispinacolato-9,9-dimethyl-9H-fluoren (137 g, 307 mmol), 1-Bromnaphthalin-2-carbonsäure (173 g, 620 mmol) und Tri-kalium-phosphatmonohydrat (283 g, 1.23 mol) werden in einem Gemisch aus Wasser / Toluol / Dioxan (1:1:1, 1.5 L) suspendiert. Die Lösung wird entgast und mit Argon gesättigt. Danach werden Tri(o-tolyl)-phosphin (22.4 g, 73 mmol) und Palladium(II)-acetat (2.76 g, 12.3 mmol) zugegeben. Die Reaktionsmischung wird für 5.5h unter Schutzgasatmosphäre zum Sieden erhitzt. Die Phasen werden getrennt und die wässrige Phase wird mit Toluol gewaschen. Die organische Phase wird über Na₂SO₄ getrocknet und einrotiert. Das Gemisch wird über Kieselgel und AlOx mit Toluol filtriert und einrotiert. Das gelbes Öl wird im Vakuumtrockenschrank getrocknet und nicht gereinigt.

Das gelbe Öl in 500 mL THF wird in eine Mischung von Cerium(III)-trichlorid (166 g, 670 mmol) und 500 mL THF zugetropft. Die Reaktionsmischung wird für 1h auf Raumtemperatur gerührt, dann auf 0°C gekühlt. Methylmagnesiumchlorid (813 mL, 3M in THF) wird bei dieser Temperatur zugetropft. Die Reaktionsmischung wird über Nacht gerührt. Der Ansatz wird mit 500 mL Wasser versetzt und mit THF filtriert. Die Phasen der Mutterlauge werden getrennt. Die organische Phase wird über Na₂SO₄ getrocknet und einrotiert. Das gelbe Öl wird im Vakuumtrockenschrank getrocknet und nicht weiter aufgereinigt.

Das gelbe Öl und Polyphosphorsäure (446 g, 4.56 mol) werden in 1 L Dichloromethan suspendiert. Methansulfonsäure (296 mL, 4.56 mol) wird langsam zugetropft. Die Reaktionsmischung wird 1h gerührt. 700 mL Ethanol wird zugegeben. Der Ansatz wird filtriert und der verbleibende Rückstand wird in Toluol umkristallisiert. Die Ausbeute beträgt 109 g (68 % d. Th.) als gelber Feststoff. Reinheit 99.7 % (HPLC).

Analog dazu werden folgende Verbindungen hergestellt:

| **Verbindung** | 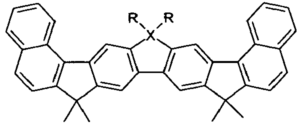 | **Ausbeute** |
|---|---|---|
| | 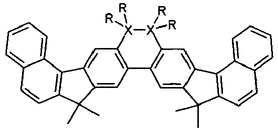 | |
| **Ia** | 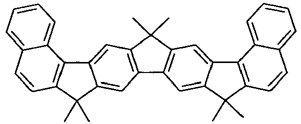 | 68% |
| **Ib** | 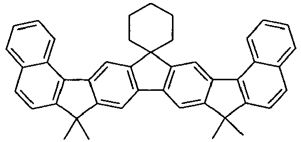 | 70% |
| **Ic** | 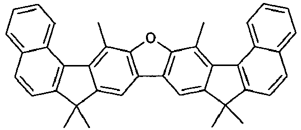 | 80% |
| **Id** | 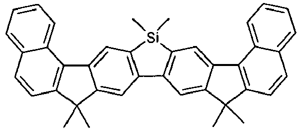 | 59% |
| **Ie** | 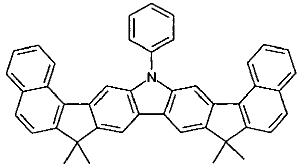 | 52% |
| **If** | 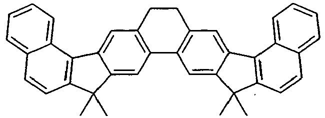 | 64% |
| **Ig** | 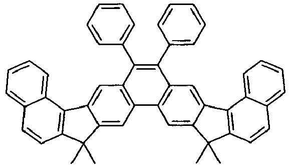 | 45% |
| **Ih** | 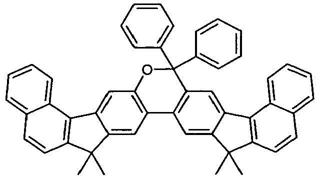 | 48% |

### Verbindung Int-c

Ia (80 g, 152 mmol) wird in 500 mL DCM gelöst. Bei 0°C wird Br₂ (16 mL, 311 mmol) in 300 mL DCM zugetropft. Die Reaktionsmischung wird über Nacht bei Raumtemperatur gerührt. 20 mL Natriumthiosulfat-Lösung wird zugegeben und 15 min gerührt. Der Ansatz wird mit Ethanol filtriert. Der verbleibende Rückstand wird dreimal in Toluol umkristallisiert. Die Ausbeute beträgt 60 g (57 % d. Th.) als grauer Feststoff. Reinheit 96.3 % (HPLC).

Analog dazu werden folgende Verbindungen hergestellt:

| **Verbindung** | 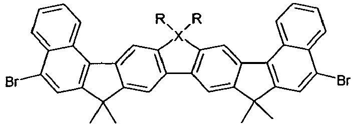 | **Ausbeute** |
|---|---|---|
| | 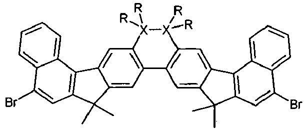 | |
| **Int-c1** | 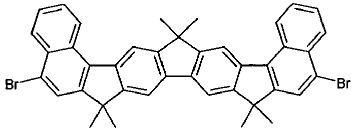 | 57% |
| **Int-c2** | 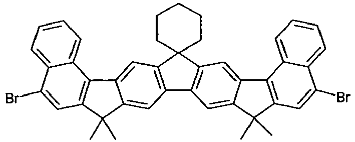 | 65% |
| **Int-c3** | 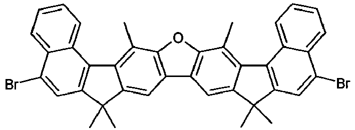 | 58% |
| **Int-c4** | 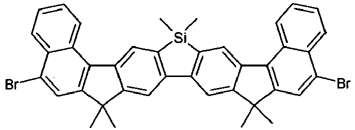 | 50% |
| **Int-c5** | 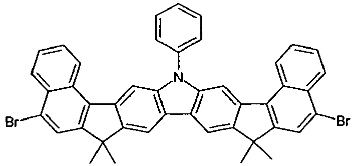 | 65% |
| **Int-c6** | 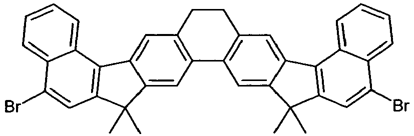 | 69% |
| **Int-c7** | 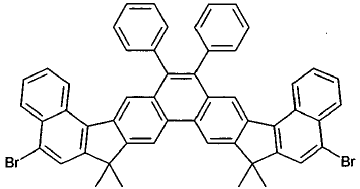 | 48% |
| **Int-c8** | 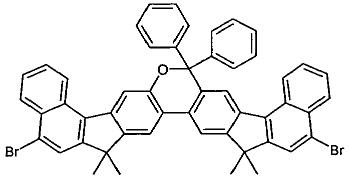 | 52% |

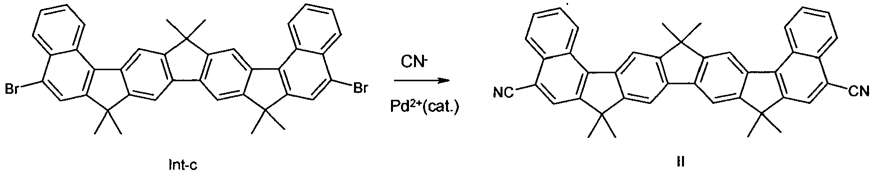

### Verbindung II

Int-c (17 g, 24 mmol), K₄[Fe(CN)₆]*3H₂O (10.5 g, 24 mmol) und Natriumcarbonat (7.9 g, 75 mmol) werden in 400 mL DMF suspendiert. Die Lösung wird entgast und mit Argon gesättigt. Danach werden SPhos (816 mg, 2 mmol) und Palladium(II)-acetat (223 mg, 1 mmol) zugegeben. Die Reaktionsmischung wird über Nacht unter Schutzgasatmosphäre zum Sieden erhitzt. Die Reaktionsmischung wird abgekühlt, dann einrotiert. Der resultierende Feststoff wird im Soxhlet-Extraktor über Aluminiumoxid mit Toluol extrahiert, dann 7x in Chloroform umkristallisiert. Die Ausbeute beträgt 2.5 g (17.5 % d. Th.) als grauer Feststoff. Reinheit 99.9 % (HPLC).

Analog dazu werden folgende Verbindungen hergestellt:

| **Verbindung** | 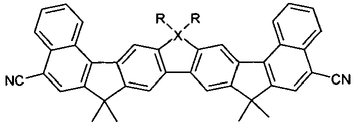 | **Ausbeute** |
|---|---|---|
| **IIa** | 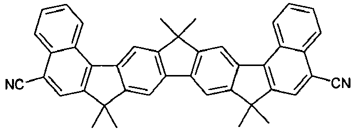 | 17.5% |
| **IIb** | 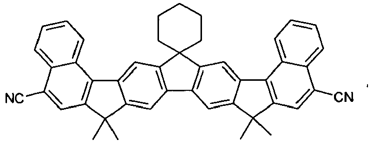 | 26% |
| **IIc** | 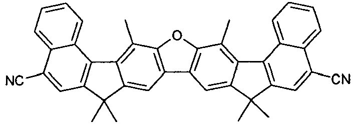 | 10% |
| **IId** | 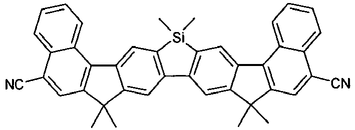 | 11% |
| **IIe** | 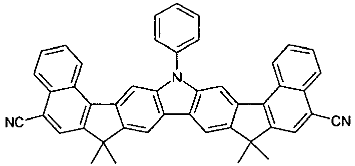 | 8% |
| **IIf** | 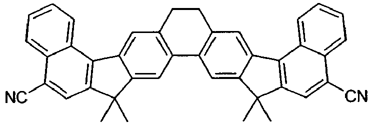 | 21% |
| **IIg** | 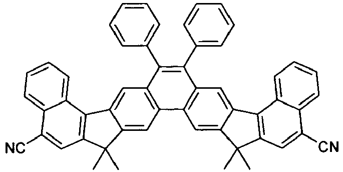 | 8% |
| **IIh** | 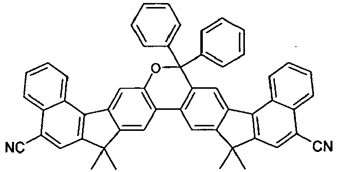 | 10% |

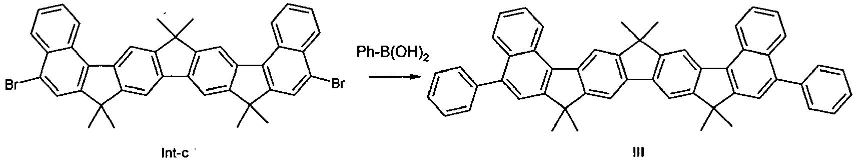

### Verbindung III

Int-c (800 mg, 1.5 mmol), Benzolboronsäure (342 mg, 3 mmol) und Trikaliumphosphatmonohydrat (1.08 g, 4.7 mmol) werden in einem Gemisch Wasser / Toluol / Dioxan (1:1:1, 6 mL) suspendiert. Die Lösung wird entgast und mit Argon gesättigt. Danach werden Tri(o-tolyl)-phosphin (43 mg, 0.14 mmol) und Palladium(II)-acetat (10 mg, 0.05 mmol) zugegeben. Die Reaktionsmischung wird über Nacht unter Schutzgasatmosphäre zum Sieden erhitzt. Die Phasen werden getrennt und die wässrige Phase wird mit Toluol gewaschen. Die organische Phase wird über Na₂SO₄ getrocknet und einrotiert. Das Gemisch wird über Kieselgel und AlOx mit Toluol filtriert und einrotiert. Der Feststoff wird in Toluol umkristallisiert. Die Ausbeute beträgt 505 mg (64 % d. Th.) als gelber Feststoff. Reinheit 99 % (HPLC).

Analog dazu werden folgende Verbindungen hergestellt:

| **Verbindung** | 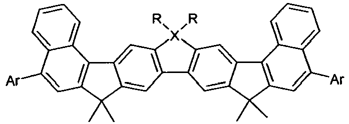 | **Ausbeute** |
|---|---|---|
| **IIIa** | 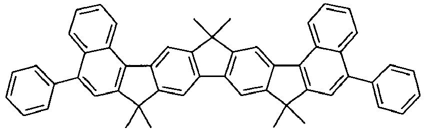 | 64% |
| **IIIb** | 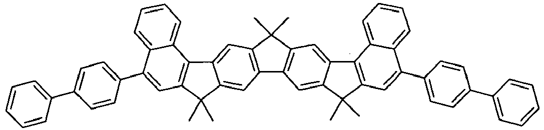 | 42% |
| **IIIc** | 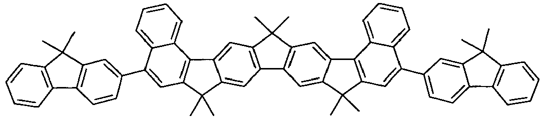 | 59% |
| **IIId** | 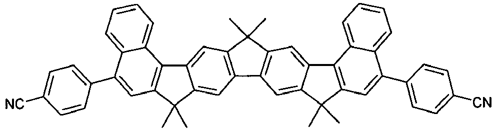 | 5% |
| **IIIe** | 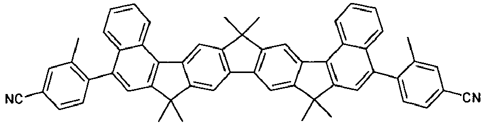 | 12% |
| **IIIf** | 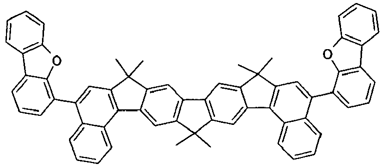 | 26% |
| **IIIg** | 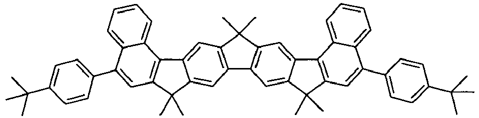 | 46% |
| **IIIh** | 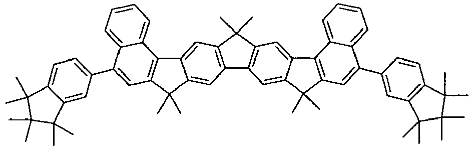 | 8% |
| **IIIi** | 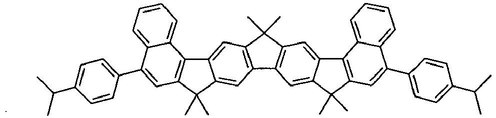 | 12% |
| **IIIj** | 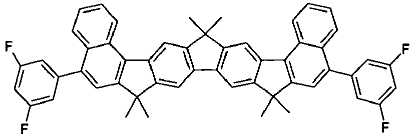 | 83% |
| **IIIk** | 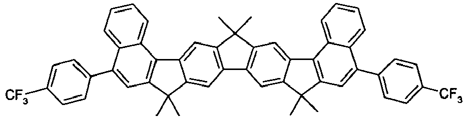 | 21% |
| **IIIl** | 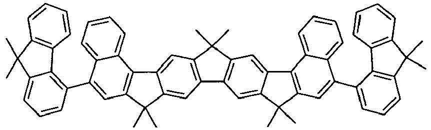 | 28% |
| **IIIm** | 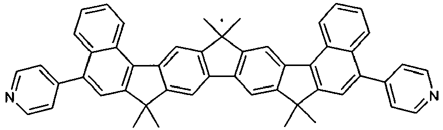 | 25% |
| **IIIn** | 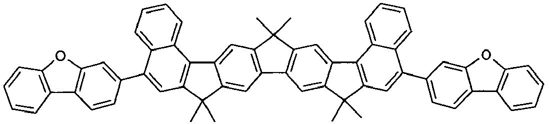 | 15% |
| **IIIo** | 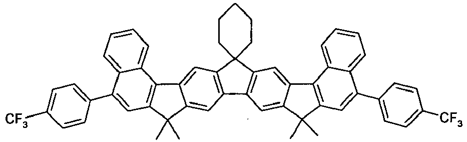 | 78% |
| **IIIq** | 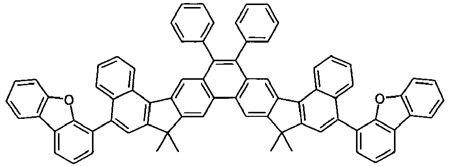 | 18% |
| **IIIr** | 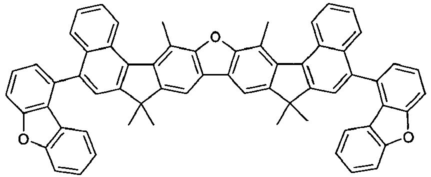 | 51% |

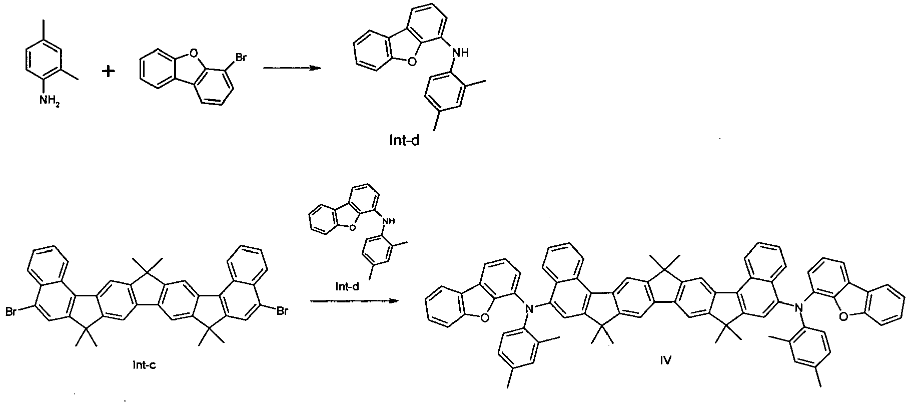

### Verbindung Int-d

2,4-Dimethylphenylamin (1.12 mL, 8.9 mmol), 4-Brom-dibenzofuran (2 g, 8.1 mmol) und Natrium-tert-butoxid (1.9 g, 20 mmol) werden in 150 mL Toluol suspendiert. Die Lösung wird entgast und mit Argon gesättigt. Danach wird PdCl₂(dppf)-CH₂Cl₂ (132 mg, 162 mmol) zugegeben. Die Reaktionsmischung wird über Nacht unter Schutzgasatmosphäre zum Sieden erhitzt. Das Gemisch wird über Kieselgel und AlOx mit Toluol filtriert und einrotiert. Das Öl wird über eine Kieselgelsäule mit Heptan gereinigt. Die Ausbeute beträgt 1.9 g (82 % d. Th.) als hellbraunes Öl. Reinheit 94 % (HPLC).

Analog dazu werden folgende Verbindungen hergestellt:

| **Verbindung** | 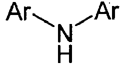 | **Ausbeute** |
|---|---|---|
| **Int-d1** | 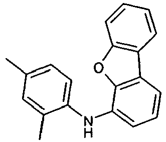 | 82% |
| **Int-d2** | 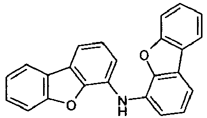 | 30% |
| **Int-d3** | 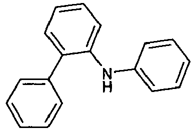 | 80% |
| **Int-d4** | 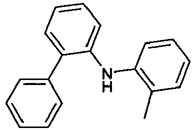 | 85% |
| **Int-d5** | 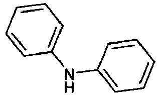 | 90% |
| **Int-d6** | 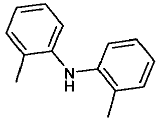 | 88% |

### Verbindungen IVa und IVb

Int-c (1g, 1.46 mmol), die Dibenzofuranverbindung (903 mg, 3.14 mmol) und Natrium-tert-butoxid (421 mg, 4.38 mmol) werden in 40 mL Toluol suspendiert. Die Lösung wird entgast und mit Argon gesättigt. Danach werden Tri-tert-butylphosphin (117 µL, 1M in Toluol) und Palladium(II)acetat (48 mg, 0.06 mmol) zugegeben. Die Reaktionsmischung wird 4h unter Schutzgasatmosphäre zum Sieden erhitzt. Das Gemisch wird über Kieselgel und AlOx mit Toluol filtriert und einrotiert. Das Produkt wird dreimal mit Toluol umkristallisiert. Die Ausbeute beträgt 200 mg (13 % d. Th.) als hellbraunes Öl. Reinheit 96.7 % (HPLC).

| **Verbindung** | 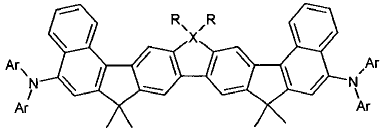 | **Ausbeute** |
|---|---|---|
| **IVa** | 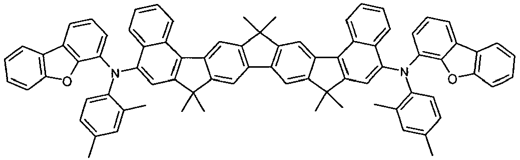 | 13% |
| **IVb** | 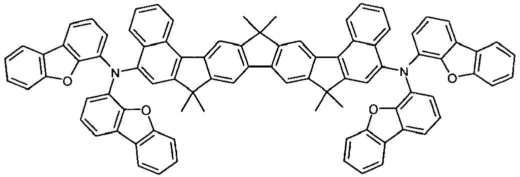 | 16% |
| **IVc** | 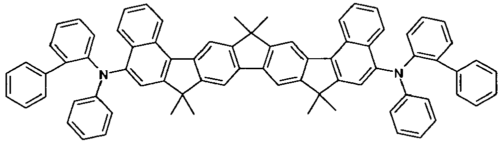 | 8% |
| **IVd** | 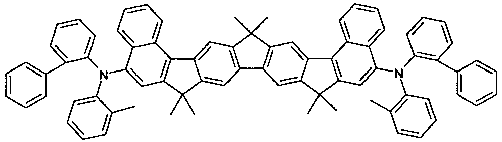 | 12% |
| **IVe** | 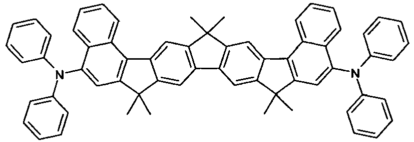 | 80% |
| **IVf** | 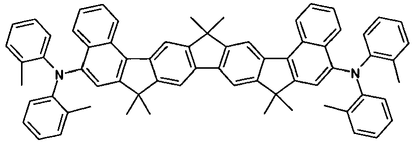 | 8% |
| **IVg** | 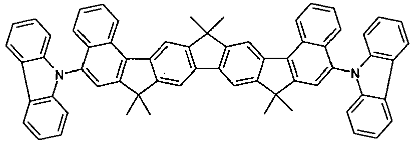 | 10% |

### A-2) Variante II

Es wird nach folgendem allgemeinen Schema vorgegangen:

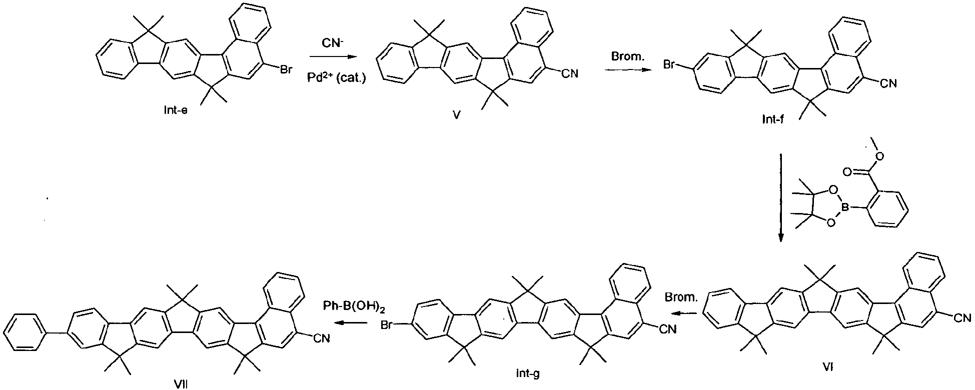

### Verbindung V

Int-e (10 g, 20 mmol), K₄[Fe(CN)₆]*3H₂O (4.3 g, 10 mmol) und Natriumcarbonat (3.3 g, 31 mmol) werden in 150 mL DMF suspendiert. Die Lösung wird entgast und mit Argon gesättigt. Danach werden SPhos (336 mg, 0.82 mmol) und Palladium(II)-acetat (92 mg, 0.41 mmol) zugegeben. Die Reaktionsmischung wird über Nacht unter Schutzgasatmosphäre zum Sieden erhitzt. Die Reaktionsmischung wird abgekühlt, dann einrotiert. Der resultierende Feststoff wird im Soxhlet-Extraktor über Aluminiumoxid mit Toluol extrahiert, dann 7x in Chloroform umkristallisiert. Die Ausbeute beträgt 5.3 g (67 % d. Th.) als gelber Feststoff. Reinheit 99.6 % (HPLC).

### Verbindung Int-f

Verbindung V (3 g, 7.7 mmol) wird in 25 mL DCM gelöst. Bei 0°C wird Br₂ (394 µL, 7.7 mmol) in 25 mL DCM zugetropft. Die Reaktionsmischung wird über Nacht bei Raumtemperatur gerührt. 10 mL Natriumthiosulfat-Lösung wird zugegeben und 15 min gerührt. Der Ansatz wird mit Ethanol filtriert. Der verbleibende Rückstand wird dreimal in Heptan/Toluol 1:1 umkristallisiert. Die Ausbeute beträgt 1.7 g (44 % d. Th.) als gelber Feststoff. Reinheit 94 % (HPLC).

### Verbindung VI

Verbindung Int-f (1 g, 2.2 mmol), 1-Brom-naphthalin-2-Carbonsäure (649 mg, 2.5 mmol) und Trikaliumphosphat Monohydrat (1.5 g, 6.5 mmol) werden in einem Gemisch Wasser / Toluol / Dioxan (1:1:1, 30 mL) suspendiert. Die Lösung wird entgast und mit Argon gesättigt. Danach werden Tri(o-tolyl)-phosphin (79 mg, 0.3 mmol) und Palladium(II)-acetat (9.7 mg, 0.04 mmol) zugegeben. Die Reaktionsmischung wird für 7h unter Schutzgasatmosphäre zum Sieden erhitzt. Die Phasen werden getrennt und die wässrige Phase mit Toluol gewaschen. Die organische Phase wird über Na₂SO₄ getrocknet und einrotiert. Das Gemisch wird über Kieselgel und AlOx mit Toluol filtriert und einrotiert. Der gelbe Feststoff wird im Vakuumtrockenschrank getrocknet und nicht weiter aufgereinigt .

Der gelbe Feststoff in 20 mL THF wird in eine Mischung von Cer(III)-trichlorid (600 mg, 2.37 mmol) und 20 mL THF zugetropft. Die Reaktionsmischung wird für 1h auf Raumtemperatur gerührt, dann auf 0°C gekühlt. Methylmagnesiumchlorid (1.25 mL, 3M in THF) wird bei dieser Temperatur zugetropft. Die Reaktionsmischung wird über Nacht gerührt. Der Ansatz wird mit 20 mL Wasser versetzt und mit THF filtriert. Die Phasen der Mutterlauge werden getrennt. Die organische Phase wird über Na₂SO₄ getrocknet und einrotiert. Der gelbe Feststoff wird im Vakuumtrockenschrank getrocknet und nicht gereinigt.

Der gelbe Feststoff und Polyphosphorsäure (2.1 g, 21.5 mol) werden in 30 mL Dichloromethan suspendiert. Methansulfonsäure (1.4 mL, 21.5 mol) wird langsam zugetropft. Die Reaktionsmischung wird 1h gerührt. 30 mL Ethanol wird zugegeben. Der Ansatz wird filtriert und der verbleibende Rückstand wird in Toluol umkristallisiert. Die Ausbeute beträgt 809 mg (75 % d. Th.) als gelber Feststoff. Reinheit 97 % (HPLC).

### Verbindung Int-g

Verbindung VI (809 mg, 1.6 mmol) wird in 25 mL DCM gelöst. Bei 0°C wird Br₂ (83 µL, 1.6 mmol) in 25 mL DCM zugetropft. Die Reaktionsmischung wird über Nacht bei Raumtemperatur gerührt. 10 mL Natriumthiosulfat-Lösung wird zugegeben und 15 min gerührt. Der Ansatz wird mit Ethanol filtriert. Der verbleibende Rückstand wird dreimal in Heptan/Toluol 1:1 umkristallisiert. Die Ausbeute beträgt 790 mg (85 % d. Th.) als gelber Feststoff. Reinheit 96% (HPLC).

### Verbindung VII

Int-g (790 mg, 1.3 mmol), Benzolboronsäure (342 mg, 3 mmol) und Trkaliumphosphat Monohydrat (1.08 g, 4.7 mmol) werden in einem Gemisch Wasser / Toluol / Dioxan (1:1:1, 6 mL) suspendiert. Die Lösung wird entgast und mit Argon gesättigt. Danach werden Tri(*o*-tolyl)-phosphin (43 mg, 0.14 mmol) und Palladium(II)-acetat (10 mg, 0.05 mmol) zugegeben. Die Reaktionsmischung wird über Nacht unter Schutzgasatmosphäre zum Sieden erhitzt. Die Phasen werden getrennt, und die wässrige Phase wird mit Toluol gewaschen. Die organische Phase wird über Na₂SO₄ getrocknet und einrotiert. Das Gemisch wird über Kieselgel und AlOx mit Toluol filtriert und einrotiert. Der Feststoff wird in Toluol umkristallisiert. Die Ausbeute beträgt 675 mg (73 % d. Th.) als gelber Feststoff. Reinheit 97 % (HPLC).

Analog dazu werden folgende Verbindungen hergestellt:

| **Verbindung** | 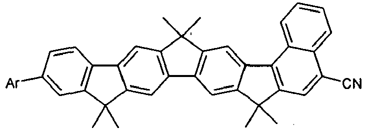 | **Ausbeute** |
|---|---|---|
| **Vlla** | 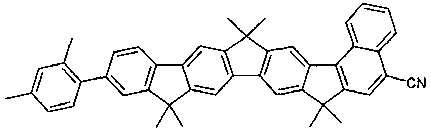 | 55% |
| **Vllb** | 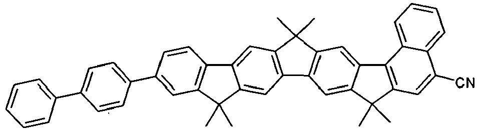 | 69% |

### B) Devicebeispiele: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen (siehe Tabellen 1 bis 3) werden die Daten verschiedener OLEDs vorgestellt. Als Substrate werden Glassubstrate verwendet, die mit strukturiertem ITO (Indium Zinn Oxid) der Dicke 50 nm beschichtet sind. Die OLEDs haben prinzipiell folgenden Schichtaufbau: Substrat / Lochinjektionsschicht1 (95% HTL1+ 5% HIL, 20 nm) / Lochtransportschicht (HTL, Dicke in Tabelle 1 angegeben) / Emissionsschicht (EML, 20nm) / Elektronentransportschicht (ETL, 20 nm) / Elektroneninjektionsschicht (EIL, 3 nm) und abschließend eine Kathode. Die Kathode wird durch eine 100 nm dicke Aluminiumschicht gebildet. Als Buffer wird eine 20nm dicke Schicht Clevios P VP AI 4083 (bezogen von Heraeus Clevios GmbH, Leverkusen) durch Spincoating aufgebracht. Alle restlichen Materialien werden in einer Vakuumkammer thermisch aufgedampft. Der Aufbau der OLEDs ist in Tabelle 1 gezeigt. Die verwendeten Materialien sind in Tabelle 3 gezeigt.

Die Emissionsschicht (EML) besteht immer aus mindestens einem Matrixmaterial (Host=H) und einer emittierenden Verbindung (Dotand=D), die dem Matrixmaterial durch Coverdampfung in einem bestimmten Volumenanteil beigemischt wird. Eine Angabe wie H1:D1 (97%:3%) bedeutet hierbei, dass das Material H1 in einem Volumenanteil von 97% und D1 in einem Anteil von 3% in der Schicht vorliegt.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet und abschließend die Lebensdauer der Bauteile bestimmt. Die Elektrolumineszenzspektren werden bei einer Leuchtdichte von 1000 cd/m² aufgenommen und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². Die Lebensdauer LD95 @ 1000 cd/m² ist die Zeit, die vergeht, bis die Starthelligkeit von 1000 cd/m² um 5% gesunken ist. Die erhaltenen Daten für die verschiedenen OLEDs sind in Tabelle 2 zusammengefasst.

### Verwendung der erfindungsgemäßen Verbindungen als Emitter in fluoreszierenden OLEDs

Es werden die erfindungsgemäßen Verbindungen D3, D4, D5, D6, D7, D8, D9, D10, D11 und D12 einzeln als Emitter in der emittierenden Schicht von OLEDs eingesetzt (Struktur s. Tabelle 3). Als Matrixmaterial der emittierenden Schicht wird dabei die Verbindung V-H2 verwendet. Die erhaltenen OLEDs sind E4 bis E6 und E9 bis E15. Sie zeigen sehr gute Lebensdauer bei tiefblauer Emission (Tabelle 2). Verglichen mit im Stand der Technik bekannten Emittermaterialien (V-D1 und V-D2, vgl. V1 bis V3) ist die Lebensdauer stark verbessert, bei gleichbleibender Quanteneffizienz.

Insbesondere der Vergleich mit dem Material V-D1 zeigt die Verbesserung, die durch das erfindungsgemäße Bis-Indenofluoren-Grundgerüst gegenüber dem im Stand der Technik bekannten Indenofluoren-Grundgerüst erzielt wird.

### Verwendung der erfindungsgemäßen Verbindungen als Matrixmaterialien in fluoreszierenden OLEDs

Beispiel E7, in dem die erfindungsgemäße Verbindung H3 als Matrixmaterial eingesetzt wird, zeigt ebenfalls gute Lebensdauer und Quanteneffizienz bei tiefblauer Emission (Tabelle 2). Dies belegt die gute Eignung der erfindungsgemäßen Verbindungen als Matrixmaterialien in der emittierenden Schicht.

### Verwendung der erfindungsgemäßen Verbindungen als Lochtransportmaterialien in OLEDs

Beispiel E8, in dem die erfindungsgemäße Verbindung H3 als Lochtransportmaterial in der Lochtransportschicht eingesetzt wird, zeigt ebenfalls gute Lebensdauer und Quanteneffizienz bei tiefblauer Emission (Tabelle 2). Dies belegt die gute Eignung der erfindungsgemäßen Verbindungen als lochtransportierende Verbindungen.

| | **Tabelle 1: Aufbau der OLEDs** | |
|---|---|---|
| **Bsp.** | **HTL** | **EML** |
| | Material | Material |
| | Dicke/nm | |
| V1 | HTL3 | V-H1(97%):V-D1(3%) |
| | 195nm | |
| V2 | HTL3 | V-H2(97%):V-D1 (3%) |
| | 195nm | |
| V3 | HTL3 | V-H2(97%):V-D2(3%) |
| | 195nm | |
| E4 | HTL3 | V-H2(97%):D3(3%) |
| | 195nm | |
| E5 | HTL3 | V-H2(97%): D4(3%) |
| | 195nm | |
| E6 | HTL3 | V-H2(97%):D5(3%) |
| | 195nm | |
| E7 | HTL2 | H3(95%):D3(1%) |
| | 20nm | |
| E8 | D3 | V-H2(95%)V-D2(5%) |
| | 20nm | |
| E9 | HTL3 | V-H2(97%):D6(3%) |
| | 195nm | |
| E10 | HTL3 | V-H2(97%):D7(3%) |
| | 195nm | |
| E11 | HTL3 | V-H2(97%):D8(3%) |
| | 195nm | |
| E12 | HTL3 | V-H2(97%):D9(3%) |
| | 195nm | |
| E13 | HTL3 | V-H2(97%):D10(3%) |
| | 195nm | |
| E14 | HTL3 | V-H2(97%):D11(3%) |
| | 195nm | |
| E15 | HTL3 | V-H2(97%):D12(3%) |
| | 195nm | |

| **Tabelle 2: Daten der OLEDs** | | | | |
|---|---|---|---|---|
| ***Bsp.*** | ***EQE* @ *1000 cd*/*m²*** | ***LD95* @ *1000cd*/*m²*** | ***CIE*** | |
| | % | *[h]* | *x* | y |
| *V1* | *7.5* | *90* | 0.13 | 0.13 |
| *V2* | *7.8* | *110* | 0.13 | 0.14 |
| *V3* | *7.9* | *90* | 0.13 | 0.10 |
| *E4* | *7.4* | *350* | 0.14 | 0.11 |
| *E5* | *7.6* | *320* | 0.14 | 0.14 |
| *E6* | *6.9* | *150* | 0.14 | 0.13 |
| *E7* | *6.9* | *120* | 0.13 | 0.13 |
| *E8* | *7.3* | *140* | 0.13 | 0.14 |
| *E9* | *7.0* | *140* | 0.14 | 0.11 |
| *E10* | *7.2* | *370* | 0.14 | 0.14 |
| *E11* | *7.8* | *140* | 0.13 | 0.10 |
| *E12* | *7.6* | *280* | 0.14 | 0.25 |
| *E13* | *7.5* | *270* | 0.13 | 0.09 |
| *E14* | *7.3* | *280* | 0.14 | 0.11 |
| *E15* | *7.8* | *350* | 0.14 | 0.26 |

| **Tabelle 3: Strukturen der verwendeten Materialien** | |
|---|---|
| 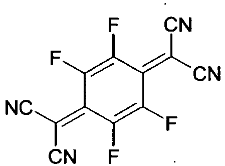 | 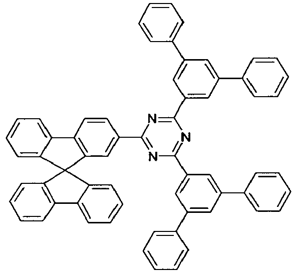 |
| HIL | ETL |
| 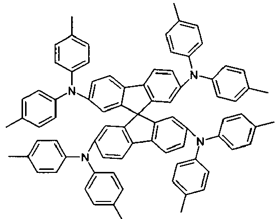 | 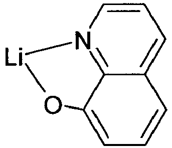 |
| HTL1 | EIL |
| 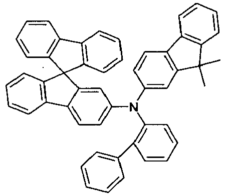 | 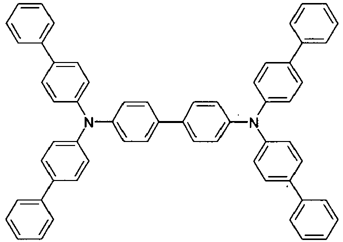 |
| HTL2 | HTL3 |
| 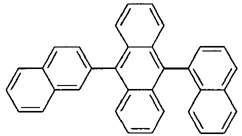 | 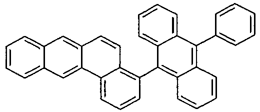 |
| V-H1 | V-H2 |
| 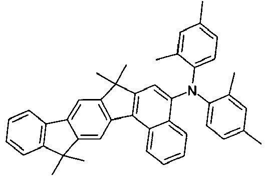 | 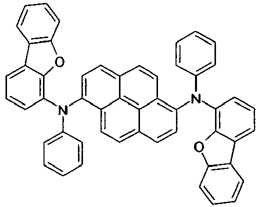 |
| V-D1 | V-D2 |
| 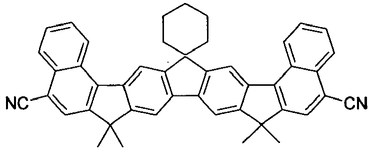 | 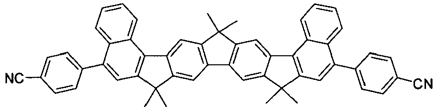 |
| D3 | D4 |
| 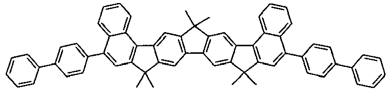 | 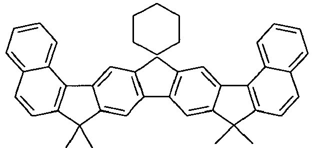 |
| D5 | H3 |
| 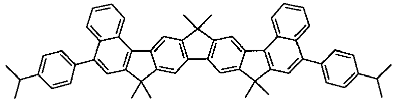 | 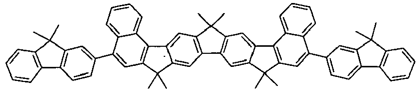 |
| D6 | D7 |
| 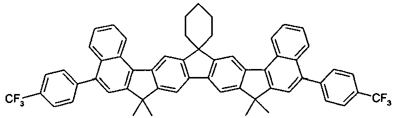 | 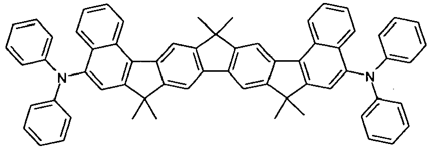 |
| D8 | D9 |
| 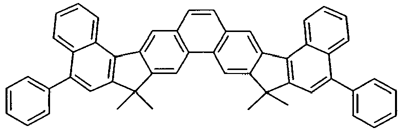 | 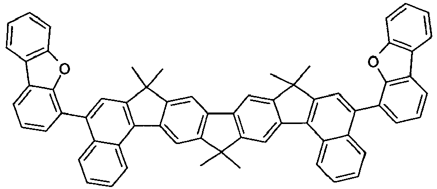 |
| D10 | D11 |
| 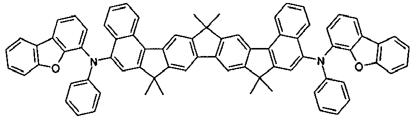 | |
| D12 | |

## Patentansprüche

1. Verbindung gemäß einer Formel (I) 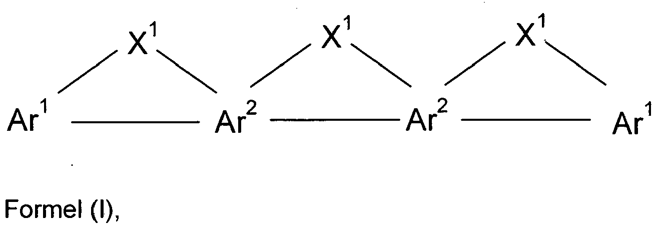 wobei gilt:
Ar¹ ist bei jedem Auftreten gleich oder verschieden eine Aryl- oder Heteroarylgruppe mit 6 bis 18 aromatischen Ringatomen, die mit einem oder mehreren Resten R¹ substituiert sein kann;
Ar² ist bei jedem Auftreten gleich oder verschieden eine Aryl- oder Heteroarylgruppe mit 6 aromatischen Ringatomen, die mit einem oder mehreren Resten R² substituiert sein kann;
X¹ ist bei jedem Auftreten gleich oder verschieden BR³, C(R³)₂, -C(R³)₂-C(R³)₂-, -C(R³)₂-O-, -C(R³)₂-S-,-R³C=CR³-, -R³C=N-, Si(R³)₂, -Si(R³)₂-Si(R³)₂-, C=O, O, S, S=O, SO₂, NR³, PR³ oder P(=O)R³;
R¹, R², R³ ist bei jedem Auftreten gleich oder verschieden H, D, F, Cl, Br, I, C(=O)R⁴, CN, Si(R⁴)₃, N(R⁴)₂, P(=O)(R⁴)₂, OR⁴, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁵ substituiert sein kann, wobei zwei oder mehr Reste R⁴ miteinander verknüpft sein können und einen Ring bilden können;
R⁵ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁵ miteinander verknüpft sein und einen Ring bilden;
wobei mindestens eine der beiden Gruppen Ar¹ 10 oder mehr aromatische Ringatome aufweisen muss; und
wobei wenn eine der beiden Gruppen Ar¹ eine Phenylgruppe ist, die andere der beiden Gruppen Ar¹ nicht mehr als 14 aromatische Ringatome aufweisen darf.

2. Verbindung nach Anspruch 1, **dadurch gekennzeichnet, dass** an den Gruppen Ar² die Bindungen zur benachbarten Gruppe Ar¹ bzw. Ar² jeweils in para-Position zueinander vorliegen.

3. Verbindung nach Anspruch 1 oder 2, **dadurch gekennzeichnet, dass** die Gruppen Ar¹ bei jedem Auftreten gleich oder verschieden gewählt sind aus Arylgruppen oder Heteroarylgruppen mit 6 bis 14 aromatischen Ringatomen, die jeweils mit einem oder mehreren Resten R¹ substituiert sein können.

4. Verbindung nach Anspruch 3, **dadurch gekennzeichnet, dass** die Gruppen Ar¹ bei jedem Auftreten gewählt sind aus Arylgruppen oder Heteroarylgruppen mit 6 bis 10 aromatischen Ringatomen, die jeweils mit einem oder mehreren Resten R¹ substituiert sein können.

5. Verbindung nach einem oder mehreren der Ansprüche 1 bis 4, **dadurch gekennzeichnet, dass** die Gruppen Ar² Phenylgruppen sind, die mit einem oder mehreren Resten R² substituiert sein können.

6. Verbindung nach einem oder mehreren der Ansprüche 1 bis 5, **dadurch gekennzeichnet, dass** die Gruppen Ar¹ Naphthylgruppen sind, die mit einem oder mehreren Resten R¹ substituiert sein können, und die Gruppen Ar² Phenylgruppen sind, die mit einem oder mehreren Resten R² substituiert sein können.

7. Verbindung nach einem oder mehreren der Ansprüche 1 bis 5, **dadurch gekennzeichnet, dass** eine der beiden Gruppen Ar¹ eine Phenylgruppe ist, die mit einem oder mehreren Resten R¹ substituiert sein kann, und die andere der beiden Gruppen Ar¹ eine Naphthylgruppe ist, die mit einem oder mehreren Resten R¹ substituiert sein kann, und die Gruppen Ar² Phenylgruppen sind, die mit einem oder mehreren Resten R² substituiert sein können.

8. Verbindung nach einem oder mehreren der Ansprüche 1 bis 7, **dadurch gekennzeichnet, dass** X¹ bei jedem Auftreten gleich oder verschieden gewählt ist aus C(R³)₂, -C(R³)₂-C(R³)₂-, -C(R³)₂-O-, Si(R³)₂, O, S, und NR³.

9. Verbindung nach einem oder mehreren der Ansprüche 1 bis 8, **dadurch gekennzeichnet, dass** X¹ gleich C(R³)₂ ist.

10. Verbindung nach einem oder mehreren der Ansprüche 1 bis 9, **dadurch gekennzeichnet, dass** R² gleich H oder D ist.

11. Oligomer, Polymer oder Dendrimer enthaltend eine oder mehrere Verbindungen nach einem oder mehreren der Ansprüche 1 bis 10, wobei die Bindung(en) zum Polymer, Oligomer oder Dendrimer an beliebigen, in Formel (I) mit R¹, R² oder R³ substituierten Positionen lokalisiert sein können.

12. Formulierung, enthaltend mindestens eine Verbindung nach einem oder mehreren der Ansprüche 1 bis 10, oder mindestens ein Oligomer, Polymer oder Dendrimer nach Anspruch 11, sowie mindestens ein Lösungsmittel.

13. Elektronische Vorrichtung, ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), enthaltend mindestens eine Verbindung nach einem oder mehreren der Ansprüche 1 bis 10, oder mindestens ein Oligomer, Polymer oder Dendrimer nach Anspruch 11.

14. Elektronische Vorrichtung nach Anspruch 13, ausgewählt aus organischen Elektrolumineszenzvorrichtungen, enthaltend Kathode, Anode und mindestens eine organische Schicht, wobei die mindestens eine organische Schicht mindestens eine Verbindung nach einem oder mehreren der Ansprüche 1 bis 10 oder mindestens ein Oligomer, Polymer oder Dendrimer nach Anspruch 11 enthält.

15. Elektronische Vorrichtung nach Anspruch 14, **dadurch gekennzeichnet, dass** die Verbindung nach einem oder mehreren der Ansprüche 1 bis 10 oder das Oligomer, Polymer oder Dendrimer nach Anspruch 11 als Lochtransportmaterial in einer Lochtransportschicht, als emittierende Verbindung in einer emittierenden Schicht oder als Matrixverbindung in einer emittierenden Schicht vorliegt.

16. Verwendung einer Verbindung nach einem oder mehreren der Ansprüche 1 bis 10 oder eines Oligomers, Polymers oder Dendrimers nach Anspruch 11 in einer elektronischen Vorrichtung.

17. Verfahren zur Herstellung einer Verbindung nach einem oder mehreren der Ansprüche 1 bis 10, **dadurch gekennzeichnet, dass** es mindestens eine metalkatalysierte Kupplungsreaktion und mindestens eine Ringschlussreaktion umfasst.

## Claims

1. Compound of a formula (I) 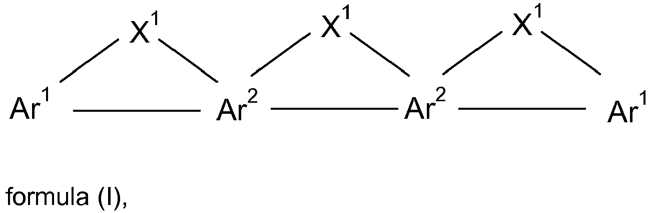 where:
Ar¹ is on each occurrence, identically or differently, an aryl or heteroaryl group having 6 to 18 aromatic ring atoms, which may be substituted by one or more radicals R¹;
Ar² is on each occurrence, identically or differently, an aryl or heteroaryl group having 6 aromatic ring atoms, which may be substituted by one or more radicals R²;
X¹ is on each occurrence, identically or differently, BR³, C(R³)₂, -C(R³)₂-C(R³)₂-, -C(R³)₂-O-, -C(R³)₂-S-,-R³C=CR³-, -R ³C=N-, Si(R³)₂, -Si(R³)₂-Si(R³)₂-, C=O, O, S, S=O, SO₂, NR³, PR³ or P(=O)R³;
R¹, R², R³ are on each occurrence, identically or differently, H, D, F, Cl, Br, I, C(=O)R⁴, CN, Si(R⁴)₃, N(R⁴)₂, P(=O)(R⁴)₂, OR⁴, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may in each case be substituted by one or more radicals R⁴, where two or more radicals R³ may be linked to one another and may form a ring;
R⁴ is on each occurrence, identically or differently, H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R⁵ and where one or more CH₂ groups in the above-mentioned groups may be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂, or an aromatic or heteroaromatic ring system having 5 to 30 aromatic ring atoms, which may in each case be substituted by one or more radicals R⁵, where two or more radicals R⁴ may be linked to one another and may form a ring;
R⁵ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁵ here may be linked to one another and may form a ring;
where at least one of the two groups Ar¹ must contain 10 or more aromatic ring atoms; and
where, if one of the two groups Ar¹ is a phenyl group, the other of the two groups Ar¹ must not contain more than 14 aromatic ring atoms.

2. Compound according to Claim 1, **characterised in that** the bonds from the groups Ar² to the adjacent group Ar¹ or Ar² are in each case present in the para-position to one another.

3. Compound according to Claim 1 or 2, **characterised in that** the groups Ar¹ are selected on each occurrence, identically or differently, from aryl groups or heteroaryl groups having 6 to 14 aromatic ring atoms, which may in each case be substituted by one or more radicals R¹.

4. Compound according to Claim 3, **characterised in that** the groups Ar¹ are selected on each occurrence from aryl groups or heteroaryl groups having 6 to 10 aromatic ring atoms, which may in each case be substituted by one or more radicals R¹.

5. Compound according to one or more of Claims 1 to 4, **characterised in that** the groups Ar² are phenyl groups, which may be substituted by one or more radicals R².

6. Compound according to one or more of Claims 1 to 5, **characterised in that** the groups Ar¹ are naphthyl groups, which may be substituted by one or more radicals R¹, and the groups Ar² are phenyl groups, which may be substituted by one or more radicals R².

7. Compound according to one or more of Claims 1 to 5, **characterised in that** one of the two groups Ar¹ is a phenyl group, which may be substituted by one or more radicals R¹, and the other of the two groups Ar¹ is a naphthyl group, which may be substituted by one or more radicals R¹, and the groups Ar² are phenyl groups, which may be substituted by one or more radicals R².

8. Compound according to one or more of Claims 1 to 7, **characterised in that** X¹ is selected on each occurrence, identically or differently, from C(R³)₂, -C(R³)₂-C(R³)₂-, -C(R³)₂-O-, Si(R³)₂, O, S, and NR³.

9. Compound according to one or more of Claims 1 to 8, **characterised in that** X¹ is equal to C(R³)₂.

10. Compound according to one or more of Claims 1 to 9, **characterised in that** R² is equal to H or D.

11. Oligomer, polymer or dendrimer containing one or more compounds according to one or more of Claims 1 to 10, where the bond(s) to the polymer, oligomer or dendrimer may be localised at any desired positions in formula (I) which are substituted by R¹, R² or R³.

12. Formulation comprising at least one compound according to one or more of Claims 1 to 10, or at least one oligomer, polymer or dendrimer according to Claim 11, and at least one solvent.

13. Electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), comprising at least one compound according to one or more of Claims 1 to 10, or at least one oligomer, polymer or dendrimer according to Claim 11.

14. Electronic device according to Claim 13, selected from organic electroluminescent devices comprising cathode, anode and at least one organic layer, where the at least one organic layer comprises at least one compound according to one or more of Claims 1 to 10 or at least one oligomer, polymer or dendrimer according to Claim 11.

15. Electronic device according to Claim 14, **characterised in that** the compound according to one or more of Claims 1 to 10 or the oligomer, polymer or dendrimer according to Claim 11 is present as hole-transport material in a hole-transport layer, as emitting compound in an emitting layer or as matrix compound in an emitting layer.

16. Use of a compound according to one or more of Claims 1 to 10 or an oligomer, polymer or dendrimer according to Claim 11 in an electronic device.

17. Process for the preparation of a compound according to one or more of Claims 1 to 10, **characterised in that** it comprises at least one metal-catalysed coupling reaction and at least one ring-closure reaction.

## Revendications

1. Composé d'une formule (I) : 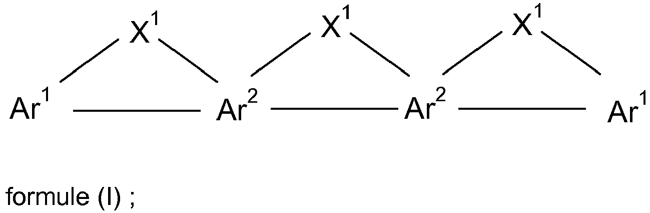 dans laquelle :
Ar¹ est pour chaque occurrence, de manière identique ou différente, un groupe aryle ou hétéroaryle qui comporte de 6 à 18 atomes de cycle aromatique, lequel peut être substitué par un radical ou par plusieurs radicaux R¹ ;
Ar² est pour chaque occurrence, de manière identique ou différente, un groupe aryle ou hétéroaryle qui comporte 6 atomes de cycle aromatique, lequel peut être substitué par un radical ou par plusieurs radicaux R² ;
X¹ est pour chaque occurrence, de manière identique ou différente, BR³,
C(R³)₂, -C(R³)₂-C(R³)₂-, -C(R³)₂-O-, -C(R³)₂-S-, -R³C=CR³-, -R ³C=N-, Si(R³)₂, -Si(R³)₂-Si(R³)₂-, C=O, O, S, S=O, SO₂, NR³, PR³ ou P(=O)R³;
R¹, R², R³ sont pour chaque occurrence, de manière identique ou différente, H, D, F, Cl, Br, I, C(=O)R⁴, CN, Si(R⁴)₃, N(R⁴)₂, P(=O)(R⁴)₂, OR⁴, S(=O)R⁴, S(=O)₂R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O,
C=NR⁴, -C(=O)O-, -C(=O)NR⁴-, NR⁴, P(=O)(R⁴), -O-, -S-, SO ou SO₂, ou un système de cycle aromatique ou hétéroaromatique qui comporte de 5 à 30 atomes de cycle aromatique, lequel peut dans chaque cas être substitué par un radical ou par plusieurs radicaux R⁴, où deux radicaux R³ ou plus peuvent être liés l'un à l'autre ou les uns aux autres et peuvent former un cycle ;
R⁴ est pour chaque occurrence, de manière identique ou différente, H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R⁵ et où un ou plusieurs groupe(s) CH₂ dans les groupes qui ont été mentionnés ci-avant peut/peuvent être remplacé(s) par -R⁵C=CR⁵-, -C≡C-, Si(R⁵)₂, C=O,
C=NR⁵, -C(=O)O-, -C(=O)NR⁵-, NR⁵, P(=O)(R⁵), -O-, -S-, SO ou SO₂, ou un système de cycle aromatique ou hétéroaromatique qui comporte de 5 à 30 atomes de cycle aromatique, lequel peut dans chaque cas être substitué par un radical ou par plusieurs radicaux R⁵, où deux radicaux R⁴ ou plus peuvent être liés l'un à l'autre ou les uns aux autres et peuvent former un cycle ;
R⁵ est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/peuvent être remplacé(s) par D ou F ; deux substituants R⁵ ou plus peuvent ici être liés l'un à l'autre ou les uns aux autres et peuvent former un cycle ;
où au moins l'un des deux groupes Ar¹ doit contenir 10 atomes de cycle aromatique ou plus ; et
où, si l'un des deux groupes Ar¹ est un groupe phényle, l'autre des deux groupes Ar¹ ne doit pas contenir plus de 14 atomes de cycle aromatique.

2. Composé selon la revendication 1, **caractérisé en ce que** les liaisons depuis les groupes Ar² sur le groupe adjacent Ar¹ ou Ar² sont dans chaque cas présentes au niveau de la position para de l'un à l'autre.

3. Composé selon la revendication 1 ou 2, **caractérisé en ce que** les groupes Ar¹ sont sélectionnés pour chaque occurrence, de manière identique ou différente, parmi des groupes aryle ou des groupes hétéroaryle qui comportent de 6 à 14 atomes de cycle aromatique, lesquels peuvent dans chaque cas être substitués par un radical ou par plusieurs radicaux R¹.

4. Composé selon la revendication 3, **caractérisé en ce que** les groupes Ar¹ sont sélectionnés pour chaque occurrence parmi des groupes aryle ou des groupes hétéroaryle qui comportent de 6 à 10 atomes de cycle aromatique, lesquels peuvent dans chaque cas être substitués par un radical ou par plusieurs radicaux R¹.

5. Composé selon une ou plusieurs des revendications 1 à 4, **caractérisé en ce que** les groupes Ar² sont des groupes phényle, lesquels peuvent être substitués par un radical ou par plusieurs radicaux R².

6. Composé selon une ou plusieurs des revendications 1 à 5, **caractérisé en ce que** les groupes Ar¹ sont des groupes naphtyle, lesquels peuvent être substitués par un radical ou par plusieurs radicaux R¹, et les groupes Ar² sont des groupes phényle, lesquels peuvent être substitués par un radical ou par plusieurs radicaux R².

7. Composé selon une ou plusieurs des revendications 1 à 5, **caractérisé en ce que** l'un des deux groupes Ar¹ est un groupe phényle, lequel peut être substitué par un radical ou par plusieurs radicaux R¹, et l'autre des deux groupes Ar¹ est un groupe naphtyle, lequel peut être substitué par un radical ou par plusieurs radicaux R¹, et les groupes Ar² sont des groupes phényle, lesquels peuvent être substitués par un radical ou par plusieurs radicaux R².

8. Composé selon une ou plusieurs des revendications 1 à 7, **caractérisé en ce que** X¹ est sélectionné pour chaque occurrence, de manière identique ou différente, parmi C(R³)₂, -C(R³)₂-C(R³)₂-, -C(R³)₂-O-, Si(R³)₂, O, S, et NR³.

9. Composé selon une ou plusieurs des revendications 1 à 8, **caractérisé en ce que** X¹ est égal à C(R³)₂.

10. Composé selon une ou plusieurs des revendications 1 à 9, **caractérisé en ce que** R² est égal à H ou D.

11. Oligomère, polymère ou dendrimère contenant un ou plusieurs composé(s) selon une ou plusieurs des revendications 1 à 10, dans lequel la liaison/les liaisons sur le polymère, sur l'oligomère ou sur le dendrimère peut/peuvent être localisée(s) au niveau de n'importe quelles positions souhaitées dans la formule (I) qui sont substituées par R¹, R² ou R³.

12. Formulation comprenant au moins un composé selon une ou plusieurs des revendications 1 à 10, ou au moins un oligomère, un polymère ou un dendrimère selon la revendication 11, et au moins un solvant.

13. Dispositif électronique sélectionné parmi le groupe qui est constitué par les circuits intégrés organiques (OIC), les transistors à effet de champ organiques (OFET), les transistors à film mince organiques (OTFT), les transistors à émission de lumière ou luminescents organiques (OLET), les cellules solaires organiques (OSC), les détecteurs optiques organiques, les photorécepteurs organiques, les dispositifs à extinction de champ organiques (OFQD), les cellules électrochimiques à émission de lumière ou luminescentes organiques (OLEC), les diodes laser organiques (O-laser) et les dispositifs électroluminescents organiques (OLED), comprenant au moins un composé selon une ou plusieurs des revendications 1 à 10, ou au moins un oligomère, un polymère ou un dendrimère selon la revendication 11.

14. Dispositif électronique selon la revendication 13, sélectionné parmi les dispositifs électroluminescents organiques qui comprennent une cathode, une anode et au moins une couche organique, dans lequel l'au moins une couche organique comprend au moins un composé selon une ou plusieurs des revendications 1 à 10, ou au moins un oligomère, un polymère ou un dendrimère selon la revendication 11.

15. Dispositif électronique selon la revendication 14, **caractérisé en ce que** le composé selon une ou plusieurs des revendications 1 à 10 ou l'oligomère, le polymère ou le dendrimère selon la revendication 11 est présent en tant que matériau de transport de trous dans une couche de transport de trous, en tant que composé d'émission dans une couche d'émission ou en tant que composé de matrice dans une couche de matrice.

16. Utilisation d'un composé selon une ou plusieurs des revendications 1 à 10 ou d'un oligomères, d'un polymère ou d'un dendrimère selon la revendication 11 dans un dispositif électronique.

17. Procédé pour la préparation d'un composé selon une ou plusieurs des revendications 1 à 10, **caractérisé en ce qu'**il comprend au moins une réaction de couplage catalysée par métal et au moins une réaction de fermeture de cycle.
